(19) Europäisches Patentamt / European Patent Office / Office européen des brevets

(11) **EP 2 363 025 A1**

## (12) EUROPEAN PATENT APPLICATION

(43) Date of publication:
**07.09.2011 Bulletin 2011/36**

(21) Application number: **10185107.9**

(22) Date of filing: **26.03.2004**

(51) Int Cl.:
*A01N 61/00* [(2006.01)]  *C12Q 1/00* [(2006.01)]
*G01N 33/566* [(2006.01)]  *G01N 33/573* [(2006.01)]
*G01N 33/574* [(2006.01)]  *C12Q 1/68* [(2006.01)]

(84) Designated Contracting States:
**AT BE BG CH CY CZ DE DK EE ES FI FR GB GR HU IE IT LI LU MC NL PL PT RO SE SI SK TR**

(30) Priority: **27.03.2003 US 458067 P**

(62) Document number(s) of the earlier application(s) in accordance with Art. 76 EPC:
**04758540.1 / 1 613 160**

(71) Applicant: **PTC Therapeutics, Inc.**
**South Plainfield, NJ 07080 (US)**

(72) Inventor: **Trotta, Christopher R.**
**Somerset, NJ 08873 (US)**

(74) Representative: **Jones Day**
**Rechtsanwälte, Attorneys-at-Law, Patentanwälte**
**Prinzregentenstrasse 11**
**80538 München (DE)**

Remarks:
•Claims filed after the date of receipt of the divisional application (Rule 68(4) EPC).
•A request for correction of the abstract has been filed pursuant to Rule 139 EPC. A decision on the request will be taken during the proceedings before the Examining Division (Guidelines for Examination in the EPO, A-V, 3.).
•This application was filed on 30-09-2010 as a divisional application to the application mentioned under INID code 62.

(54) **Targeting enzymes of the TRNA splicing pathway for identification of anti-fungal and/or anti-proliferative molecules**

(57) Solid forms comprising 4-[9-(tetrahydro-furan-3-yl)-8-(2,4,6-trifluoro- phenylamino)-9H-purin-2-ylamino]-cyclohexan-1-ol, compositions comprising the solid forms, methods of making the solid forms and methods of their use for the treatment of various diseases and/or disorders are disclosed.

HTS Fluorescent Screening

5'    3'

5' SS    3' SS

*Fig. 1A*

EP 2 363 025 A1

**Fig. 1B**

**Fig. 1C**

**Description**

1. <u>FIELD OF THE INVENTION</u>

The present invention relates to a method for screening and identifying compounds that modulate the activity of one or more components in the tRNA splicing pathway. In particular the invention relates to a method for screening and identifying compounds that modulate the activity tRNA splicing endonuclease and/or tRNA splicing ligase. The invention provides assays for the identification of compounds that inhibit animalia tRNA splicing endonuclease and/or animalia tRNA splicing ligase. The invention also provides assays for the identification of compounds that inhibit fungal tRNA splicing endonuclease and/or fungal tRNA splicing ligase. The methods of the present invention provide a simple, sensitive assay for high-throughput screening of libraries of compounds to identify pharmaceutical leads useful for treating and/or preventing cancer and/or fungal infections.

2. <u>BACKGROUND OF THE INVENTION</u>

2.1 <u>CANCER AND NEOPLASTIC DISEASE</u>

Cancer is the second leading cause of death in the United States. The American Cancer Society estimated that in 2001, there would be 1.3 million new cases of cancer and that cancer will cause 550,000 deaths. Overall rates have declined by 1% per year during the 1990s. There are 9 million Americans alive who have ever had cancer. NIH estimates the direct medical costs of cancer as $60 billion.

Currently, cancer therapy involves surgery, chemotherapy and/or radiation treatment to eradicate neoplastic cells in a patient *(see,* for example, Stockdale, 1998, "Principles of Cancer Patient Management", in Scientific American: Medicine, vol. 3, Rubenstein and Federman, eds., Chapter 12, Section IV). All of these approaches pose significant drawbacks for the patient. Surgery, for example, can be contraindicated due to the health of the patient or can be unacceptable to the patient. Additionally, surgery might not completely remove the neoplastic tissue. Radiation therapy is effective only when the irradiated neoplastic tissue exhibits a higher sensitivity to radiation than normal tissue, and radiation therapy can also often elicit serious side effects. (*Id.*) With respect to chemotherapy, there are a variety of chemotherapeutic agents available for the treatment of neoplastic disease. However, despite the availability of a variety of chemotherapeutic agents, traditional chemotherapy has many drawbacks *(see,* for example, Stockdale, 1998, "Principles Of Cancer Patient Management" in Scientific American Medicine, vol. 3, Rubenstein and Federman, eds., ch. 12, sect. 10). Almost all chemotherapeutic agents are toxic, and chemotherapy can cause significant, and often dangerous, side effects, including severe nausea, bone marrow depression, immunosuppression, etc. Additionally, many tumor cells are resistant or develop resistance to chemotherapeutic agents through multidrug resistance.

Therefore, there is a significant need in the art for novel compounds and compositions, and methods that are useful for treating cancer or neoplastic disease with reduced or without the aforementioned side effects. Further, there is a need for cancer treatments that provide cancer-cell-specific therapies with increased specificity and decreased toxicity.

2.2 <u>FUNGAL INFECTION</u>

2.2.1, <u>PATHOGENESIS</u>

Fungi are eukaryotic microorganisms and can occur as yeasts, molds, or as a combination of both forms. Some fungi are capable of causing superficial, cutaneous, subcutaneous, systemic or allergic diseases. Yeasts are microscopic fungi consisting of solitary cells that reproduce by budding. Molds, in contrast, occur in long filaments known as hyphae, which grow by apical extension. Hyphae can range from sparsely septate to regularly septate and possess a variable number of nuclei. Regardless of their shape or size, fungi are all heterotrophic and digest their food externally by releasing hydrolytic enzymes into their immediate surroundings (absorptive nutrition).

Fungal and other mycotic pathogens (some of which are described in Human Mycoses, E.S. Beneke, Upjohn Co.:Kalamazoo, MI, 1979; Opportunistic Mycoses of Man and Other Animals, J.M.B. Smith, CAB International:Wallingford, UK, 1989; and Scrip's Antifungal Report, by PJB Publications Ltd, 1992) are responsible for a variety of diseases in humans, animals, and plants ranging from mycoses involving skin, hair, or mucous membranes, such as, but not limited to, Aspergillosis, Black piedra, Candidiasis, Chromomycosis, Cryptococcosis, Onychomycosis, or Otitis externa (otomycosis), Phaeohyphomycosis, Phycomycosis, Pityriasis versicolor, ringworm, Tinea barbae, Tinea capitis, Tinea corporis, Tinea cruris, Tinea favosa, Tinea imbricata, Tinea manuum, Tinea nigra (palmaris), Tinea pedis, Tinea unguium, Torulopsosis, Trichomycosis axillaris, White piedra, and their synonyms, to severe systemic or opportunistic infections, such as, but not limited to, Actinomycosis, Aspergillosis, Candidiasis, Chromomycosis, Coccidioidomycosis, Cryptococcosis, Entomophthoramycosis, Geotrichosis, Histoplasmosis, Mucormycosis, Mycetoma, Nocardiosis, North American Blastomycosis, Paracoccidioidomycosis, Phaeohyphomycosis, Phycomycosis, pneumocystic pneumonia, Pythiosis, Sporotrichosis, and Torulopsosis, and their synonyms, some of which may be fatal.

Known fungal and mycotic pathogens include, but are not limited to, Absidia spp., Actinomadura madurae, Actinomyces spp., Allescheria boydii, Alternaria spp., Anthopsis deltoidea, Apophysomyces elegans, Arnium leoporinum, Aspergillus spp., Aureobasidium pullulans, Basidiobolus ranarum, Bipolaris spp., Blastomyces dermatitidis, Candida spp., Cephalosporium spp., Chaetoconidium spp., Chaetomium spp., Cladosporium spp., Coccidioides immitis, Conidiobolus spp., Corynebacterium tenuis, Cryptococcus spp., Cunninghamella bertholletiae, Curvularia spp., Dactylaria spp., Epidermophyton spp., Epidermophyton floccosum, Exserophilum spp., Exophiala spp., Fonsecaea spp., Fusarium spp., Geotrichum spp., Helminthosporium spp., Histoplasma spp., Lecythophora spp., Madurella spp., Malassezia furfur, Microsporum spp., Mucor spp., Mycocentrospora acerina, Nocardia spp., Paracoccidioides brasiliensis, Penicillium spp., Phaeosclera dematioides, Phaeoannellomyces spp., Phialemonium obovatum, Phialophora spp., Phoma spp., Piedraia hortai, Pneumocystis carinii, Pythium insidiosum, Rhinocladiella aquaspersa, Rhizomucor pusillus, Rhizopus spp., Saksenaea vasiformis, Sarcinomyces phaeomuriformis, Sporothrix schenckii, Syncephalastrum racemosum, Taeniolella boppii, Torulopsosis spp., Trichophyton spp., Trichosporon spp., Ulocladium chartarum, Wangiella dermatitidis, Xylohypha spp., and their synonyms. Other fungi that "obviously have pathogenic potential" (Smith, op. cit.) include, but are not limited to, Thermomucor indicaeseudaticae, Radiomyces spp., and other species of known pathogenic genera. There are also reports implicating Saccharomyces as a human pathogen (*e.g.*, Fungemia with Saccharomycetacea, H. Nielson, J. Stenderup, & B. Bruun, Scand. J. Infect. Dis. 22:581-584, 1990). Fungal infections in humans, to a large extent, have been satisfactorily controlled by a human host's immune response mechanisms combined with the aid of conventionally-accepted and readily available antifungal treaments; however, in recent years, there has been a marked increase in the number of serious mycoses as a result of the growing number of immunosuppressed and immunocompromised individuals, such as transplant recipients, patients receiving chemotherapy, and HIV-infected individuals.

Fungal infection is also a significant problem in veterinary medicine including, but not limited to, candidiasis, cryptococcosis, aspergillosis, mucormycosis, pythiosis, entomophthoramycosis, oomycosis, chromomycosis, torulopsosis, infections with Penicillium spp., Trichosporon spp., Paecilomyces spp., Microsporum spp., and a variety of miscellaneous/rarer opportunistic mycoses (Opportunistic Mycoses of Man and Other Animals, J.M.B. Smith, CAB International, Wallingford, UK, 1989). Fungal infections are a common cause of nasal disease in dogs and cats (Fungal Diseases of the Nasal Cavity of the Dog and Cat, Wolf, A.M., 1992, Vet. Clin. of North Amer.:Small Anim. Prac. 22, 1119-1132). A variety of fungi, including, but not limited to, Aspergillus spp., Candida spp., Paecilomyces spp., Penicillium spp., Alternaria spp., Geotrichum spp., and Cladosporium spp., have been isolated from animal eyes and may cause fungal keratitis in several species including, but not limited to, horses, dogs, and cats (Microbiology of the Canine and Feline Eye, P.A. Gerding and I. Kakoma, 1990, Vet. Clin. of North Amer.:Small Anim. Prac. 20, 615-625). Skin infections by fungi, including, but not limited to, <u>Microsporum canis</u>, <u>Trichophyton mentagrophytes</u>, <u>Trichophyton verucosum</u>, <u>Microsporum equinum</u>, <u>Microsporum gallinae</u>, and <u>Microsporum nanum</u>, occur in many different animals, both wild and domestic with some infections being specific to a given host species (Fungal Skin Infections Associated with Animal Contact, W.H. Radentz, 1991, AFP 43, 1253-1256).

Some of the fungi that infect animals can be transmitted from animals to humans. Fungal zoonotic diseases are most commonly associated with animals used as pets, with a higher frequency found among veterinary personnel owing to higher levels of contact with animals (<u>ibid.</u>, M.R. Lappin, Vet. Clin. of North Amer.:Small Anim. Prac. 23, 57-78). Topical and systemic antifungal agents are used to treat both humans and animals.

Fungal infections or infestations are also a very serious problem in agriculture with fungicides being employed to protect vegetable, fruit, and nut crops (F.L. McEwen and G.R. Stephenson, 1979, The Use and Significance of Pesticides in the Environment. Wiley, NY). Fungicides are applied to soil, seeds, propagating material, growing plants, and produce to combat pathogens. Seed and soil-borne pathogens include but are not limited to Aphamomyces spp., Armillaria spp., Cephalosporium spp., Cylindrocladium spp., Fusarium spp., Helminthosporium spp., Macrophomina spp., Magnaporthe spp., Ophiobolus spp., Phymatotrichum spp., Phytophthora spp., Pythium spp., Rhizoctonia spp., Scerotium spp., Sclerotinia spp., Thielaviopsis spp., Ustilago spp., Verticillium spp., and Whetxelinia spp., (R. Rodriguez-Kabana, P.A. Backman, and E.A. Curl, Control of Seed and Soil-Borne Plant Diseases. In Antifungal Compounds, M. Siegel and H. Sisler, eds., Marcel Dekker Inc., NY, 1977). Post-harvest diseases of fresh fruits and vegetable are caused by fungi including, but not limited to, Alternaria spp., Botrytis spp., Centrospora spp., Ceratocystis spp., Colletotrichum spp, Cryptoporiopsis spp., Diplodia spp., Fusarium spp., Helminthosporium spp. Monilinia spp., Nectria spp., Oospora spp., Penicillium spp., Phlyctaena spp., Phoma spp., Phomopsis spp., Rhizopus spp., Sclerotinia spp., and Verticillium spp.

It has been estimated that fungicides are employed in the growing of one-half of the world's crops (G. Ordish and J.F. Mitchell. 1967, World Fungicide Usage. In Fungicides, an Advanced Treatise, Vol. 1, pp.39-62. D.C. Torgeson, ed. Academic Press, NY.) either to control disease during crop development, to improve the storage of produce, or to increase production of a particular crop. Approximately 20% of U.S. non-pasture crop land is treated with fungicides (E.W. Palm, Estimated Crop Losses Without the Use of Fungicides and Nematicides and Without Nonchemical Controls. CRC Handbook of Pest Management in Agriculture, Vol. 1, p.139f.). In economic terms, the cessation of fungicide use would result in losses to field crops, vegetable crops, and fruit and nut crops estimated to total over two billion dollars (ibid.). Some crops would be particularly hard hit, *e.g.*, peanut losses would be expected to be >70% of the total crop, pecan losses >65% of the total crop, tomato losses >60% of the total crop, potato losses >40% of the total crop, and fruits such as apples, cherries, peaches, and pears each >50% of their total crop (ibid.).

Fungal attack of wood products is also of major economic importance with an estimated one billion dollars in damage annually (not including damage to living trees) in the U.S., even with the extensive use of existing preservatives (M.P. Levi, Fungicides in Wood Preservation, In Antifungal Compounds, M. Siegel and H. Sisler, eds., Marcel Dekker Inc., NY, 1977). Hundreds of fungal species have been isolated from wood products. Surface molds result from infestation by genera including, but not limited to, Trichoderma spp., Gliocladium spp., Penicillium spp., Aspergillus spp., and Alternaria spp. Sap stain fungi include, but are not limited to, Ceratocystis spp., Diplodia spp., Graphium spp., Aureobasidium spp., and Cytospora spp. Decay fungi responsible for a large proportion of the economic losses include, but are not limited to, Coniophora spp., Lentinus spp., Lenzites spp., Polyporus spp., Poria spp., and Merulius spp. Soft-rot fungi include, but are not limited to, Ascomycetes spp., Chaetomium spp., and Fungi Imperfecti.

Additional products that are susceptible to fungal infestation include textiles, plastics, paper, rubber, adhesives, emulsion polymers, leather, cosmetics, household disinfectants, deodorants, and paint. (C.C. Yeager, Fungicides in Industry, in Antifungal Compounds, M. Siegel and H. Sisler, eds., Marcel Dekker Inc., NY, 1977). More work has been done on paint than on any other substrate. Fungi that attack painted surfaces often disfigure the paint film to the point where replacement is required. Repainting can solve the problem only temporarily as the organism may erupt through the new coating. Paint infestations include, but are not limited to, Pullularia spp., Cladosporium spp., Aspergillus spp., and Penicillium spp. The only successful method of combating fungal growth on paint systems requires the addition of a suitable fungistat or fungicide.

## 2.2.2 CURRENT THERAPY

The development of antifungal drug therapies has not evolved as rapidly as the development of antibacterial drug therapies in large part because the human or animal host and the fungal pathogen are both eukaryotes and have many drug targets in common. To date, most of the antifungal drugs and lead compounds have been active against components of the fungal cell surface or membrane (New Antifungal Agents, J.R. Graybill, Eur. J. Clin. Microbiol. Dis. 8:402-412, 1989; Targets for Antifungal Drug Discovery, Y. Koltin, Annual Reports in Medicinal Chemistry 25:141-148, 1989; Screening of Natural Products for Antimicrobial Agents, L. Silver & K. Bostian, Eur. J. Clin. Microbiol. Dis. 9: 455-461, 1990; New Approaches for Antifungal Drugs, P.B. Fernandes, ed, Birkhauser:Boston, 1992; Scrip's Antifungal Report, by PJB Publications Ltd, 1992). For example, polyene macrolides bind to fungal-specific ergosterol on the cell surface and azole drugs inhibit an ergosterol biosynthetic enzyme. While there has been some effort directed at intracellular targets, such as tubulin and nucleotide metabolism, the resulting compounds, such as benomyl and fluorocytosine, have problems with toxicity and resistance. Cycloheximide (Actidione) is used as a fungicide on some crops even though it is not particularly specific for fungi. Blasticidin S is also used as an antifungal agent on crops.

Not only are fungal-specific therapeutics difficult to identify, but many of the drugs currently available for treatment of mycoses have significant side effects or lack effectiveness against some important pathogens. For example, amphotericin B, an antifungal polyene macrolide antibiotic, has both short-term and long-term adverse effects, ranging from nausea and vomiting to kidney damage. Azole drugs such as clotrimazole and miconazole have such adverse side effects that their use is generally limited to the treatment of topical or superficial infections. The more recently developed triazole drugs, such as fluconazole, have fewer side effects but are not completely effective against all pathogens. Also, some evidence exists for the development of resistance to these drugs. There is therefore an ongoing need for novel antifungal drugs with few, if any, side effects and with effectiveness against pathogens for which current drugs are inadequate.

Furthermore, fungal and mycotic pathogens often are either naturally resistant, or develop resistance, to many therapeutics by virtue of cellular permeability barriers to drug entry. Development of fungicide resistance occurs when a fungal cell or a fungal population that originally was sensitive to a fungicide becomes less sensitive by heritable changes after a period of exposure to the fungicide. Most instances of resistance are related to a change at the site of action or a change in the uptake of the fungicide, with detoxification being a rare event (J. Dekker, Preventing and Managing Fungicide Resistance, Pesticide Resistance: Strategies and Tactics in Man). In certain applications (*e.g.*, agriculture) it is possible to combat resistance through alternation of fungicides or the use of fungicide mixtures. To prevent or delay the buildup of a resistant pathogen population, different chemicals that are effective against a particular disease must be available. One way of increasing the number of available chemicals is to search for new site-specific inhibitors (id.). Thus, the challenge is to develop methods for identifying compounds which can penetrate the pathogen and specifically kill it or arrest its growth without also adversely affecting the human, animal, or plant host.

Classical approaches for identifying antifungal compounds have relied almost exclusively on inhibition of fungal growth as an endpoint. Libraries of natural products, semisynthetic, or synthetic chemicals are screened for their ability to kill or arrest growth of the target pathogen or a related nonpathogenic model organism. These tests are cumbersome and provide no information about a compound's mechanism of action. The promising lead compounds that emerge from such screens must then be tested for possible toxicity to the human, animal, or plant host, and detailed mechanism-of-action studies must subsequently be conducted to identify the affected molecular target and precisely how the drug interacts with this target.

Because treatment of mycoses are assuming even greater public importance, especially in light of the growing number of immunocompromised or immunosuppressed individuals and pronounced public apprehension of mycotic infestion in residences and places of work, pressure has mounted to develop more effective methods for antifungal and antimycotic drug discovery.

Commercial success of antifungal agents is heavily dependent on efficacy relative to existing therapies for the target indication. Thus, the heightened specificity and expected lower cytotoxicity of inhibitors of the tRNA splicing pathway identified and developed through the methods of the present invention will lead to a drug with a competitive advantage to those currently on the market.

### 2.3 tRNA PRODUCTION

Maturation and maintenance of tRNA within eucaryal cells requires several processing events including 5' and 3' end-trimming, modification of specific bases and in some cases, intron removal. The enzymes for these various steps in processing have been characterized in the yeast, archaeal, mammalian and bacterial systems (Deutscher, M.P. tRNA Processing Nucleases, in tRNA:Structure, Biosynthesis and Function, D. Soll and U. RjaBhandary (eds.), American Society for Microbiology, Washington DC, (1995), pp. 51-65). 5' end trimming requires the activity of Rnase P and 3' end trimming requires the function of various endo- and exo- nucleases. Modification occurs through interaction of tRNA with various modification enzymes. Most tRNAs contain a number of global as well as, species-specific modifications (Bjork, G. Biosynthesis and Function of Modified Nucleosides, in tRNA: Structure, Biosynthesis and Function, D. Soll and U. RajBhandary (eds.), American Society for Microbiology, Washington DC, (1995), pp. 165-205). In archaea and eucarya, several isoaccepting groups of tRNA contain intervening sequences ranging in size from 14-105 nucleotides (Trotta, C.R. and Abelson, J.N. tRNA Splicing: An RNA World Add-On or an Ancient Reaction? In RNA World II, Tom Cech, Ray Gesteland and John Atkins (eds.), Cold Spring Harbor Laboratory Press (1999) and Abelson et al., 1998, Journal of Biological Chemistry 273:12685-12688). Removal of the intron requires the activity of 3 enzymes. In the first step, the tRNA is recognized and cleaved at the 5' and 3' junction by the tRNA splicing endonuclease. The archaeal and eucaryal tRNA endonuclease are evolutionary conserved enzymes and contain a similar active site to achieve cleavage at the 5' and 3' splice sites. However, they have diverged to recognize the tRNA substrate in a different manner. The archaeal enzyme recognizes a conserved intronic structure known as the bulge-helix-bulge. This structure is comprised of two 3-nucleotide bulges separated by a 4-nucleotide helix. Cleavage occurs within each bulge to release the intron. The eucaryal endonuclease recognizes the tRNA substrate in a mature domain dependent fashion, measuring a set distance from the mature domain to the 5' and 3' splice sites (Reyes et al., 1988, Cell 55:719-730). It has recently been demonstrated, however, that the eucaryal enzyme requires a bulge at each splice site and that the enzyme has actually retained the ability to recognize tRNA by an intron-dependent recognition mechanism identical to that of the archaeal endonuclease (Fruscoloni et al., 2001, EMBO Rep 2:217-221).

Once cleaved, the tRNA half molecules are ligated by the action of a unique tRNA splicing ligase (Trotta, C.R. and Abelson, J.N. tRNA Splicing: An RNA World Add-On or an Ancient Reaction? In RNA World II, Tom Cech, Ray Gesteland and John Atkins (eds.), Cold Spring Harbor Laboratory Press (1999) and Abelson et al., 1998, Journal of Biological Chemistry 273:12685-12688). In yeast, the product of ligation is a tRNA with a phosphate at the splice junction. Removal of the phosphate is carried out by a tRNA 2'-phosphotransferase to yield a mature tRNA product (Trotta, C.R. and Abelson, J.N. tRNA Splicing: An RNA World Add-On or an Ancient Reaction? In RNA World II, Tom Cech, Ray Gesteland and John Atkins (eds.), Cold Spring Harbor Laboratory Press (1999) and Abelson et al., 1998, Journal of Biological Chemistry 273:12685-12688).

tRNA is an important component in the translational machinery and is quite stable compared to various other protein-based components (elongation factors, amino-acyl synthetases, etc.). tRNA molecules have very long half-lives. Furthermore, like rRNA and ribosomes, tRNA is present in excess within the cytoplasm of actively growing cells (Ikemura, T. and Okeki, H., 1983, Cold Spring Harbor Symp. Quant. Biol. 47:1087-1097). Thus, specific targeting of tRNA molecules allows a selective inhibition of uncontrolled cell proliferation and not cell growth.

### 3. SUMMARY OF THE INVENTION

The present invention provides methods for identifying a compound that modulates the activity of one or more components (*e.g.*, enzymes such tRNA splicing endonuclease and tRNA splicing ligase) of an animalia or fungal tRNA splicing pathway. In particular, the invention provides methods for identifying a compound that inhibits or reduces the activity of an animalia and/or fungal tRNA splicing endonuclease. The invention also provides methods for identifying a compound that inhibits or reduces the activity of an animalia and/or fungal tRNA splicing ligase. The invention encompasses the use of the compounds identified utilizing the methods of the invention that inhibit or reduce the activity of one or more components of a fungal tRNA splicing pathway for the prevention, treatment, management or amelioration of a fungal infection or a symptom thereof. More particularly, the invention encompasses the use of the compounds identified utilizing the methods of the invention that inhibit or reduce the activity of a fungal tRNA splicing endonuclease and/or a fungal tRNA splicing ligase for the prevention, treatment, management or amelioration of a fungal infection or a symptom thereof. The invention also encompasses the use of compounds identified utilizing the methods of the invention that inhibit or reduce the activity of an animalia tRNA splicing pathway for the prevention, treatment, management or amelioration of a proliferative disorder or a symptom thereof. More specifically, the invention also encompasses the use of compounds identified utilizing the methods of the invention that inhibit or reduce the activity of an animalia tRNA splicing endonuclease and/or an animalia tRNA splicing ligase for the prevention, treatment, management or amelioration of a proliferative disorder or a symptom thereof.

The invention provides cell-based and cell-free assays for the identification of a compound that modulates the activity of a component of a fungal or animalia tRNA splicing pathway (*e.g.*, an animalia tRNA splicing endonuclease, a fungal tRNA splicing endonuclease, an animalia tRNA splicing ligase or a fungal tRNA splicing ligase). These assays may be reporter gene-based assays, fluorescence resonance energy transfer ("FRET")-based assays, or fluorescence polarization assays and may be conducted in a high throughput screen format. Further, these assays directly or indirectly measure the ability of a compound to modulate a component of a fungal or animalia tRNA splicing pathway (*e.g.*, an animalia tRNA splicing endonuclease, a fungal tRNA splicing endonuclease, an animalia tRNA splicing ligase or a fungal tRNA splicing ligase). In a preferred embodiment, the ability of a compound to modulate of a fungal or animalia tRNA splicing pathway (*e.g.*, an animalia tRNA splicing endonuclease, a fungal tRNA splicing endonuclease, an animalia tRNA splicing ligase or a fungal tRNA splicing ligase) that was identified utilizing an indirect assay (*e.g.*, a cell-based assay such as a reporter gene cell-based assay or a FRET cell-based assay) is confirmed utilizing a more direct assay (*e.g.*, a FISH assay).

The reporter gene-based assays may be conducted by contacting a compound with an animalia or fungal cell genetically engineered to express a nucleic acid comprising a reporter gene, wherein the reporter gene comprises a tRNA intron, and measuring the expression of said reporter gene. Alternatively, the reporter gene-based assays may be conducted by contacting a compound with an animalia or fungal cell-free extract and a nucleic acid comprising a reporter gene, wherein the reporter gene comprises a tRNA intron, and measuring the expression of said reporter gene. The alteration in reporter gene expression relative to a previously determined reference range, or to the expression in the absence of the compound or the presence of an appropriate control (*e.g.*, a negative control) in such reporter-gene based assays indicates that a particular compound modulates the activity of a component of an animalia or fungal tRNA splicing pathway. In particular, a decrease in reporter gene expression relative to a previously determined reference range, or to the expression in the absence of the compound or the presence of an appropriate control (*e.g.*, a negative control) in such reporter-gene based assays indicates that a particular compound reduces or inhibits the activity of a component of an animalia or fungal tRNA splicing pathway (*e.g.*, an animalia or fungal tRNA splicing endonuclease by, *e.g.,* inhibiting or reducing the recognition or cleavage of a tRNA intron the endonuclease, or an animalia or fungal tRNA splicing ligase). In contrast, an increase in reporter gene expression relative to a previously determined reference range, or to the expression in the absence of the compound or the presence of an appropriate control (*e.g.*, a negative control) in such reporter-gene based assays indicates that a particular compound enhances the activity of an animalia or fungal tRNA splicing pathway (*e.g.*, an animalia or fungal tRNA splicing endonuclease, or an animalia or fungal tRNA splicing ligase).

In one embodiment, the invention provides a method for identifying a compound that modulates the activity of an enzyme in an animalia or fungal tRNA splicing tRNA splicing pathway (*e.g.*, an animalia or fungal tRNA splicing endonuclease, or an animalia or fungal tRNA splicing ligase), said method comprising: (a) expressing a nucleic acid comprising a reporter gene in an animalia or fungal cell, wherein the reporter gene comprises a tRNA intron; (b) contacting said cell with a member of a library of compounds; and (c) detecting the expression of said reporter gene, wherein a compound that modulates the activity of an enzyme in the tRNA splicing pathway is identified if the expression of said reporter gene in the presence of a compound is altered relative to a previously determined reference range, or the expression of said reporter gene in the absence of the compound or the presence of an appropriate control (*e.g.*, a negative control).

In another embodiment, the invention provides a method for identifying a compound that modulates an enzyme in an animalia or fungal tRNA splicing tRNA splicing pathway (*e.g.*, an animalia or fungal RNA splicing endonuclease, or an animalia or fungal tRNA splicing ligase), said method comprising: (a) contacting a member of a library of compounds with an animalia or fungal cell containing a nucleic acid comprising a reporter gene, wherein the reporter gene comprises a tRNA intron; and (b) detecting the expression of said reporter gene, wherein a compound that modulates the activity of an enzyme in the tRNA splicing pathway is identified if the expression of said reporter gene in the presence of a compound is altered relative to a previously determined reference range, or the expression of said reporter gene in the absence of said compound or the presence of an appropriate control (*e.g.*, a negative control).

In another embodiment, the invention provides a method for identifying a compound that modulates an enzyme in an animalia or fungal tRNA splicing tRNA splicing pathway (*e.g.*, an animalia or fungal tRNA splicing endonuclease, or an animalia or fungal tRNA splicing ligase), said method comprising: (a) contacting a member of a library of compounds with an animlia or fungal cell-free extract and a nucleic acid comprising a reporter gene, wherein the reporter gene comprises a tRNA intron; and (b) detecting the expression of said reporter gene, wherein a compound that modulates the activity of an enzyme in the tRNA splicing pathway is identified if the expression of said reporter gene in the presence of a compound is altered relative to a previously determined reference range, or the expression of said reporter gene in the absence of said compound or the presence of an appropriate control (*e.g.*, a negative control).

In accordance with the invention, the step of contacting a compound with a cell, or cell-free extract and a nucleic acid in the reporter gene-based assays described herein is preferably conducted in an aqueous solution comprising a buffer and a combination of salts (such as KCl, NaCl and/or $MgCl_2$). The optimal concentration of each salt used in the aqueous solution is dependent on the tRNA splicing pathway enzyme and the compounds used, and can be determined using routine experimentation. In a specific embodiment, the aqueous solution approximates or mimics physiologic conditions. In another specific embodiment, the aqueous solution further comprises a detergent or a surfactant.

The reporter gene constructs utilized in the reporter gene-based assays described herein may comprise the coding region of a reporter gene and a tRNA intron that renders the mRNA coding the reporter gene out of frame. Alternatively, the reporter gene constructs utilized in the reporter gene-based assays described herein comprise a tRNA intron within the 5' untranslated region, 3' untranslated region or both the 5' and 3' untranslated regions. In another alternative, the tRNA intron interrupts an mRNA splicing element. In a specific embodiment, a reporter gene construct utilized in the reporter gene-based assays described herein comprises the coding region of a reporter gene and a tRNA intron within the open reading frame of the reporter gene. The intron utilized in the reporter gene constructs described herein may comprise a bulge-helix-bulge conformation. In a preferred embodiment, a reporter gene construct utilized in the reporter-gene-based assays described herein comprises a mature domain containing a tRNA intron.

Any reporter gene well-known to one of skill in the art may be utilized in the reporter gene constructs described herein. Examples of reporter genes include, but are not limited to, the gene encoding firefly luciferase, the gene coding renilla luciferase, the gene encoding click beetle luciferase, the gene encoding green fluorescent protein, the gene encoding yellow fluorescent protein, the gene encoding red fluorescent protein, the gene encoding cyan fluorescent protein, the gene encoding blue fluorescent protein, the gene encoding beta-galactosidase, the gene encoding beta-glucoronidase, the gene encoding beta-lactamase, the gene encoding chloramphenicol acetyltransferase, and the gene encoding alkaline phosphatase.

The reporter gene-based assays described herein may be conducted in an animalia or fungal cell genetically engineered to express a reporter gene or *in vitro* utilizing an animalia or fungal cell-free extract. Any cell or cell line of any animal or fungal species well-known to one of skill in the art may be utilized in accordance with the methods of the invention. Further, a cell-free extract may be derived from any cell or cell line of any animal or fungal species well-known to one of skill in the art. Examples of cells and cell types include, but are not limited to, human cells, cultured mouse cells, yeast cells, cultured rat cells or Chinese hamster ovary ("CHO") cells.

The effect of a compound on the activity of a component of an animalia or fungal tRNA splicing pathway (*e.g.*, an animalia tRNA splicing endonuclease, a fungal tRNA splicing endonuclease, an animalia tRNA splicing ligase or a fungal tRNA splicing ligase) may be determined utilizing a tRNA endonuclease suppression assay. In such an assay, a host cell is engineered to contain a reporter gene and a suppressor tRNA, wherein the reporter gene construct comprises a reporter gene with a nonsense codon in its open reading frame such that the open reading frame is interrupted and the suppressor tRNA's expression is regulated by an inducible regulatory element and the suppressor tRNA contains a tRNA intron in the antisense codon; the expression of the suppressor tRNA is induced; the host cell is contacted with a compound; and the expression of the reporter gene and/or the activity of the protein encoded by the reporter gene is measured utilizing techniques well-known to one of skill in the art or described herein. A compound that inhibits or reduces the activity of a component of an animalia or fungal tRNA splicing pathway (*e.g.*, an animalia tRNA splicing endonuclease, a fungal tRNA splicing endonuclease, an animalia tRNA splicing ligase or a fungal tRNA splicing ligase) will inhibit or reduce the production of functional suppressor tRNA and thus, reduce the expression of the reporter gene relative to a previously determined reference range, or the expression of the reporter gene in the absence of the compound or the presence of an appropriate control (*e.g.*, a negative control). A compound that enhances the activity of a component of an animalia or fungal tRNA splicing pathway (*e.g.*, an animalia tRNA splicing endonuclease, a fungal tRNA splicing endonuclease, an animalia tRNA splicing ligase or a fungal tRNA splicing ligase) will enhance the production of functional suppressor tRNA and thus, enhance the production of the reporter gene relative to a previously determined reference range, or the expression of the reporter gene in the absence of the compound or the presence of an appropriate control (*e.g.*, a negative control).

Fluoroscent resonance energy transfer ("FRET") assays may be used to identify a compound that modulates the activity of an enzyme in an animalia or fungal tRNA splicing tRNA splicing pathway (*e.g.*, an animalia or fungal tRNA splicing endonuclease, or an animalia or fungal tRNA splicing ligase). The FRET assays may be conducted utilizing a labeled enzyme in an animalia or fungal tRNA splicing pathway or labeled subunits of an enzyme in an animalia or fungal tRNA splicing pathway, or labeled substrates for an enzyme in an animalia or fungal tRNA splicing pathway. The FRET cell-based assays may be conducted by microinjecting or transfecting a substrate for an animalia or fungal tRNA splicing endonuclease into an animalia or fungal cell and contacting the cell with a compound, wherein the substrate is labeled at the 5' end with a fluorophore and labeled at the 3' end with a quencher, or, alternatively, the substrate is labeled at the 5' end with a quencher and labeled at the 3' end with a fluorophore, and measuring the fluorescence of the substrate by, *e.g.*, fluorescence microscopy or a fluorescence emission detector such as a Viewlux or Analyst. The endogenous tRNA splicing endonuclease will cleave the substrate and result in the production of a detectable fluorescent signal. A compound that inhibits or reduces the activity of the endogenous tRNA splicing endonuclease will inhibit or reduce the cleavage of the substrate and thus, inhibit or reduce the production of a detectable fluorescent signal. A compound that enhances the activity of the endogenous endonuclease will enhance the cleavage of the substrate and thus, increase the production of a detectable fluorescent signal. Alternatively, the FRET cell-based assays may be conducted by microinjecting or transfecting a substrate for an animalia or fungal tRNA splicing endonuclease into an animalia or fungal cell and contacting the cell with a compound, wherein the substrate is labeled at the 5' end with a fluorescent donor moiety and labeled at the 3' end with a fluorescent acceptor moiety, or alternatively, the substrate is labeled at the 5' end with a fluorescent acceptor moiety and labeled at the 3' end with a fluorescent donor moiety, and measuring the fluorescence of the substrate by, *e.g.*, fluoresence microscopy or a fluorescence emission detector such as a Viewlux or Analyst. The endogenous tRNA splicing endonuclease will cleave the substrate and result in a decrease in the fluorescence emission by the fluorescent donor moiety and fluorescent acceptor moiety at the wavelength of the fluorescent donor moiety. A compound that inhibits or reduces the activity of the endogenous tRNA splicing endonuclease will inhibit or reduce cleavage of the substrate and thus, increase the fluorescence emission of the fluorescent acceptor moiety at the wavelength of the fluorescent donor moiety. A compound that enhances the activity of the endogenous tRNA splicing endonuclease will enhance the cleavage of the substrate and thus, reduce the fluorescence emission of the fluorescent acceptor moiety at the wavelength of the fluorescent donor moiety.

Optionally, an agent known to inhibit or reduce the activity of an animalia or fungal tRNA splicing ligase such as an antibody that specifically binds to an animalia or fungal tRNA splicing ligase is included in the contacting step of the FRET assays to exclude the possibility that the compound is solely inhibiting or reducing the activity of the ligase. Alternatively, an animalia or fungal cell that is deficient in tRNA splicing ligase is used in the FRET assays. In some embodiments, the activity of a tRNA splicing ligase is inhibited or reduced by excluding ATP from the reaction mixture. Although not intending to be bound by a particular mechanism of action, since the activity of tRNA splicing ligase is dependent on the presence of ATP, excluding ATP from the reaction effectively reduces the activity of the tRNA splicing ligase.

In one embodiment, the invention provides a method of identifying an antiproliferative compound that inhibits or reduces animalia or fungal tRNA splicing endonuclease activity, said method comprising: (a) microinjecting or transfecting a substrate of a tRNA splicing endonuclease into an animalia or fungal cell, wherein the substrate is labeled at the 5' end with a fluorophore and labeled at the 3' end with a quencher, or, alternatively, the substrate is labeled at the 5' end with a quencher and labeled with a fluorophore; (b) contacting the cell with a member of a library of compounds; and (c) measuring the activity of the tRNA splicing endonuclease, wherein an antiproliferative compound that inhibits or reduces tRNA splicing activity is identified if a fluorescent signal is not detectable or reduced in the presence of the compound relative to the absence of the compound or the presence of a negative control. In another embodiment, the invention provides a method of identifying an antiproliferative compound that inhibits or reduces animalia or fungal tRNA splicing endonuclease activity, said method comprising: (a) contacting an animalia or fungal cell containing a substrate of a tRNA splicing endonuclease with a member of a library of compounds, wherein the substrate is labeled at the 5' end with a fluorophore and labeled at the 3' end with a quencher, or, alternatively, the substrate is labeled at the 5' end with a quencher or labeled at the 3' end with a fluorophore; and (b) measuring the activity of the tRNA splicing endonuclease, wherein an antiproliferative compound that inhibits or reduces tRNA splicing activity is identified if a fluorescent signal is not detectable or reduced in the presence of the compound relative to the absence of the compound or the presence of a negative control.

In another embodiment, the invention provides a method of identifying an antiproliferative compound that inhibits or reduces animalia or fungal tRNA splicing endonuclease activity, said method comprising: (a) microinjecting or transfecting a substrate of a tRNA splicing endonuclease into an animalia or fungal cell, wherein said substrate is labeled at the 5' end with a fluorescent donor moiety and labeled at the 3' end with a fluorescent acceptor moiety, or, alternatively, the substrate is labeled with at the 5' end with a fluorescent acceptor moiety and labeled at the 3' end with a fluorescent donor moiety; (b) contacting the cell with a member of a library of compounds; and (c) measuring the activity of the tRNA splicing endonuclease, wherein an antiproliferative compound that inhibits or reduces tRNA splicing activity is identified if the fluorescence emission of the fluorescent acceptor moiety at the wavelength of the fluorescent donor moiety in the presence of the compound is decreased relative to the absence of the compound or the presence of a negative control. In another embodiment, the invention provides a method of identifying an antiproliferative compound that inhibits or reduces animalia or fungal tRNA splicing endonuclease activity, said method comprising: (a) contacting an animalia or fungal cell containing substrate of a tRNA-splicing endonuclease with a member of a library of compounds, wherein the substrate is labeled at the 5' end with a fluorescent donor moiety and labeled at the 3' end with a fluorescent acceptor moiety, or, alternatively, the substrate is labeled at the 5' end with a fluorescent acceptor moiety and labeled at the 3' end with a fluorescent donor moiety; and (b) measuring the activity of the tRNA splicing endonuclease, wherein an antiproliferative compound that inhibits or reduces tRNA splicing activity is identified if the fluorescence emission of the fluorescent acceptor moiety at the wavelength of a fluorescent donor moiety in the presence of the compound is decreased relative to the absence of the compound or the presence of a negative control.

The FRET cell-free-based assays may be conducted by contacting a substrate for an animalia or fungal tRNA splicing endonuclease with an animalia or fungal cell-free extract (preferably, a tRNA splicing endonuclease extract) or a purified animalia or fungal tRNA splicing endonuclease and a compound, wherein the substrate is labeled at the 5' end with a fluorophore and labeled at the 3' end with a quencher, or, alternatively, the substrate is labeled at the 5' end with a quencher and labeled at the 3' end with a fluorophore, and measuring the fluorescence of the substrate by, *e.g.*, a fluorescence emission detector such as a Viewlux or Analyst. The tRNA splicing endonuclease in the animalia or fungal cell-free extract or the purified animalia or fungal tRNA splicing endonuclease will cleave the substrate and result in the production of a detectable fluorescent signal. A compound that inhibits or reduces the activity of the animalia tRNA splicing endonuclease will inhibit or reduce the cleavage of the substrate and thus, inhibit or reduce the production of a detectable fluorescent signal. A compound that enhances the activity of the animalia tRNA splicing endonuclease will enhance the cleavage of the substrate and thus, increase the production of a detectable fluorescent signal. Alternatively, the FRET cell-free-based assays may be conducted by contacting a substrate for an animalia or fungal tRNA splicing endonuclease with an animalia or fungal cell-free extract or a purified animalia or fungal tRNA splicing endonuclease and a compound, wherein the substrate is labeled at the 5' end with a fluorescent donor moiety and labeled at the 3' end with a fluorescent acceptor moiety, or, alternatively, the substrate is labeled at the 5' end with a fluorescent acceptor moiety and labeled at the 3' end with a fluorescent donor moiety, and measuring the fluorescence of the substrate by, *e.g.*, a fluorescence emission detector such as a Viewlux or Analyst. The tRNA splicing endonuclease in the animalia or fungal cell-free extract or the purified animalia or fungal tRNA splicing endonuclease will cleave the substrate and result in the production of a detectable fluorescent signal by the fluorescent donor moiety and fluorescent acceptor moiety at the wavelength of the fluorescent donor moiety. A compound that inhibits or reduces the activity of the tRNA splicing endonuclease will inhibit or reduce cleavage of the substrate and thus, increase the fluorescence emission of the fluorescent acceptor moiety at the wavelength of the fluorescent donor moiety. A compound that enhances the activity of the endogenous tRNA splicing endonuclease will enhance the cleavage of the substrate and thus, reduce the fluorescence emission of the fluorescent acceptor moiety at the wavelength of the fluorescent donor moiety.

Optionally, an agent known to inhibit or reduce the activity of an animalia or fungal tRNA splicing ligase such as an antibody that specifically binds to an animalia or fungal tRNA splicing ligase is included in the contacting step of the FRET assays to exclude the possibility that the compound is solely inhibiting or reducing the activity of the ligase. Alternatively, an animalia or fungal cell-free extract that is deficient in tRNA splicing ligase is used in the FRET assays. In some embodiments, the activity of a tRNA splicing ligase is inhibited or reduced by excluding ATP from the reaction mixture. Although not intending to be bound by a particular mechanism of action, since the activity of tRNA splicing ligase is dependent on the presence of ATP, excluding ATP from the reaction effectively reduces the activity of the tRNA splicing ligase.

In one embodiment, the invention provides a method of identifying an antiproliferative compound that inhibits or reduces animalia or fungal tRNA splicing endonuclease activity, said method comprising: (a) contacting an animalia or fungal cell-free extract (preferably, a tRNA splicing endonuclease extract) or a purified animalia or fungal tRNA splicing endonuclease with a substrate of a tRNA splicing endonuclease and a member of a library of compounds, wherein the substrate is labeled at the 5' end with a fluorophore and at the 3' end with a quencher, or, alternatively, the substrate is labeled at the 5' end with a quencher and labeled at the 3' end with a fluorophore; and (b) measuring the activity of the tRNA splicing endonuclease, wherein an antiproliferative compound that inhibits or reduces tRNA splicing endonuclease activity is identified if a greater fluorescent signal is detectable in the presence of the compound relative to the absence of the compound or the presence of a negative control. In another embodiment, the invention provides a method of identifying an antiproliferative compound that inhibits or reduces animalia or fungal tRNA splicing endonuclease activity, said method comprising: (a) contacting an animalia or fungal cell-free extract (preferably, a tRNA splicing endonuclease extract) or a purified animalia or fungal tRNA splicing endonuclease with a substrate of a tRNA splicing endonuclease and a member of a library of compounds, wherein said substrate is labeled at the 5' end with a fluorescent donor moiety and labeled at the 3' end with a fluorescent acceptor moiety, or, alternatively, the substrate is labeled at the 5' end with a fluorescent acceptor moiety and labeled at the 3' end with a fluorescent donor moiety; and (b) measuring the activity of the tRNA splicing endonuclease, wherein an antiproliferative compound that inhibits or reduces tRNA splicing endonuclease activity is identified if the fluorescence emission of the fluorescent acceptor moiety at the wavelength of the fluorescent donor moiety in the presence of the compound is increased relative to the absence of the compound or the presence of a negative control.

The substrates for a tRNA splicing endonuclease utilized in the FRET assays described herein comprise an intron. In a preferred embodiment, the substrate for a tRNA splicing endonuclease utilized in the FRET assays described herein comprises a tRNA intron. The intron may have a bulge-helix-bulge conformation In a preferred embodiment, the substrate comprises a mature domain that contains an intron.

The effect of a compound on the activity of an animalia or fungal tRNA splicing ligase may be determined utilizing a FRET assay. The FRET assay for an animalia or fungal tRNA splicing ligase may be conducted by contacting a substrate for a tRNA splicing ligase a population of 5' tRNA half molecules and a population of 3' tRNA half molecules with an animalia or fungal cell-free extract or a purified animalia or fungal tRNA splicing ligase and a compound or a member of a library of compounds, wherein the termini of one of the populations of tRNA molecules is labeled with a fluorophore and the other is labeled with a quencher, and measuring the fluorescence of the substrate. The tRNA splicing ligase will join the tRNA half molecules and prevent or reduce the fluorescence of the joined tRNA half molecules. A compound that inhibits the activity of the tRNA splicing ligase will inhibit or reduce the ligation of the tRNA half molecules and thus, increase the fluorescent signal detectable relative to the signal in the absence of the compound or the presence of a negative control (*e.g.*, PBS). A compound that enhances the activity of the tRNA splicing ligase will enhance the ligation of the tRNA half molecules and thus, maintain or decrease the detectable fluorescent signal relative to the signal in the absence of the compound or the presence of a negative control (*e.g.*, PBS).

The invention also encompasses a FRET cell-free assay for identifying a compound that modulates the activity of an animalia or fungal tRNA splicing endonuclease, comprising: contacting a substrate for an animalia or fungal tRNA splicing ligase comprising a population of 5' tRNA half molecules and a population of 3' tRNA half molecules with a cell-free extract (preferably, a tRNA splicing ligase extract) or a purified animalia or fungal tRNA splicing ligase and a compound or a member of a library of compounds, wherein the terminus of one population is labeled with a fluorescent donor moiety and the terminus of the other population is labeled with a fluorescent acceptor moiety; and measuring the fluorescence of the substrate at the wavelength of the fluorescent donor moiety. The tRNA splicing ligase join the tRNA half molecules and result in the fluorescence of the fluorescent acceptor moiety at the wavelength of the fluorescent donor moiety. A compound that inhibits the activity of the tRNA splicing ligase will inhibit or reduce the ligation of the tRNA half molecules and thus, decrease the fluorescence emission of the fluorescent acceptor moiety at the wavelength of the fluorescent donor moiety relative to the fluorescence emission in the absence of the compound or the presence of a negative control (*e.g.*, PBS). A compound that enhances the activity of the tRNA splicing ligase will enhance the ligation of the tRNA half molecules and thus, maintain or increase the fluorescence emission of the fluorescent acceptor moiety at the wavelength of the fluorescent donor moiety relative to the fluorescence emission in the absence of the compound or the presence of a negative control (*e.g.*, PBS).

The effect of a compound on the activity of a component of an animalia or fungal tRNA splicing pathway (in particular, tRNA splicing endonuclease and tRNA splicing ligase) may be determined utilizing a fluorescence polarization-based assay. In such an assay, a fluorescently labeled substrate for an animalia or fungal tRNA splicing endonuclease is contacted with an animalia or fungal cell-free extract or a purified animalia or fungal tRNA splicing endonuclease and a compound or member of a library of compounds; and the fluorescent polarized light emitted is measured utilizing techniques well-known to one of skill in the art or described herein, wherein an alteration in the fluorescently polarized light emitted relative to a control or the absence of the compound or the member of a library of compounds indicates that the compound or member of a library of compounds modulates animalia or fungal tRNA splicing endonuclease activity. Alternatively, a fluorescently labeled substrate for an animalia or fungal tRNA splicing ligase (*e.g.*, fluorescently labeled tRNA half molecules) is contacted with an animalia or fungal cell-free extract or a purified animalia or fungal tRNA splicing ligase and a compound or a member of a library of compounds, and the fluorescent polarized light emitted is measured utilizing techniques well-known to one of skill in the art or described herein, wherein an alteration in the fluorescently polarized light emitted relative to a control or the absence of the compound or the member of a library of compounds indicates that the compound or member of a library of compounds modulates animalia or fungal tRNA splicing ligase activity.

Further, the effect of a compound on the activity of a component of an animalia or fungal tRNA splicing pathway (*e.g.,* an animalia or fungal tRNA splicing endonuclease, or an animalia or fungal tRNA splicing ligase) may be determined utilizing a FISH assay. In particular, a FISH assay may be used to measure the accumulation of products of the reactions of an animalia or fungal tRNA splicing endonuclease, or an animalia or fungal tRNA splicing ligase. Other assays such as gel electrophoresis-based assays that measure the products of the reactions of an animalia or fungal tRNA splicing endonuclease, or an animalia or fungal tRNA splicing ligase can be used to assess the effect of a compound on the activity of a component of an animalia or fungal tRNA splicing pathway (*e.g.*, an animalia or fungal tRNA splicing endonuclease, or an animalia or fungal tRNA splicing ligase).

The assays of the present invention can be performed using different incubation times. In a cell-free system, the cell-free extract or the purified enzyme in the tRNA splicing pathway (*e.g.*, purified tRNA splicing endonuclease) and substrate for the enzyme (*e.g.*, the substrate for a tRNA splicing endonuclease) can be incubated together before the addition of a compound or a member of a library of compounds. In certain embodiments, the cell-free extract or the purified enzyme in the tRNA splicing pathway (*e.g.*, purified tRNA splicing endonuclease) are incubated with a substrate for the enzyme in the tRNA splicing pathway before the addition of a compound or a member of a library of compounds for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day. In other embodiments, a cell-free extract or purified enzyme in the tRNA splicing pathway, or a substrate for the enzyme a tRNA splicing pathway is incubated with a compound or a member of a library of compounds before the addition of the substrate, or the cell-free extract or the purified enzyme in tRNA splicing pathway, respectively. In certain embodiments, a compound or a member of a library of compounds is incubated with a substrate for an enzyme in a tRNA splicing pathway or cell-free extract or purified enzyme in the tRNA splicing pathway before the addition of the remaining component, i. e., cell-free extract or purified enzyme, or substrate, respectively, is at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day. Once the reaction vessel comprises the three components, *i.e.*, a compound or a member of a library of compounds, the cell-free extract or the purified enzyme in a tRNA splicing pathway, and substrate for an enzyme in a tRNA splicing pathway, the reaction may be further incubated for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day.

The progress of the reaction can be measured continuously. For example, if a substrate for animalia tRNA splicing endonuclease or subunits of animalia tRNA splicing endonuclease are labeled with fluorophore(s), the progress of the reaction can be monitored continuously using a fluorescence emission detector such as a Viewlux or Analyst. Alternatively, time-points may be taken at different times of the reaction to monitor the progress of the reaction.

Certain assays of the present invention, such as the tRNA endonuclease suppression assay and the cell-based assays, are indirect assays for compounds that affect a component of an animalia or fungal tRNA splicing pathway and may detect compounds that affect another aspect of the tRNA splicing pathway. In order to confirm or ensure that a compound is a modulator of a particular component of an animalia or fungal tRNA splicing pathway (*e.g.*, tRNA splicing endonuclease or tRNA splicing ligase), any assay that measures the direct effect of the compound on the activity of the component (*e.g.*, tRNA splicing endonuclease or tRNA splicing ligase activity) can be performed. Such assays include assays using a purified enzyme (*e.g.*, a purified tRNA splicing endonuclease or purified tRNA splicing ligase) and are described below.

The compounds utilized in the assays described herein may be members of a library of compounds. In specific embodiment, the compound is selected from a combinatorial library of compounds comprising peptoids; random biooligomers; diversomers such as hydantoins, benzodiazepines and dipeptides; vinylogous polypeptides; nonpeptidal peptidomimetics; oligocarbamates; peptidyl phosphonates; peptide nucleic acid libraries; antibody libraries; carbohydrate libraries; and small organic molecule libraries. In a preferred embodiment, the small organic molecule libraries are libraries of benzodiazepines, isoprenoids, thiazolidinones, metathiazanones, pyrrolidines, morpholino compounds, or diazepindiones.

In certain embodiments, the compounds are screened in pools. Once a positive pool has been identified, the individual compounds of that pool are tested separately. In certain embodiments, the pool size is at least 2, at least 5, at least 10, at least 25, at least 50, at least 75, at least 100, at least 150, at least 200, at least 250, or at least 500 compounds.

Once a compound that modulates the activity of a tRNA splicing endonuclease is identified, the structure of the compound may be determined utilizing well-known techniques or by referring to a predetermined code. For example, the structure of the compound may be determined by mass spectroscopy, NMR, vibrational spectroscopy, or X-ray crystallography.

A compound identified in accordance with the methods of the invention may directly bind to a component of an animalia or fungal tRNA splicing pathway (*e.g.*, the tRNA splicing endonuclease or tRNA splicing ligase). Alternatively, a compound identified in accordance with the methods of invention may bind to a substrate for an enzyme in an animalia or fungal tRNA splicing pathway. A compound identified in accordance with the methods of the invention may bind to an intron. A compound identified in accordance with the methods of invention may also disrupt an interaction between a tRNA intron and a tRNA splicing endonuclease. Further, a compound identified in accordance with the methods of the invention may disrupt the interaction between the tRNA mature domain and the tRNA splicing endonuclease. In some embodiments, a compound identified in accordance with the methods of the invention can inhibit or reduce the interaction between subunits of an endonuclease splicing enzyme, in particular a fungal endonuclease splicing enzyme. In a specific embodiments, a compound identified in accordance with the methods of the invention can inhibit or reduce the interaction between the loop 10 of the 54KDa and the 15KDa subunit of a fungal endonuclease splicing enzyme.

In a preferred embodiment, a compound identified in accordance with the methods of the invention inhibits or reduces animalia tRNA splicing endonuclease activity. In another preferred embodiment, a compound identified in accordance with the methods of the invention preferentially inhibits or reduces animalia tRNA splicing endonuclease activity. In another preferred embodiment, a compound identified in accordance with the methods of the invention inhibits or reduces fungal tRNA splicing endonuclease activity. In another preferred embodiment, a compound identified in accordance with the methods of the invention preferentially inhibits or reduces fungal tRNA splicing endonuclease activity.

In a preferred embodiment, a compound identified in accordance with the methods of the invention inhibits or reduces animalia tRNA splicing ligase activity. In another preferred embodiment, a compound identified in accordance with the methods of the invention inhibits preferentially inhibits or reduces animalia tRNA splicing ligase activity. In another preferred embodiment, a compound identified in accordance with the methods of the invention inhibits or reduces fungal tRNA splicing ligase activity. In another preferred embodiment, a compound identified in accordance with the methods of the invention preferentially inhibits or reduces fungal tRNA splicing ligase activity.

In certain embodiments of the invention, the compound identified using the assays described herein is a small molecule. In a specific embodiment, the compound identified using the assays described herein is not known to affect the activity of an animalia or fungal tRNA splicing endonuclease. In another embodiment, the compound identified using the assays described herein is not known to affect the activity of an animalia or fungal tRNA splicing ligase. In another embodiment, the compound identified using the assays described herein has not been used as or suggested to be an anti-proliferative agent or an antifungal agent.

The compounds identified utilizing the methods of the invention may affect one or more functions of the endonuclease and/or ligase which in turn modulates the activity of the enzyme. Thus, for example, the compounds identified in accordance with the methods of the invention may affect the recognition and/or cleavage of a substrate by a tRNA splicing endonuclease and thus, the cleavage of the substrate.

A compound that modulates the activity of a tRNA splicing endonuclease described herein may be tested in *in vitro* assays or *in vivo* assays (*e.g.*, cell-based assays or cell-free assays) well-known to one of skill in the art or described herein for the effect of said compound on mRNA translation. The compounds identified by the methods of the present invention can be screened for their effect on the production of mature tRNA from any of the 28 intron containing human pre-tRNAs. *In vitro* and *in vivo* assays well-known to one of skill in the art or described herein may be used to determine the antiproliferative effect of a particular compound on hyperproliferative cells versus normal cells. Further, a particular compound identified utilizing the assays described herein may be tested in an animal model for cancer to determine the efficacy of the compound in the prevention, treatment, management or amelioration of cancer or a symptom thereof. In addition, the specificity of a compound identified utilizing the assays described herein may be tested for its specificity for tRNA splicing endonuclease or tRNA splicing ligase and its effect on tRNA splicing endonuclease or tRNA splicing ligase from different species (*e.g.*, its effect on fungal versus animalia tRNA splicing endonuclease).

The invention provides for methods for preventing, treating, managing or ameliorating a proliferative disorder or a symptom thereof, said method comprising administering to a subject in need thereof a therapeutically or prophylactically effective amount of a compound, or a pharmaceutically acceptable salt thereof, identified according to the methods described herein that inhibits or reduces the activity of a component an animalia tRNA splicing pathway (in particular, an animalia tRNA splicing endonuclease or an animalia tRNA splicing ligase). In particular, the invention provides for a method of preventing, treating, managing or ameliorating cancer or a symptom thereof, said method comprising administering to a subject in need thereof an effective amount of a compound, or a pharmaceutically acceptable salt thereof, identified according to the methods described herein that inhibits or reduces the activity of a component an animalia tRNA splicing pathway (in particular, an animalia tRNA splicing endonuclease or an animalia tRNA splicing ligase). The invention also provides methods for preventing, treating, managing or ameliorating a proliferative disorder or a symptom thereof (*e.g.*, cancer, psoriasis or pulmonary fibrosis), said method comprising administering to a subject in need thereof an effective amount of a compound, or a pharmaceutically acceptable salt thereof, identified according to the methods described herein that inhibits or reduces the activity of a component of both the animalia and fungal tRNA splicing pathway (in particular, an animalia and fungal tRNA splicing endonuclease and/or an animalia and fungal tRNA splicing ligase).

Without being bound by theory, compounds that target the tRNA splicing pathway should only be toxic to highly proliferative transformed, malignant cells, while allowing for normal cellular growth and metabolism because not all tRNAs require splicing and tRNA splicing occurs more frequently in proliferating cells. There are only a handful of tRNA species that require removal of intronic sequences (Trotta, C.R. and Abelson, J.N. tRNA Splicing: An RNA World Add-On or an Ancient Reaction? In RNA World II, Tom Cech, Ray Gesteland and John Atkins (eds.), Cold Spring Harbor Laboratory Press (1999)). The current version of the sequence of the human genome has identified 648 tRNA species. Of these, only 28 contain an intron that must be removed by the tRNA splicing endonuclease. The 28 intron containing tRNAs encode 8 different isoaccepting groups. Seven of these isoaccepting groups contain redundant, non-intron-containing versions or can be decoded due to wobble rules of the codon-anticodon interaction (Bjork, G. Biosynthesis and Function of modified Nucleoside in tRNA: Structure, Biosynthesis and Function, D. Soll and V. RayBhandary (eds.), American Society for Microbiology, Washington D.C. (1995). Thus, this leaves one tRNA as a potential limiting component upon inhibition of tRNA splicing. By targeting the tRNA splicing endonuclease, an enzyme dedicated to removal of tRNA introns, the inhibition of tRNA production is fine-tuned to a very few essential tRNA molecules (potentially only a single tRNA). Thus, by inhibiting this process, a very mild toxicity, if any, to normal cells will be produced, while the ability of rapidly proliferating transformed cells to divide will be reduced or ablated as a result of the loss in translational capacity.

The invention provides for methods for preventing, treating, managing or ameliorating a fungal infection or a symptom thereof, said method comprising administering to a subject in need thereof a therapeutically or prophylactically effective amount of a compound, or a pharmaceutically acceptable salt thereof, identified according to the methods described herein that inhibits or reduces the activity of a component a fungal tRNA splicing pathway (in particular, a fungal tRNA splicing endonuclease or a fungal tRNA splicing ligase). In particular, the invention provides for a method of preventing, treating, managing or ameliorating a yeast infection or a symptom thereof, said method comprising administering to a subject in need thereof an effective amount of a compound, or a pharmaceutically acceptable salt thereof, identified according to the methods described herein that inhibits or reduces the activity of a component a fungal tRNA splicing pathway (in particular, a fungal tRNA splicing endonuclease or a fungal tRNA splicing ligase). The invention also provides for methods of disinfecting objects or rooms by using a compound, or a pharmaceutically acceptable salt thereof, identified according to the methods described herein that inhibits or reduces the activity of a component a fungal tRNA splicing pathway (in particular, a fungal tRNA splicing endonuclease or a fungal tRNA splicing ligase).

The invention further provides methods for verifying or confirming the ability of a compound to modulate the activity of a component of an animalia or fungal tRNA splicing pathway (*e.g.*, an animalia tRNA splicing endonuclease, a fungal tRNA splicing endonuclease, an animalia tRNA splicing ligase or a fungal tRNA splicing ligase). The ability of a compound to modulate the activity of a component of an animalia or fungal tRNA splicing pathway (*e.g.*, an animalia tRNA splicing endonuclease, a fungal tRNA splicing endonuclease, an animalia tRNA splicing ligase or a fungal tRNA splicing ligase) can be verified or confirmed utilizing one of the assays described herein to identify such a compound.

## 3.1 <u>TERMINOLOGY</u>

As used herein, the term "biologically active fragment" in the context of an enzyme in a tRNA splicing pathway or a subunit thereof refers to a fragment that retains the activity of the enzyme or subunit. Thus, for example, a biological active fragment of a ligase retains ligase activity and a biological active fragment of an endonuclease or subunit thereof retains the ability to recognize and cleave its substrate.

As used herein, the term "compound" refers to any agent or complex that is being tested for its ability to modulate the activity of a component (*e.g.*, an enzyme) in a tRNA splicing pathway or has been identified as modulating the activity of a component (*e.g.*, an enzyme) in a tRNA splicing pathway (*e.g.*, tRNA splicing endonuclease and/or tRNA splicing ligase).

As used herein, the terms "disorder" and "disease" refer to a condition in a subject, including, without limitation, a proliferative disorder (*e.g.*, cancer) and a fungal infection.

As used herein, the term "effective amount" refers to the amount of a compound which is sufficient to reduce or ameliorate the progression, severity, and/or duration of a disease or disorder (*e.g.*, a proliferative disorder or a fungal infection) or one or more symptoms thereof, prevent the advancement of a disease or disorder (*e.g.*, a proliferative disorder or a fungal infection), cause the regression of a disease or disorder (*e.g.*, a proliferative disorder), prevent the recurrence, development or onset of one or more symptoms associated with a disease or disorder (*e.g.*, a proliferative disorder), or a fungal infection, or enhance or improve the prophylactic or therapeutic effect(s) of another therapy (*e.g.*, a prophylactic or therapeutic agent).

As used herein, the term "fluorescent acceptor moiety" refers to a fluorescent compound that absorbs energy from a fluorescent donor moiety and re-emits the transferred energy as fluorescence. Examples of fluorescent acceptor moieties include, but are not limited to, coumarins and related fluorophores, xanthenes (*e.g.*, fluoresceins, rhodols, and rhodamines), resorufins, cyanines, difluoroboradiazindacenes and phthalocyanines.

As used herein, the term "fluorescent donor moiety" refers to a fluorescent compound that can absorb energy and is capable of transferring the energy to an acceptor, such as another fluorescent compound. Examples of fluorescent donor moieties include, but are not limited to, coumarins and related dyes, xanthene dyes (*e.g.*, fluoresceins, rhodols and rhodamines), resorufins, cyanine dyes, bimanes, acridines, isoindoles, dansyl dyes, aminophthalic hydrazides (*e.g.*, luminol and isoluminol derivatives), aminophthalimides, aminonaphthalimides, aminobenzofurans, aminoquinolines, dicyanohydroquinones, fluorescent europium, terbium complexes and related compounds.

As used herein, the term "fluorophore" refers to a chromophore that fluoresces.

As used herein, the term "purified," in the context of a compound, *e.g.*, a compound identified in accordance with the method of the invention, refers to a compound that is substantially free of chemical precursors or other chemicals when chemically synthesized. In a specific embodiment, the compound is 60%, preferably 65%, 70%, 75%, 80%, 85%, 90%, or 99% free of other, different compounds. In a preferred embodiment, a compound identified in accordance with the methods of the invention is purified.

As used herein, the term "purified," in the context of a proteinaceous agent (*e.g.*, a peptide, polypeptide, or protein, such as a tRNA splicing endonuclease or subunit thereof) refers to a proteinaceous agent which is substantially free of cellular material or contaminating proteins from the cell or tissue source from which it is derived, or substantially free of chemical precursors or other chemicals when chemically synthesized. The language "substantially free of cellular material" includes preparations of a proteinaceous agent in which the proteinaceous agent is separated from cellular components of the cells from which it is isolated or recombinantly produced. Thus, a proteinaceous agent that is substantially free of cellular material includes preparations of a proteinaceous agent having less than about 30%, 20%, 10%, or 5% (by dry weight) of heterologous protein, polypeptide, peptide, or antibody (also referred to as a "contaminating protein"). When the proteinaceous agent is recombinantly produced, it is also preferably substantially free of culture medium, *i.e.,* culture medium represents less than about 20%, 10%, or 5% of the volume of the protein preparation. When the proteinaceous agent is produced by chemical synthesis, it is preferably substantially free of chemical precursors or other chemicals, *i.e.,* it is separated from chemical precursors or other chemicals which are involved in the synthesis of the proteinaceous agent. Accordingly, such preparations of a proteinaceous agent have less than about 30%, 20%, 10%, 5% (by dry weight) of chemical precursors or compounds other than the proteinaceous agent of interest. Preferably, proteinaceous agents disclosed herein are isolated.

As used herein, the term "host cell" includes a particular subject cell transfected with a nucleic acid molecule and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

As used herein, the terms "manage", "managing", and "management" refer to the beneficial effects that a subject derives from a therapy (*e.g.*, a prophylactic or therapeutic agent), which does not result in a cure of the disease or disorder. In certain embodiments, a subject is administered one or more therapies to "manage" a disease or disorder so as to prevent the progression or worsening of the disease or disorder.

As used herein, the term "in combination" refers to the use of more than one therapies (*e.g.*, prophylactic and/or therapeutic agents). The use of the term "in combination" does not restrict the order in which therapies (*e.g.*, prophylactic and/or therapeutic agents) are administered to a subject with a disease or disorder (*e.g.*, a proliferative disorder or a fungal infection). A first therapy (*e.g.*, a prophylactic or therapeutic agent such as a compound identified in accordance with the methods of the invention) can be administered prior to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g.*, a second prophylactic or therapeutic agent such as a chemotherapeutic agent, a TNF-$\alpha$ antagonist or a well-known anti-fungal agent) to a subject with a disease or disorder (*e.g.*, a proliferative disorder or a fungal infection).

As used herein, the term "library" refers to a plurality of compounds. A library can be a combinatorial library, *e.g.*, a collection of compounds synthesized using combinatorial chemistry techniques, or a collection of unique chemicals of low molecular weight (less than 1000 daltons) that each occupy a unique three-dimensional space.

As used herein, the term "ORF" refers to the open reading frame of a mRNA, *i.e.*, the region of the mRNA that is translated into protein.

As used herein, the terms "non-responsive" and refractory" describe patients treated with a currently available therapy (*e.g.*, a prophylactic or therapeutic agent) for a disease or disorder (*e.g.*, a proliferative disorder such as a cancer or a fungal infection), which is not clinically adequate to relieve one or more symptoms associated with such disease or disorder. Typically, such patients suffer from severe, persistently active disease and require additional therapy to ameliorate the symptoms associated with their disease or disorder (*e.g.*, a proliferative disorder or fungal infection).

As used herein, the phrase "pharmaceutically acceptable salt(s)," includes, but is not limited to, salts of acidic or basic groups that may be present in compounds identified using the methods of the present invention. Compounds that are basic in nature are capable of forming a wide variety of salts with various inorganic and organic acids. The acids that can be used to prepare pharmaceutically acceptable acid addition salts of such basic compounds are those that form non-toxic acid addition salts, *i.e.,* salts containing pharmacologically acceptable anions, including but not limited to sulfuric, citric, maleic, acetic, oxalic, hydrochloride, hydrobromide, hydroiodide, nitrate, sulfate, bisulfate, phosphate, acid phosphate, isonicotinate, acetate, lactate, salicylate, citrate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate (*i.e.,* 1,1'-methylene-bis-(2-hydroxy-3-naphthoate)) salts. Compounds that include an amino moiety may form pharmaceutically acceptable salts with various amino acids, in addition to the acids mentioned above. Compounds that are acidic in nature are capable of forming base salts with various pharmacologically acceptable cations. Examples of such salts include alkali metal or alkaline earth metal salts and, particularly, calcium, magnesium, sodium lithium, zinc, potassium, and iron salts.

As used herein, the term "previously determined reference range" refers to a reference range for a readent measured in a particular assay. In a particular, such a term refers to the reference range for the expression and/or the activity of a reporter gene by a particular cell or in a particular cell-free extract. Each laboratory will establish its own reference range for each particular assay, each cell type and each cell-free extract. In a preferred embodiment, at least one positive control and at least one negative control are included in each batch of compounds analyzed.

As used herein, the terms "prevent", " preventing" and "prevention" refer to the prevention of the recurrence, development, progression or onset of one or more symptoms of a disease or disorder (*e.g.*, a proliferative disorder or a fungal infection) resulting from the administration of one or more compounds identified in accordance the methods of the invention or the administration of a combination of such a compound and a known therapy for a disease or disorder (*e.g.*, a proliferative disorder or a fungal infection).

As used herein, the terms "prophylactic agent" and "prophylactic agents" refer to any agent(s) which can be used in the prevention of a disease or disorder (*e.g.*, a proliferative disorder or fungal infection) or one or more symptoms thereof. In certain embodiments, the term "prophylactic agent" refers to a compound identified in the screening assays described herein. In certain other embodiments, the term "prophylactic agent" does not refer a compound identified in the screening assays described herein and is an agent which is known to be useful to, or has been or is currently being used to prevent or impede the onset, development, progression and/or severity of a disease or disorder (*e.g.*, a proliferative disorder or a fungal infection) or one or more symptoms thereof. Prophylactic agents may be characterized as different types of agents based upon one or more effects that the agents have *in vitro* and/or *in vivo.* For example, an immunomodulatory agent may also be characterized as an anti-angiogenic agent.

As used herein, the phrase "prophylactically effective amount" refers to the amount of a therapy (*e.g.*, prophylactic agent) which is sufficient to result in the prevention of the development, recurrence or onset of a disease or disorder (*e.g.*, a proliferative disorder or fungal infection) thereof, or to enhance or improve the prophylactic effect(s) of another therapy (*e.g.*, a prophylactic agent).

As used herein, the term "quencher" refers to a molecule or a part of a compound that is capable of reducing the emission from a fluorescent moiety. Such reduction includes reducing the light after the time when a photon is normally emitted from a fluorescent moiety.

As used herein, the term "protocol" includes dosing schedules and dosing regiments.

As used herein, the term "small molecules" and analogous terms include, but are not limited to, peptides, peptidomimetics, amino acids, amino acid analogs, polynucleotides, polynucleotide analogs, nucleotides, nucleotide analogs, organic or inorganic compounds (*i.e*,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, organic or inorganic compounds having a molecular weight less than about 100 grams per mole, and salts, esters, and other pharmaceutically acceptable forms of such compounds. Salts, esters, and other pharmaceutically acceptable forms of such compounds are also encompassed.

As used herein, the terms "subject" and "patient" are used interchangeably herein. The terms "subject" and "subjects" refer to an animal, preferably a mammal including a non-primate (*e.g.*, a cow, pig, horse, cat, dog, rat, and mouse) and a primate *(e.g.,* a chimpanzee, a monkey such as a cynomolgous monkey and a human), and more preferably a human. In one embodiment, the subject is refractory or non-responsive to current therapies for a proliferative disorder or a fungal infection. In another embodiment, the subject is a farm animal (*e.g.*, a horse, a cow, a pig, etc.) or a pet *(e.g.,* a dog or a cat). In a preferred embodiment, the subject is a human.

As used herein, the phrase "a substrate for an animalia tRNA splicing endonuclease" refers to any nucleotide sequence recognized and excised by an animalia tRNA splicing endonuclease. For example, a nucleotide sequence comprising a bulge-helix-bulge structure or a mature domain of a precursor tRNA may be utilized as a substrate for an animalia tRNA splicing endonuclease in an assay described herein. A nucleotide sequence recognized and excised by an animalia tRNA splicing endonuclease may comprise 10 nucleotides, 15 nucleotides, 20 nucleotides, 25 nucleotides, 25 nucleotides, 30 nucleotides, 40 nucleotides, 45 nucleotides, 50 nucleotides, 55 nucleotides, 60 nucleotides, 65 nucleotides, 75 nucleotides, 100 nucleotides, 125 nucleotides, 150 nucleotides, or more. In a specific embodiment, the substrates for a tRNA splicing endonuclease utilized in the assays described herein comprise a tRNA intron. The substrate may comprise a mature domain or a precursor tRNA or a bulge-helix-bulge conformation. In a preferred embodiment, the substrate comprises a mature domain of a precursor tRNA.

A substrate for an animalia tRNA endonuclease may be produced by any method well-known to one of skill in the art. For example, the substrate may be chemically synthesized using phosphoramidite or other solution or solid-phase methods. Detailed descriptions of the chemistry used to form polynucleotides by the phosphoramidite method are well-known *(see, e.g.,* Caruthers et al., U.S. Pat. Nos. 4,458,066 and 4,415,732; Caruthers et al., 1982, Genetic Engineering 4:1-17; Users Manual Model 392 and 394 Polynucleotide Synthesizers, 1990, pages 6-1 through 6-22, Applied Biosystems, Part No. 901237; Ojwang, et al.,1997, Biochemistry, 36:6033-6045). After synthesis, the substrate can be purified using standard techniques known to those skilled in the art *(see* Hwang et al., 1999, Proc. Natl. Acad. Sci. USA 96(23):12997-13002 and references cited therein). Depending on the length of the substrate and the method of its synthesis, such purification techniques include, but are not limited to, reverse-phase high-performance liquid chromatography ("reverse-phase HPLC"), fast performance liquid chromatography ("FPLC"), and gel purification.

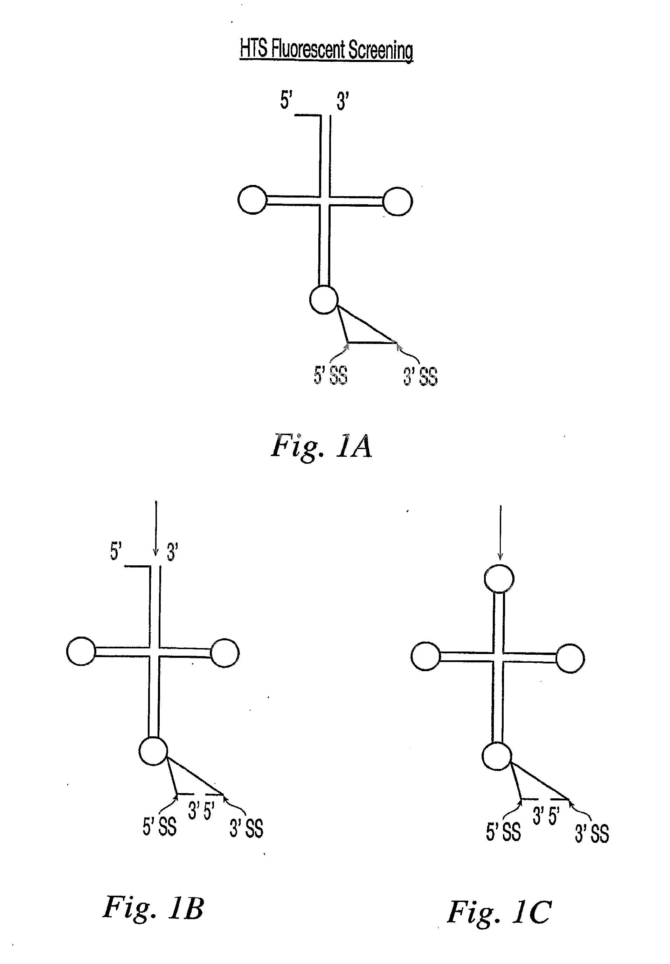

In a specific embodiment, the substrates depicted in Figure 1 are utilized in the *in vitro* assay described herein. To generate the hybridized tRNA substrate, both strands of the hybridized tRNA substrate are transcribed separately and the two strands are subsequently hybridized by heating and cooling. For synthesis of the circularly permuted tRNA substrate, the RNA is transcribed from the 5' end in the intron (Figure 1 C) to the 3' end in the intron.

As used herein, the term "synergistic" refers to a combination of therapies (*e.g.*, a compound identified using one of the methods described herein) and another therapy (*e.g.*, another agent) which has been or is currently being used to prevent, treat or manage a disease or disorder (*e.g.*, a proliferative disorder or a fungal infection), which is more effective than the additive effects of any two or more single therapies. A synergistic effect of a combination of therapies (*e.g.*, prophylactic or therapeutic agents) permits the use of lower dosages of one or more of the therapies (*e.g.*, agents) and/or less frequent administration of said therapies (*e.g.*, agents) to a subject with a disease or disorder (*e.g.,* proliferative disorder or a fungal infection). The ability to utilize lower dosages of a therapy (*e.g.*, a prophylactic or therapeutic agent) and/or to administer said therapy (*e.g.,* agent) less frequently reduces the toxicity associated with the administration of said therapy (agent) to a subject without reducing the efficacy of said therapies (*e.g.*, agents) in the prevention, treatment or management of a disease or disorder (*e.g.*, a proliferative disorder or a fungal infection). In addition, a synergistic effect can result in improved efficacy of therapies (*e.g.*, agents) in the prevention, treatment or management of a disease or disorder (*e.g.*, a proliferative disorder or a fungal infection). Finally, a synergistic effect of a combination of therapies (*e.g.*, prophylactic or therapeutic agents) may avoid or reduce adverse or unwanted side effects associated with the use of either therapy (*e.g.*, agent) alone.

As used herein, the terms "therapeutic agent" and "therapeutic agents" refer to any agent(s) which can be used in the prevention, treatment, management or amelioration of disease or disorder (*e.g.*, a proliferative disorder or a fungal infection) or one or more symptoms thereof. In certain embodiments, the term "therapeutic agent" refers to a compound identified in the screening assays described herein. In other embodiments, the term "therapeutic agent" does not refer to a compound identified in the screening assays described herein and is an agent which is known to be useful for, or has been or is currently being used for the prevention, treatment, management or amelioration of a disease or disorder (*e.g.*, a proliferative disorder or a fungal infection) or one or more symptoms thereof. Therapeutic agents may be characterized as different types of agents based upon one or more effects of the agents have *in vivo* and/or *in vitro.* For example, an antiinflammatory agent may be also characterized as an immunomodulatory agent.

As used herein, the term "therapeutically effective amount" refers to that amount of a therapy (*e.g.*, a therapeutic agent) which is sufficient to reduce the severity of a disease or disorder or one or more symptoms thereof, reduce the duration of a disease or disorder, ameliorate one or more symptoms of a disease or disorder, prevent advancement of a disease or disorder, cause regression of a disease or disorder, or enhance or improve the therapeutic effect(s) of another therapy. In specific embodiments, with respect to the treatment of cancer, a therapeutically effective amount refers to the amount of a therapy (e.g., therapeutic agent) that inhibits or reduces the proliferation of cancerous cells, inhibits or reduces the spread of tumor cells (metastasis), inhibits or reduces the onset, development, progression or recurrence of cancer or of one or more symptoms thereof, or reduces the size of a tumor. In a preferred embodiment, a therapeutically effective of a therapy (*e.g.*, a therapeutic agent) reduces the proliferation of cancerous cells or the size of a tumor by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 90% relative to a control (*e.g.*, phosphate buffered saline ("PBS")). In other embodiments, a therapeutically effective amount reduces the replication of a fungus, or reduces the spread of a fungus to other organs or tissues in a subject or to other subjects. In a preferred embodiment, a therapeutically effective amount reduces the replication of a fungus by at least 5% preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 98% relative to a control such as PBS.

As used herein, the terms "therapy" and "therapies" refer to any method, protocol and/or agent that can be used in the prevention, treatment, management or amelioration of a disease or disorder (*e.g.*, a proliferative disorder or a fungal infection) or one or more symptoms thereof. In certain embodiments, such terms refer to antifungal therapy, biological therapy, chemotherapy, radiation therapy, supportive therapy and/or other therapies useful in the prevention, treatment, management or amelioration of a disease or disorder (*e.g.*, a proliferative disorder or a fungal infection) or one or more symptoms thereof known to skilled medical personnel.

As used herein, the terms "treat", "treatment" and "treating" refer to the reduction or amelioration of the severity, duration and/or progression of a disease or disorder or one or more symptoms thereof resulting from the administration of one or more therapies (*e.g.*, one or more compounds identified in accordance the methods of the invention). In specific embodiments, such terms refer to the inhibition or reduction in the proliferation of cancerous cells, the inhibition or reduction the spread of tumor cells (metastasis), the inhibition or reduction in the onset, development or progression of cancer or thereof one or more symptoms, or the reduction in the size of a tumor. In other embodiments, such terms refer to a reduction in the replication of a fungus, or a reduction in the spread of a fungus to other organs or tissues in a subject or to other subjects.

As used herein, the term "tRNA intron" refers to any nucleotide sequence recognized and excised by an animalia and/or fungal tRNA splicing endonuclease. In particular, the term "tRNA intron" refers to an intron typically found in a precursor tRNA.

As used herein, the term "tRNA splicing endonuclease" refers to the enzyme that is responsible for the recognition of the splice sites contained in precursor tRNA and the cleavage of the introns present in precursor tRNA. The archaeal tRNA splicing endonuclease recognizes the bulge-helix-bulge motif in archaeal precursor tRNA. The eukaryotic tRNA splicing endonuclease recognizes the splice sites contained in precursor tRNA by measuring the distance from the mature domain to the splice sites. The eukaryotic tRNA splicing endonuclease also has the capacity to recognize a bulge-helix-bulge motif contained in precursor tRNA. The yeast tRNA endonuclease is a heterotetramer comprising subunits having the molecular masses of 54 kDa (SEN54), 44 kDa (SEN2), 34 kDa (SEN 34), and 15 kDa (SEN 15). The human homologs of the SEN2 and SEN34 subunits have been identified and the amino acid sequences can be found in GenBank under accession numbers NP_079541 and XP_085899, respectively. In a specific embodiment, the tRNA splicing endonuclease utilized in the assays described herein is derived from or encodes an animal tRNA splicing endonuclease (preferably, a mammalian tRNA splicing endonuclease). In another embodiment, the tRNA splicing endonuclease utilized in the assays described herein is derived from or encodes a fungal tRNA splicing endonuclease (e.g., a yeast tRNA splicing endonuclease). In a particular embodiment, the tRNA splicing endonuclease utilized in the assays described herein is a human tRNA splicing endonuclease.

As used herein, the term "tRNA splicing endonuclease extract" refers to an extract from a cell containing tRNA splicing endonuclease activity. In certain embodiments, a tRNA splicing endonuclease extract is a cell-extract containing tRNA splicing endonuclease activity and the components necessary for the transcription and translation of a gene.

As used herein, the terms "tRNA half molecules" and "a substrate for a tRNA splicing ligase" refer to the product formed following the cleavage of a substrate for tRNA splicing endonuclease by tRNA splicing endocuclease. tRNA half molecules can be synthesized or purified/isolating utilizing techniques well-known to one of skill in the art.

As used herein, the term "tRNA splicing ligase extract" refers to an extract from a cell containing tRNA splicing ligase activity. In certain embodiments, a tRNA splicing ligase extract is a cell-extract containing tRNA splicing ligase activity and the components necessary for the transcription and translation of a gene.

Abbreviation

HTS        High Throughput Screen

FP         fluorescence polarization

FRET       Fluorescence Resonance Energy Transfer

HPLC       high-performance liquid chromatography

FPLC       fast performance liquid chromatography

FACS       Fluorescence activated cell sorter

## 4. <u>BRIEF DESCRIPTION OF THE FIGURES</u>

Figure 1: tRNA Splicing Endonuelease Substrates for HTS Fluorescent screening. The endogenous tRNA is shown in panel A: the hybridized tRNA substrate is shown in panel B; and the circularly permuted tRNA substrate is shown in panel C. The 5' ss designates the 5' splice site and 3' ss designates the 3' splice site.

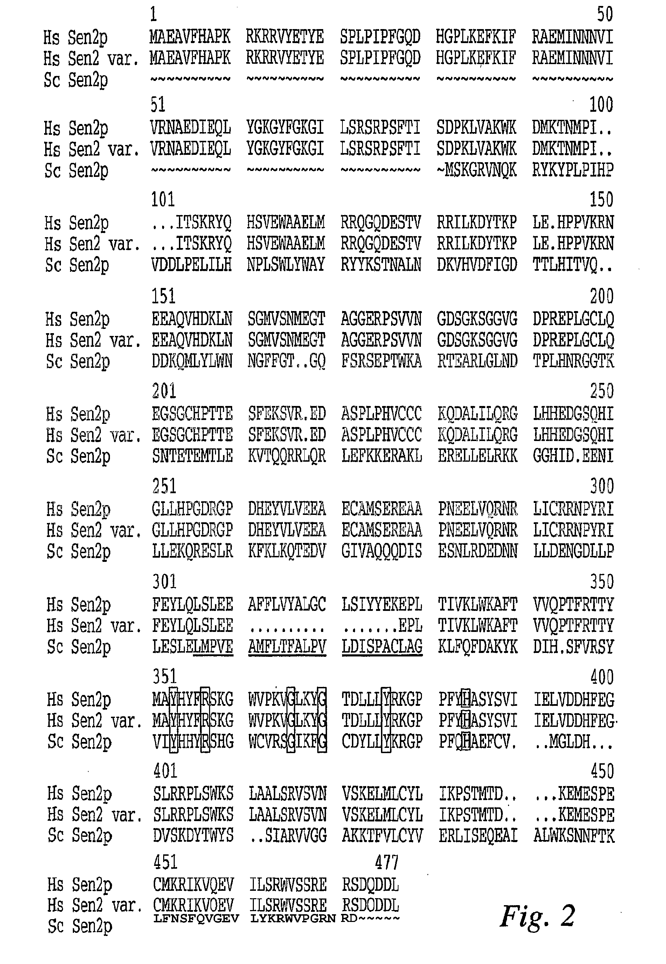

Figure 2: Amino acid Sequence Alignment of HsSen2 (SEQ. ID. NO.: 1) and HsSen2 var (SEQ. ID. NO.: 2); human and yeast (ScSen2p (SEQ. ID. NO.: 3) tRNA splicing endonuclease Sen2 Subunit. The boxed amino acid residues indicate the YRGGY (SEQ ID NO.: 4) active site motif, the circled amino acid residues indicates the active site histidine, and the underlined amino acid residues indicate the yeast putative transmembrane domain.

## 5. DETAILED DESCRIPTION OF THE INVENTION

The present invention provides methods of identifying compounds that affect the activity of one or more components of the tRNA splicing pathway. In particular, the invention provides methods for identifying compounds that modulate the activity of an animalia and/or fungal tRNA splicing endonuclease and/or an animalia and/or fungal tRNA splicing ligase. Examples of assays which can be used to identify compounds that modulate; the activity of an animalia and/or fungal tRNA splicing endonuclease and/or an animalia and/or fungal tRNA splicing ligase include, but are not limited to, reporter-based assays, FRET assays, fluorescence polarization assays and FISH assays. Such assays either directly or indirectly assess the effect of a compound on an animalia and/or fungal tRNA splicing endonuclease and/or an animalia and/or fungal tRNA splicing ligase. Compounds identified as modulating the activity of the endonuclease or ligase are preferably tested for their specificity for the endonuclease or ligase, respectively, and their effect on such enzymes from different species. The structure of a compound that modulates the activity of an animalia and/or fungal tRNA splicing endonuclease or an animalia and/or fungal tRNA splicing ligase can be determined utilizing techniques well-known in the art or described herein.

Preferably, a compound identified in accordance with the methods of the invention inhibitor reduces the activity of either a tRNA splicing endonuclease or a tRNA splicing ligase and exhibits species specificity. Thus, in one embodiment, a compound identified in accordance with the methods of the invention inhibits or reduced the activity of a fungal tRNA splicing endonuclease without inhibiting or reducing the activity of an animalia tRNA splicing endonuclease. In another embodiment, a compound identified in accordance with the methods of the invention inhibits or reduces the activity of an animalia tRNA splicing endonuclease without inhibiting or reducing the activity of a fungal tRNA splicing endonuclease. In another embodiment, a compound identified in accordance with the methods of the invention inhibits or reduces the activity of a fungal tRNA splicing ligase without inhibiting or reducing the activity of an animalia tRNA splicing ligase. In yet another embodiment, a compound identified in accordance with the methods of the invention inhibits or reduces the activity of an animalia tRNA splicing ligase without inhibiting or reducing the activity of a fungal tRNA splicing ligase.

The compounds identified in accordance with the methods of the invention that differentially inhibitor reduce the activity of a component (*e.g.* enzyme) in a fungal tRNA splicing pathway can be used to prevent, treat, manage or ameliorate a fungal infection or a symptom thereof. In particular, the compounds identified in accordance with the methods of the invention that inhibit or reduce the activity of a fungal tRNA splicing endonuclease and/or a fungal tRNA splicing ligase without adversely affecting animalia cells and animals can be used to prevent, treat, manage or ameliorate a fungal infection or one or more symptoms thereof. The compounds identified in accordance with the methods of the invention that inhibit or reduce an animalia tRNA splicing endonuclease and/or an animalia tRNA splicing ligase can be used to prevent, treat, manage or ameliorate a proliferate disorder or one or more symptoms thereof, even if such compounds also exhibit some inhibitory effect on a fungal tRNA splicing endonuclease and/or a fungal tRNA splicing ligase. Moreover, in circumstances where the beneficial effect(s) of a compound that inhibits or reduces a fungal tRNA splicing endonuclease and/or a fungal tRNA splicing ligase with some minor or negligible inhibitory effect on an animalia tRNA splicing endonuclease and/or an animalia tRNA splicing ligase outweighs the side effect(s) on animalia cells and animals, such a compound may be used to prevent, treat, manage or ameliorate a fungal infection or a symptom thereof.

### 5.1 REPORTER GENE CONSTRUCTS, TRANSFECTED CELLS AND CELL EXTRACTS

The invention provides for specific vectors comprising a reporter gene comprising a tRNA intron operably linked to one or more regulatory elements and host cells transfected with the vectors. The invention also provides for the *in vitro* translation of a reporter gene comprising a tRNA intron operably linked to one or more regulatory elements. Techniques for practicing this specific aspect of this invention will employ, unless otherwise indicated, conventional techniques of molecular biology, microbiology, and recombinant DNA manipulation and production, which are routinely practiced by one of skill in the art. *See, e.g.*, Sambrook, 1989, Molecular Cloning, A Laboratory Manual, Second Edition; DNA Cloning, Volumes I and II (Glover, Ed. 1985); Oligonucleotide Synthesis (Gait, Ed. 1984); Nucleic Acid Hybridization (Hames & Higgins, Eds. 1984); Transcription and Translation (Hames & Higgins, Eds. 1984); Animal Cell Culture (Freshney, Ed. 1986); Immobilized Cells and Enzymes (IRL Press, 1986); Perbal, A Practical Guide to Molecular Cloning (1984); Gene Transfer Vectors for Mammalian Cells (Miller & Calos, Eds. 1987, Cold Spring Harbor Laboratory); Methods in Enzymology, Volumes 154 and 155 (Wu & Grossman, and Wu, Eds., respectively), (Mayer & Walker, Eds., 1987); Immunochemical Methods in Cell and Molecular Biology (Academic Press, London, Scopes, 1987), Expression of Proteins in Mammalian Cells Using Vaccinia Viral Vectors in Current Protocols in Molecular Biology, Volume 2 (Ausubel et al., Eds., 1991).

### 5.1.1 REPORTER GENES

Any reporter gene well-known to one of skill in the art may be used in reporter gene constructs to ascertain the effect of a compound on a component (*e.g.*, enzyme) in an animalia or fungal tRNA pathway. Reporter genes refer to a nucleotide sequence encoding a protein that is readily detectable either by its presence or activity. Reporter genes may be obtained and the nucleotide sequence of the elements determined by any method well-known to one of skill in the art. The nucleotide sequence of a reporter gene can be obtained, *e.g.*, from the literature or a database such as GenBank. Alternatively, a polynucleotide encoding a reporter gene may be generated from nucleic acid from a suitable source. If a clone containing a nucleic acid encoding a particular reporter gene is not available, but the sequence of the reporter gene is known, a nucleic acid encoding the reporter gene may be chemically synthesized or obtained from a suitable source (*e.g.*, a cDNA library, or a cDNA library generated from, or nucleic acid, preferably poly A+ RNA, isolated from, any tissue or cells expressing the reporter gene) by PCR amplification. Once the nucleotide sequence of a reporter gene is determined, the nucleotide sequence of the reporter gene may be manipulated using methods well-known in the art for the manipulation of nucleotide sequences, *e.g.*, recombinant DNA techniques, site directed mutagenesis, PCR, etc. (*see,* for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties), to generate reporter genes having a different amino acid sequence, for example to create amino acid substitutions, deletions, and/or insertions.

Examples of reporter genes include, but are not limited to, luciferase (*e.g.*, firefly luciferase, renilla luciferase, and click beetle luciferase), green fluorescent protein ("GFP") (*e.g.*, green fluorescent protein, yellow fluorescent protein, red fluorescent protein, cyan fluorescent protein, and blue fluorescent protein), beta-galactosidase ("b-gal"), beta-glucoronidase, beta-lactamase, chloramphenicol acetyltransferase ("CAT"), and alkaline phosphatase ("AP"). Table 1 below lists various reporter genes and the properties of the products of the reporter genes that can be assayed. In a preferred embodiment, a reporter gene utilized in the reporter constructs is easily assayed and has an activity which is not normally found in the cell or organism of interest.

TABLE 1: Reporter Genes and the Properties of the Reporter Gene Products

| Reporter Gene | Protein Activity & Measurement |
|---|---|
| CAT (chloramphenicol acetyltransferase) | Transfers radioactive acetyl groups to chloramphenicol or detection by thin layer chromatography and autoradiography |
| GAL (b-galactosidase) | Hydrolyzes colorless galactosides to yield colored products. |
| GUS (b-glucuronidase) | Hydrolyzes colorless glucuronides to yield colored products. |
| LUC (luciferase) | Oxidizes luciferin, emitting photons |
| GFP (green fluorescent protein) | fluorescent protein without substrate |
| SEAP (secreted alkaline phosphatase) | luminescence reaction with suitable substrates or with substrates that generate chromophores |
| HRP (horseradish peroxidase) | in the presence of hydrogen oxide, oxidation of 3,3',5,5'-tetramethylbenzidine to form a colored complex |
| AP (alkaline phosphatase) | luminescence reaction with suitable substrates or with substrates that generate chromophores |

Described hereinbelow in further detailed are specific reporter genes and characteristics of those reporter genes.

### 5.1.1.1 Luciferase

Luciferases are enzymes that emit light in the presence of oxygen and a substrate (luciferin) and which have been used for real-time, low-light imaging of gene expression in cell cultures, individual cells, whole organisms, and transgenic organisms (reviewed by Greer & Szalay, 2002, Luminescence 17(1):43-74).

As used herein, the term "luciferase" is intended to embrace all luciferases, or recombinant enzymes derived from luciferases which have luciferase activity. The luciferase genes from fireflies have been well characterized, for example, from the *Photinus* and *Luciola* species (*see, e.g.,* International Patent Publication No. WO 95/25798 for *Photinus pyralis,* European Patent Application No. EP 0 524 448 for *Luciola cruciata* and *Luciola lateralis,* and Devine et al., 1993, Biochim. Biophys. Acta 1173(2):121-132 for *Luciola mingrelica*). Other eucaryotic luciferase genes include, but are not limited to, the click beetle (*Photinus plagiophthalamus, see, e.g.,* Wood et al., 1989, Science 244:700-702), the sea panzy (R*enilla reniformis, see, e.g.,* Lorenz et al., 1991, Proc Natl Acad Sci U S A 88(10):4438-4442), and the glow worm (*Lampyris noctiluca, see e.g.,* Sula-Newby et al., 1996, Biochem J. 313:761-767). The click beetle is unusual in that different members of the species emit bioluminescence of different colors, which emit light at 546 nm (green), 560 nm (yellow-green), 578 nm (yellow) and 593 nm (orange) (*see, e.g,* U.S. Patent Nos. 6,475,719; 6,342,379; and 6,217,847, the disclosures of which are incorporated by reference in their entireties). Bacterial luciferin-luciferase systems include, but are not limited to, the bacterial lux genes of terrestrial *Photorhabdus luminescens* (*see, e.g.,* Manukhov et al., 2000, Genetika 36(3):322-30) and marine bacteria *Vibrio fischeri* and *Vibrio harveyi* (*see, e.g.*, Miyamoto et al., 1988, J Biol Chem. 263(26):13393-9, and Cohn et al., 1983, Proc Natl Acad Sci USA., 80(1):120-3, respectively). The luciferases encompassed by the present invention also include the mutant luciferases described in U.S. Patent No. 6,265,177 to Squirrell et al.*,* which is hereby incorporated by reference in its entirety.

In a preferred embodiment, the luciferase is a firefly luciferase, a renilla luciferase, or a click beetle luciferase, as described in any one of the references listed *supra,* the disclosures of which are incorporated by reference in their entireties.

### 5.1.1.2 Green Fluorescent Protein

Green fluorescent protein ("GFP") is a 238 amino acid protein with amino acid residues 65 to 67 involved in the formation of the chromophore which does not require additional substrates or cofactors to fluoresce (*see, e.g.,* Prasher et al., 1992, Gene 111:229-233; Yang et al., 1996, Nature Biotechnol. 14:1252-1256; and Cody et al., 1993, Biochemistry 32:1212-1218).

As used herein, the term "green fluorescent protein" or "GFP" is intended to embrace all GFPs (including the various forms of GFPs which exhibit colors other than green), or recombinant enzymes derived from GFPs which have GFP activity. In a preferred embodiment, GFP includes green fluorescent protein, yellow fluorescent protein, red fluorescent protein, cyan fluorescent protein, and blue fluorescent protein. The native gene for GFP was cloned from the bioluminescent jellyfish *Aequorea victoria* (*see, e.g.,* Morin et al., 1972, J. Cell Physiol. 77:313-318). Wild type GFP has a major excitation peak at 395 nm and a minor excitation peak at 470 nm. The absorption peak at 470 nm allows the monitoring of GFP levels using standard fluorescein isothiocyanate (FITC) filter sets. Mutants of the GFP gene have been found useful to enhance expression and to modify excitation and fluorescence. For example, mutant GFPs with alanine, glycine, isoleucine, or threonine substituted for serine at position 65 result in mutant GFPs with shifts in excitation maxima and greater fluorescence than wild type protein when excited at 488 nm (*see, e.g.,* Heim et al., 1995, Nature 373:663-664; U.S. Patent No. 5,625,048; Delagrave et al., 1995, Biotechnology 13:151-154; Cormack et al., 1996, Gene 173:33-38; and Cramer et al., 1996, Nature Biotechnol. 14:315-319). The ability to excite GFP at 488 nm permits the use of GFP with standard fluorescence activated cell sorting ("FACS") equipment. In another embodiment, GFPs are isolated from organisms other than the jellyfish, such as, but not limited to, the sea pansy, *Renilla reriformis.*

Techniques for labeling cells with GFP in general are described in U.S. Patent Nos. 5,491,084 and 5,804,387, which are incorporated by reference in their entireties; Chalfie et al., 1994, Science 263:802-805; Heim et al., 1994, Proc. Natl. Acad. Sci. USA 91:12501-12504; Morise et al., 1974, Biochemistry 13:2656-2662; Ward et al., 1980, Photochem. Photobiol. 31:611-615; Rizzuto et al., 1995, Curr. Biology 5:635-642; and Kaether & Gerdes, 1995, FEBS Lett 369:267-271. The expression of GFPs in *E. coli* and *C. elegans* are described in U.S. Patent No. 6,251,384 to Tan et al.*,* which is incorporated by reference in its entirety. The expression of GFP in plant cells is discussed in Hu & Cheng, 1995, FEBS Lett 369:331-33, and GFP expression in *Drosophila* is described in Davis et al., 1995, Dev. Biology 170: 726-729.

### 5.1.1.3 Beta-galactosidase

Beta galactosidase ("b-gal") is an enzyme that catalyzes the hydrolysis of b-galactosides, including lactose, and the galactoside analogs o-nitrophenyl-b-D-galactopyranoside ("ONPG") and chlorophenol red-b-D-galactopyranoside ("CPRG") (*see, e.g.,* Nielsen et al., 1983 Proc Natl Acad Sci USA 80(17):5198-5202; Eustice et al., 1991, Biotechniques 11:739-742; and Henderson et al., 1986, Clin. Chem. 32:1637-1641). The b-gal gene functions well as a reporter gene because the protein product is extremely stable, resistant to proteolytic degradation in cellular lysates, and easily assayed. When ONPG is used as the substrate, b-gal activity can be quantitated with a spectrophotometer or microplate reader.

As used herein, the term "beta galactosidase" or "b-gal" is intended to embrace all b-gals, including *lacZ* gene products, or recombinant enzymes derived from b-gals which have b-gal activity. The b-gal gene functions well as a reporter gene because the protein product is extremely stable, resistant to proteolytic degradation in cellular lysates, and easily assayed. In an embodiment where ONPG is the substrate, b-gal activity can be quantitated with a spectrophotometer or microplate reader to determine the amount of ONPG converted at 420 nm. In an embodiment when CPRG is the substrate, b-gal activity can be quantitated with a spectrophotometer or microplate reader to determine the amount of CPRG converted at 570 to 595 nm. In yet another embodiment, the b-gal activity can be visually ascertained by plating bacterial cells transformed with a b-gal construct onto plates containing Xgal and IPTG. Bacterial colonies that are dark blue indicate the presence of high b-gal activity and colonies that are varying shades of blue indicate varying levels of b-gal activity.

**5.1.1.4 Beta-glucoronidase**

Beta-glucuronidase ("GUS") catalyzes the hydrolysis of a very wide variety of b-glucuronides, and, with much lower efficiency, hydrolyzes some b-galacturonides. GUS is very stable, will tolerate many detergents and widely varying ionic conditions, has no cofactors, nor any ionic requirements, can be assayed at any physiological pH, with an optimum between 5.0 and 7.8, and is reasonably resistant to thermal inactivation (*see, e.g.,* U.S. Patent No. 5,268,463, which is incorporated by reference in its entirety).

In one embodiment, the GUS is derived from the *Esherichia coli* b-glucuronidase gene. In alternate embodiments of the invention, the b-glucuronidase encoding nucleic acid is homologous to the *E. coli* b-glucuronidase gene and/or may be derived from another organism or species.

GUS activity can be assayed either by fluorescence or spectrometry, or any other method described in U.S. Patent No. 5,268,463, the disclosure of which is incorporated by reference in its entirety. For a fluorescent assay, 4-trifluoromethylumbelliferyl b-D-glucuronide is a very sensitive substrate for GUS. The fluorescence maximum is close to 500 mn--bluish green, where very few plant compounds fluoresce or absorb. 4-trifluoromethylumbelliferyl b-D-glucuronide also fluoresces much more strongly near neutral pH, allowing continuous assays to be performed more readily than with MUG. 4-trifluoromethylumbelliferyl b-D-glucuronide can be used as a fluorescent indicator *in vivo.* The spectrophotometric assay is very straightforward and moderately sensitive (Jefferson et al., 1986, Proc. Natl. Acad. Sci. USA 86:844.7-84.51). A. preferred substrate for spectrophotometric measurement is p-nitrophenyl b-D-glucuronide, which when cleaved by GUS releases the chromophore p-nitrophenol. At a pH greater than its $pK_a$ (around 7.15) the ionized chromophore absorbs light at 400-420 nm, giving a yellow color.

**5.1.1.5 Beta-lactamase**

Beta-lactamases are nearly optimal enzymes in respect to their almost diffusion-controlled catalysis of b-lactam hydrolysis, making them suited to the task of an intracellular reporter enzyme *(see, e.g.,* Christensen et al., 1990, Biochem. J. 266: 853-861). They cleave the b-lactam ring of b-lactam antibiotics, such as penicillins and cephalosporins, generating new charged moieties in the process *(see, e.g.,* O'Callaghan et al., 1968, Antimicrob. Agents. Chemother. 8: 57-63 and Stratton, 1988, J. Antimicrob. Chemother. 22, Suppl. A: 23-35). A large number of b-lactamases have been isolated and characterized, all of which would be suitable for use in accordance with the present invention *(see, e.g.,* Richmond & Sykes, 1978, Adv.Microb.Physiol. 9:31-88 and Ambler, 1980, Phil. Trans. R. Soc. Lond. [Ser.B.] 289: 321-331, the disclosures of which are incorporated by reference in their entireties).

The coding region of an exemplary b-lactamase employed has been described in U.S. Patent No. 6,472,205, Kadonaga et al., 1984, J.Biol.Chem. 259: 2149-2154, and Sutcliffe, 1978, Proc. Natl. Acad. Sci. USA 75: 3737-3741, the disclosures of which are incorporated by reference in their entireties. As would be readily apparent to those skilled in the field, this and other comparable sequences for peptides having b-lactamase activity would be equally suitable for use in accordance with the present invention. The combination of a fluorogenic substrate described in U.S. Patent Nos. 6,472,205, 5,955,604, and 5,741,657, the disclosures of which are incorporated by reference in their entireties, and a suitable b-lactamase can be employed in a wide variety of different assay systems, such as are described in U.S. Patent No. 4,740,459, which is hereby incorporated by reference in its entirety.

**5.1.1.6 Chloramphenicol Acetyltransferase**

Chloramphenicol acetyl transferase ("CAT") is commonly used as a reporter gene in mammalian cell systems because mammalian cells do not have detectable levels of CAT activity. The assay for CAT involves incubating cellular extracts with radiolabeled chloramphenicol and appropriate co-factors, separating the starting materials from the product by, for example, thin layer chromatography ("TLC"), followed by scintillation counting *(see, e.g.,* U.S. Patent No. 5,726,041, which is hereby incorporated by reference in its entirety).

As used herein, the term "chloramphenicol acetyltransferase" or "CAT" is intended to embrace all CATS, or recombinant enzymes derived from CAT which have CAT activity, While it is preferable that a reporter system which does not require cell processing, radioisotopes, and chromatographic separations would be more amenable to high throughput screening, CAT as a reporter gene may be preferable in situations when stability of the reporter gene is important. For example, the CAT reporter protein has an *in vivo* half life of about 50 hours, which is advantageous when an accumulative versus a dynamic change type of result is desired.

**5.1.1.7 Secreted Alkaline Phosphatase**

The secreted alkaline phosphatase ("SEAP") enzyme is a truncated form of alkaline phosphatase, in which the cleavage of the transmembrane domain of the protein allows it to be secreted from the cells into the surrounding media.

In a preferred embodiment, the alkaline phosphatase is isolated from human placenta.

As used herein, the term "secreted alkaline phosphatase" or "SEAP" is intended to embrace all SEAP or recombinant enzymes derived from SEAP which have alkaline phosphatase activity. SEAP activity can be detected by a variety of methods including, but not limited to, measurement of catalysis of a fluorescent substrate, immunoprecipitation, HPLC, and radiometric detection. The luminescent method is preferred due to its increased sensitivity over calorimetric detection methods. The advantages of using SEAP is that a cell lysis step is not required since the SEAP protein is secreted out of the cell, which facilitates the automation of sampling and assay procedures. A cell-based assay using SEAP for use in cell-based assessment of inhibitors of the Hepatitis C virus protease is described in U.S. Patent No. 6,280,940 to Potts et al. which is hereby incorporated by reference in its entirety.

### 5.1.2 tRNA INTRONS

Any nucleotide sequence recognized and excised by an animalia or fungal tRNA splicing endonuclease may be inserted into the coding region of a reporter gene such that the mRNA coding the reporter gene is out of frame utilizing well-known molecular biology techniques. For example, a nucleotide sequence comprising a bulge-helix-bulge structure or a mature domain of a precursor tRNA may be inserted into the coding region of a reporter gene such that the mRNA coding the reporter gene is out of frame. Alternatively, a nucleotide sequence recognized and excised by an animalia or fungal tRNA splicing endonuclease may be inserted into the 5' untranslated region, 3' untranslated region or both the 5' and 3' untranslated regions of a reporter gene construct. A nucleotide sequence recognized and excised by an animalia or fungal tRNA splicing endonuclease may comprise 10 nucleotides, 15 nucleotides, 20 nucleotides, 25 nucleotides, 25 nucleotides, 30 nucleotides, 4.0 nucleotides, 45 nucleotides, 50 nucleotides, 55 nucleotides, 60 nucleotides, 65 nucleotides, 75 nucleotides, 100 nucleotides, 125 nucleotides, 150 nucleotides, or more. In certain embodiments, the nucleotide sequence is at least 10 nucleotides in length.

In a specific embodiment, a tRNA intron is inserted within the open reading frame of a reporter gene. In another embodiment, two, three, four, five or more tRNA introns are inserted within the open reading frame of a reporter gene. In an alternative embodiment, a tRNA intron is inserted within the 5' untranslated region, 3' untranslated region or both the 5' and 3' untranslated region of a reporter gene construct. In an alternative embodiment, two, three, four, five or more tRNA introns are inserted within the 5' untranslated region, 3' untranslated region or both the 5' and 3' untranslated region of a reporter gene construct. The tRNA intron may comprise a bulge-helix-bulge conformation.

A reporter gene containing a tRNA intron may be produced by any method well-known to one of skill in the art. For example, the reporter gene containing a tRNA intron may be chemically synthesized using phosphoramidite or other solution or solid-phase methods. Detailed descriptions of the chemistry used to form polynucleotides by the phosphoramidite method are well known (*see, e.g.,* Caruthers et al., U.S. Pat. Nos. 4,458,066 and 4,415,732; Caruthers et al., 1982, Genetic Engineering 4:1-17; Users Manual Model 392 and 394 Polynucleotide Synthesizers, 1990, pages 6-1 through 6-22, Applied Biosystems, Part No. 901237; Ojwang, et al., 1997, Biochemistry, 36:6033-6045). After synthesis, the reporter gene containing a tRNA intron can be purified using standard techniques known to those skilled in the art (*see* Hwang et al., 1999, Proc. Natl. Acad. Sci. USA 96(23):12997-13002 and references cited therein). Depending on the length of the reporter gene containing a tRNA intron and the method of its synthesis, such purification techniques include, but are not limited to, reverse-phase high-performance liquid chromatography ("reverse-phase HPLC"), fast performance liquid chromatography ("FPLC"), and gel purification. Methods for labeling the substrate with a fluorescent acceptor moiety, a fluorescent donor moiety and/or quencher are well-known in the art (*see, e.g.,* U.S. Patent Nos. 6,472,156, 6,451,543, 6,348,322, 6,342,379, 6,323,039, 6,297,018, 6,291,201, 6,280,981, 5,843,658, and 5,439,797, the disclosures of which are incorporated by reference in their entirety).

### 5.1.3 VECTORS

The nucleotide sequence coding for a reporter gene and the nucleotide sequence coding for a tRNA intron can be inserted into an appropriate expression vector, *i.e.*, a vector which contains the necessary elements for the transcription and translation of the inserted protein-coding sequence. The necessary transcriptional and translational signals can also be supplied by the reporter gene. A variety of host-vector systems may be utilized to express the reporter gene. These include, but are not limited to, mammalian cell systems infected with virus (*e.g.*, vaccinia virus, adenovirus, *etc.*); insect cell systems infected with virus (*e.g.*, baculovirus); microorganisms such as yeast containing yeast vectors, or bacteria transformed with bacteriophage, DNA, plasmid DNA, or cosmid DNA; and stable cell lines generated by transformation using a selectable marker. The expression elements of vectors vary in their strengths and specificities. Depending on the host-vector system utilized, any one of a number of suitable transcription and translation elements may be used.

Any of the methods previously described for the insertion of DNA fragments into a vector may be used to construct expression vectors containing a chimeric nucleic acid consisting of appropriate transcriptional/translational control signals and the protein coding sequences. These methods may include *in vitro* recombinant DNA and synthetic techniques and *in vivo* recombinants (genetic recombination). Expression of the reporter gene construct may be regulated by a second nucleic acid sequence so that the reporter gene is expressed in a host transformed with the recombinant DNA molecule. For example, expression of a reporter gene construct may be controlled by any promoter/enhancer element known in the art, such as a constitutive promoter, a tissue-specific promoter, or an inducible promoter. Specific examples of promoters which may be used to control gene expression include, but are not limited to, the SV40 early promoter region (Bernoist & Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic expression vectors such as the β-lactamase promoter (Villa-Kamaroff et al., 1978, Proc. Natl. Acad. Sci. U.S.A. 75:3727-3731), or the tac promoter (DeBoer et al., 1983, Proc. Natl. Acad. Sci. U.S.A. 80:21-25); *see* also "Useful proteins from recombinant bacteria" in Scientific American, 1980, 242:74-94; plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4. promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells *(*Swift et al., 1984, Cell 38:639-646; Ormitz et al., 1986, Cold Spring Harbor Symp. Quant. Biol. 50:399-409; MacDonald, 1987, Hepatology 7:425-515); insulin gene control region (which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

In a specific embodiment, a vector is used that comprises a promoter operably linked to a reporter gene, one or more origins of replication, and, optionally, one or more selectable markers (*e.g.*, an antibiotic resistance gene). In a preferred embodiment, the vectors are CMV vectors, T7 vectors, lac vectors, pCEP4 vectors, 5.0/F vectors, or vectors with a tetracycline-regulated promoter (*e.g.*, pcDNA™5/FRT/TO from Invitrogen).

Expression vectors containing the reporter gene construct of the present invention can be identified by three general approaches: (a) nucleic acid hybridization, (b) presence or absence of "marker" nucleic acid functions, (c) expression of inserted sequences, and (d) sequencing. In the first approach, the presence of the reporter gene inserted in an expression vector can be detected by nucleic acid hybridization using probes comprising sequences that are homologous to the inserted reporter gene. In the second approach, the recombinant vector/host system can be identified and selected based upon the presence or absence of certain "marker" nucleic acid functions (*e.g.*, thymidine kinase activity, resistance to antibiotics, transformation phenotype, occlusion body formation in baculovirus, etc.) caused by the insertion of the nucleic acid of interest, *i.e.*, the reporter gene construct, in the vector. For example, if the nucleic acid of interest is inserted within the marker nucleic acid sequence of the vector, recombinants containing the insert can be identified by the absence of the marker nucleic acid function. In the third approach, recombinant expression vectors can be identified by assaying the reporter gene product expressed by the recombinant. Such assays can be based, for example, on the physical or functional properties of the particular reporter gene.

In a preferred embodiment, the reporter gene constructs are cloned into stable cell line expression vectors. In a preferred embodiment, the stable cell line expression vector contains a site specific genomic integration site. In another preferred embodiment, the reporter gene construct is cloned into an episomal mammalian expression vector.

### 5.1.4 TRANSFECTION

Once a vector encoding the appropriate gene has been synthesized, a host cell is transformed or transfected with the vector of interest. The use of stable transformants is preferred. In a preferred embodiment, the host cell is a mammalian cell. In a more preferred embodiment, the host cell is a human cell. In another embodiment, the host cells are primary cells isolated from a tissue or other biological sample of interest. Host cells that can be used in the methods of the present invention include, but are not limited to, hybridomas, pre-B cells, 293 cells, 293T cells, HeLa cells, HepG2 cells, K562 cells, 3T3 cells. In another preferred embodiment, the host cells are immortalized cell lines derived from a source, *e.g.*, a tissue. Other host cells that can be used in the present invention include, but are not limited to, virally-infected cells.

Transformation may be by any known method for introducing polynucleotides into a host cell, including, for example packaging the polynucleotide in a virus and transducing a host cell with the virus, and by direct uptake of the polynucleotide. The transformation procedure used depends upon the host to be transformed. Mammalian transformations (i. e., transfections) by direct uptake may be conducted using the calcium phosphate precipitation method of Graham & Van der Eb, 1978, Virol. 52:546, or the various known modifications thereof. Other methods for introducing recombinant polynucleotides into cells, particularly into mammalian cells, include dextran-mediated transfection, calcium phosphate mediated transfection, polybrene mediated transfection, protoplast fusion, electroporation, encapsulation of the polynucleotide(s) in liposomes, and direct microinjection of the polynucleotides into nuclei. Such methods are well-known to one of skill in the art.

In a preferred embodiment, stable cell lines containing the constructs of interest are generated for high throughput screening. Such stable cells lines may be generated by introducing a reporter gene construct comprising a selectable marker, allowing the cells to grow for 1-2 days in an enriched medium, and then growing the cells on a selective medium. The selectable marker in the recombinant plasmid confers resistance to the selection and allows cells to stably integrate the plasmid into their chromosomes and grow to form foci which in turn can be cloned and expanded into cell lines.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk-, hgprt- or aprt- cells, respectively. Also, anti-metabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

### 5.1.5 CELL FREE EXTRACTS

The invention provides for the translation of the reporter gene constructs in a cell-free system. Techniques for practicing this specific aspect of this invention will employ, unless otherwise indicated, conventional techniques of molecular biology, microbiology, and recombinant DNA manipulation and production, which are routinely practiced by one of skill in the art. *See, e.g.,* Sambrook, 1989, Molecular Cloning, A Laboratory Manual, Second Edition; DNA Cloning, Volumes I and II (Glover, Ed. 1985); and Transcription and Translation (Hames & Higgins, Eds. 1984).

Any technique well-known to one of skill in the art may be used to generate cell-free extracts for translation *in vitro.* For example, the cell-free extracts for *in vitro* translation reactions can be generated by contriguing cells and clarifying the supernatant. In particular, a cell extract utilized in accordance with the invention may be an S1 extract (*i.e.*, the supernatant from a 1,000 x g spin) to an S500 extract (*i.e.*, the supernatant from a 500,000 x g spin), preferably an S10 extract (*i.e.*, the supernatant from a 10,000 x g spin) to an S250 extract (*i.e.*, the supernatant from a 250,000 x g spin). In a specific embodiment, a cell extract utilized in accordance with the invention is an S50 extract (*i.e.,* the supernatant from a 50,000 x g spin) to an S100 extract (*i.e.*, the supernatant from a 100,000 x g spin).

The cell-free translation extract may be isolated from cells of any species origin. For example, the cell-free translation extract may be isolated from human cells, cultured mouse cells, cultured rat cells, Chinese hamster ovary (CHO) cells, yeast cells, Xenopus oocytes, rabbit reticulocytes, wheat germ, or rye embryo *(see, e.g.,* Krieg & Melton, 1984., Nature 308:203 and Dignam et al., 1990 Methods Enzymol. 182:194-203). Alternatively, the cell-free translation extract, *e.g.*, rabbit reticulocyte lysates and wheat germ extract, can be purchased from, *e.g.*, Promega, (Madison, WI). In a preferred embodiment, the cell-free extract is an extract isolated from human cells. In a specific embodiment, the human cells are HeLa cells.

### 5.2 PURIFICATION OF ENZYMES IN THE TRNA SPLICING PATHWAY

Enzymes in the tRNA splicing pathway, in particular tRNA splicing endonuclease and tRNA splicing ligase or biologically active fragments thereof, can be expressed and purified using any method known to those skilled in the art. The invention encompasses expressing and purifying animalia and/or fungal tRNA splicing ligase or a biologically fragment thereof using any method known to one skilled in the art. The invention also encompasses expressing and purifying animalia and/or fungal tRNA splicing endonuclease or a subunit thereof utilizing any method known to the skilled artisan. In a specific embodiment, the invention encompasses expressing and purifying mammalian, preferably human, tRNA splicing endonuclease and/or tRNA splicing ligase. Animalia and fungal tRNA splicing endonuclease and animalia and fungal tRNA splicing ligase can be expressed, for example, by recombinant DNA technology. In specific embodiments, the subunits of an animalia or fungal tRNA splicing endonuclease, or animalia or fungal tRNA splicing ligase is fused to a peptide tag to facilitate purification of the tRNA splicing endonuclease or the tRNA splicing ligase. In other embodiments, the endogenous animalia or fungal tRNA splicing endonuclease, or the endogenous animalia or fungal tRNA splicing ligase is purified.

In certain embodiments, recombinant human tRNA splicing endonuclease is purified and used with the methods of the invention. In other embodiments, partially purified human tRNA splicing endonuclease from any human cell source is used with the methods of the invention.

In other embodiments, recombinant human tRNA splicing ligase is purified and used with the methods of the invention. In other embodiments, partially purified human tRNA splicing ligase from any human cell source is used with the methods of the invention.

### 5.2.1 RECOMBINANT DNA

In various embodiments, an enzyme in the tRNA splicing pathway, *e.g.*, an animalia tRNA splicing endonuclease, a fungal tRNA splicing endonuclease, an animalia tRNA splicing ligase and a fungal tRNA splicing ligase, are encoded by a specific nucleotide sequence which is to be transcribed and translated. The nucleotide sequence is inserted into an expression vector for propagation and expression in recombinant cells.

In certain embodiments, an animalia tRNA splicing endonuclease subunit is encoded by a specific nucleotide sequence which is to be transcribed and translated. The nucleotide sequence is inserted into an expression vector for propagation and expression in recombinant cells. An animalia tRNA splicing endonuclease is a heterotetramer, each of the four subunits may be expressed together in the same cell or separately in different cells; the subunits isolated and then combined to produce tRNA splicing endonuclease. Preferably, the animalia tRNA splicing endonuclease subunits are expressed in the same cell and the functional tRNA splicing endonuclease is isolated from the cell.

In other embodiments, a fungal tRNA splicing endonuclease subunit is encoded by a specific nucleotide sequence which is to be transcribed and translated. The nucleotide sequence is insertd into an expression vector for propagation and expression in recombinant. Like the animalia tRNA splicing endonuclease, the fungal tRNA splicing endonuclease is a heterotetramer. The subunits of the heterotetramer may be expressed together in the same cell or separately in different cells; if produced in separate cells the subunits may be isolated and then combined to produce tRNA splicing endonuclease.

In other embodiments, a biologically active fragment of an animalia or fungal tRNA splicing ligase is encoded by a specific nucleotide sequence which is to be transcribed and translated. The nucleotide sequence is inserted into an expression vector for propagation and expression in recombinant cells.

In some embodiments, a fragment of a tRNA splicing ligase with a particular enzymatic activity, inclduing but not limited to ligase activity, kinase activity, cyclic phosphodiesterase activity, adenylate synthetase activity, and phosphodiesterase activity is used in the methods of the invention. In one embodiment a fragment of a tRNA splicing ligase with ligation/RNA joining enzymatic activity is used in the methods and assays of the invention. In a sepecific embodiment, the fragment of the tRNA splicing ligase with ligation/RNA joining enzymatic activity corresponds to amino acids 595-827. In another embodiment, a fragment of a tRNA splicing ligase with adenylate synthestase activity is used in the methods and assays of the invention. In a sepecific embodiment, the fragment of the tRNA splicing ligase with adenylate synthestase activity corresponds to amino acids 15-397. In another embodiment, a fragment of a tRNA splicing ligase with kinase activity is used in the methods and assays of the invention. In a sepecific embodiment, the fragment of the tRNA splicing ligase with kinase activity corresponds to amino acids 397-827. In a sepecific embodiment, the fragment of the tRNA splicing ligase with adenylate synthestase activity corresponds to amino acids 15-397. In another embodiment, a fragment of a tRNA splicing ligase with phosphodiesterase activity is used in the methods and assays of the invention. In a sepecific embodiment, the fragment of the tRNA splicing ligase with phosphodiesterase activity corresponds to amino acids 596-827.

An expression construct as used herein refers to a nucleotide sequence encoding an enzyme in the tRNA splicing pathway (*e.g.*, an animalia tRNA splicing endonuclease, a fungal tRNA splicing endocuclease, an animalia tRNA splicing ligase or a fungal tRNA splicing ligase) or a biologically active fragment thereof operably linked to one or more regulatory regions or enhancer/promoter sequences which enables expression of the enzyme in an appropriate host cell. "Operably linked" refers to an association in which the regulatory regions and the nucleotide sequence encoding a tRNA splicing enzyme that is to be expressed are joined and positioned in such a way as to permit transcription, and ultimately, translation. In some embodiments, an expression construct refers to a nucleotide sequence encoding an animalia and/or fungal tRNA splicing endonuclease or an animalia and/or fungal tRNA splicing endonuclease subunit, respectively, operably linked to one or more regulatory regions or enhancer/promoter sequences which enables expression of the animalia and/or fungal tRNA splicing endonuclease or a subunit thereof in an appropriate host cell. In other embodiments, an expression construct, refers to a nucleotide sequence encoding one, two, three or four animalia tRNA splicing endonuclease subunits (preferably, human tRNA splicing endonuclease subunits) or fungal tRNA splicing endonuclease subunits operably linked to one or more regulatory regions or enhancer/promoter sequences which enables the expression of animalia tRNA splicing endonuclease subunits in an appropriate host cell. In yet other embodiments an expression construct refers to a nucleotide sequence encoding an animalia and/or fungal tRNA splicing ligase or a biologically active fragment thereof, respectively, operably linked to one or more regulatory regions or enhancer/promoter sequences which enables expression of the tRNA splicing ligase or a biologically active fragment thereof in an appropriate host cell.

The regulatory regions necessary for transcription of an enzyme in the tRNA spicing pathway (*e.g.*, an animalia tRNA splicing endonuclease, a fungal tRNA splicing endocuclease, an animalia tRNA splicing ligase or a fungal tRNA splicing ligase, or a subunit or a biologically active fragment of an enzyme in the tRNA splicing pathway) can be provided by the expression vector. In a compatible host-construct system, cellular transcriptional factors, such as RNA polymerase, will bind to the regulatory regions on the expression construct to effect transcription of the enzyme in the tRNA splicing pathway or the subunit or biologically active fragment of an enzyme in the tRNA splicing pathway in the host organism. The precise nature of the regulatory regions needed for gene expression may vary from host cell to host cell. Generally, a promoter is required which is capable of binding RNA polymerase and promoting the transcription of an operably-associated nucleic acid sequence. Such regulatory regions may include those 5'-non-coding sequences involved with initiation of transcription and translation, such as the TATA box, capping sequence, CAAT sequence, and the like. The non-coding region 3' to the coding sequence may contain transcriptional termination regulatory sequences, such as terminators and polyadenylation sites.

Constitutive, tissue-specific and/or inducible regulatory regions may be used for expression of an enzyme in the tRNA splicing pathway or a subunit or a biologically active fragment of an enzyme in the tRNA splicing pathway. It may be desirable to use inducible promoters when the conditions optimal for growth of the host cells and the conditions for high level expression of an enzyme in the tRNA splicing pathway or a subunit or a biologically active fragment of an enzyme in the tRNA splicing pathway are different. Examples of useful regulatory regions are provided below.

In order to attach DNA sequences with regulatory functions, such as promoters, to the sequence encoding an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or a biologically active fragment of an enzyme in the tRNA splicing pathway or to insert the sequence encoding an enzyme in the tRNA splicing pathway (*e.g.*, an animalia tRNA splicing endonuclease, a fungal tRNA splicing endoneuclease, an animlia tRNA splicing ligase or a fungal tRNA splicing ligase) or a subunit or a biologically active fragment of an enzyme in the tRNA splicing pathway into the cloning site of a vector, linkers or adapters providing the appropriate compatible restriction sites may be ligated to the ends of the cDNAs by techniques well known in the art (Wu et al., 1987, Methods in Enzymol 152:343-349). Cleavage with a restriction enzyme can be followed by modification to create blunt ends by digesting back or filling in single-stranded DNA termini before ligation. Alternatively, a desired restriction enzyme site can be introduced into a fragment of DNA by amplification of the DNA by use of PCR with primers containing the desired restriction enzyme site.

An expression construct comprising a sequence encoding an enzyme in the tRNA splicing pathway (*e.g.*, an animalia tRNA splicing endonuclease, a fungal tRNA splicing endonuclease, an animalia tRNA splicing ligase or fungal tRNA splicing ligase or a fungal tRNA splicing ligase) or a subunit or a biologically active fragment of an enzyme in the tRNA splicing pathway operably linked to regulatory regions (enhancer/promoter sequences) can be directly introduced into appropriate host cells for expression and production of an enzyme in the tRNA splicing pathway (*e.g.*, an animalia tRNA splicing endonuclease, a fungal tRNA splicing endonuclease, an animalia tRNA splicing ligase) or a subunit or a biologically active fragment of an enzyme in the tRNA splicing pathway without further cloning. The expression constructs can also contain DNA sequences that facilitate integration of the sequence encoding an enzyme in the tRNA splicing pathway (*e.g.*, an animalia tRNA splicing endonuclease, a fungal tRMA splicing endonuclease, an animalia tRNA splicing ligase or a fungal tRNA splicing ligase) or a subunit or a biologically active fragment of an enzyme in the tRNA splicing pathway into the genome of the host cell, *e.g.*, via homologous recombination. In this instance, it is not necessary to employ an expression vector comprising a replication origin suitable for appropriate host cells in order to propagate and express an enzyme in the tRNA splicing pathway (*e.g.*, animalia tRNA splicing endonuclease, a fungal tRNA splicing endonuclease, an animlia tRNA splicing ligase or a splicing tRNA ligase) or a subunit or a biologically active fragment of an enzyme in the tRNA splicing pathway in the host cells.

A variety of expression vectors may be used in the present invention which include, but are not limited to, plasmids, cosmids, phage, phagemids, or modified viruses. Typically, such expression vectors comprise a functional origin of replication for propagation of the vector in an appropriate host cell, one or more restriction endonuclease sites for insertion of the sequence encoding an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or a biologically active fragment of an enzyme in the tRNA splicing pathway, and one or more selection markers. The expression vector must be used with a compatible host cell which may be derived from a prokaryotic or an eukaryotic organism including but not limited to bacteria, yeasts, insects, mammals, and humans.

Vectors based on *E. coli* are the most popular and versatile systems for high level expression of foreign proteins (Makrides, 1996, Microbiol Rev, 60:512-538). Non-limiting examples of regulatory regions that can be used for expression in *E. coli* may include but not limited to lac, trp, lpp, phoA, recA, tac, T3, T7 and λP$_L$ (Makrides, 1996, Microbiol Rev, 60:512-538). Non-limiting examples of prokaryotic expression vectors may include the λgt vector series such as λgt11 (Huynh et al., 1984 in "DNA Cloning Techniques", Vol. I: A Practical Approach (D. Glover, ed.), pp. 49-78, IRL Press, Oxford), and the pET vector series (Studier et al., 1990, Methods Enzymol., 185:60-89). However, a potential drawback of a prokaryotic host-vector system is the inability to perform many of the post-translational processing of mammalian cells. Thus, a eukaryotic host-vector system is preferred. In a specific embodiment, the host-vector system is a mammalian host-vector system. In another embodiment, the host-vector system is a human host-vector system is the most preferred.

For expression of an enzyme in the tRNA splicing pathway (*e.g.*, animalia tRNA splicing endonuclease, a fungal tRMA splicing endonuclease, an animalia tRNA splicing ligase or a fungal tRNA splicing ligase) or a subunit or a biologically active fragment thereof of an enzyme in the tRNA splicing pathway in mammalian host cells, a variety of regulatory regions can be used, for example, the SV40 early and late promoters, the cytomegalovirus (CMV) immediate early promoter, and the Rous sarcoma virus long terminal repeat (RSV-LTR) promoter. Inducible promoters that may be useful in mammalian cells include, but are not limited to, those associated with the metallothionein II gene, mouse mammary tumor virus glucocorticoid responsive long terminal repeats (MMTV-LTR), β-Interferon gene, and hsp70 gene (Williams et al., 1989, Cancer Res. 49:2735-42 ; Taylor et al., 1990, Mol. Cell Biol., 10:165-75). It may be advantageous to use heat shock promoters or stress promoters to drive expression of an enzyme in the tRNA splicing pathway or a subunit or a biologically active fragment thereof of an enzyme in the tRNA splicing pathway in recombinant host cells.

In addition, the expression vector may contain selectable or screenable marker genes for initially isolating, identifying or tracking host cells that contain DNA encoding the elected enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or a biologically active fragment thereof of an enzyme in the tRNA splicing pathway. For long term, high yield production of an enzyme in the tRNA splicing pathway, stable expression in mammalian cells is preferred. A number of selection systems may be used for mammalian cells, including but not limited to the Herpes simplex virus thymidine kinase (Wigler et al., 1977, Cell 11:223), hypoxanthine-guanine phosphoribosyltransferase (Szybalski and Szybalski, 1962, Proc. Natl. Acad. Sci. USA 48:2026), and adenine phosphoribosyltransferase (Lowy et al., 1980, Cell 22:817) genes can be employed in tk-, hgprt- or aprt- cells, respectively. Also, antimetabolite resistance can be used as the basis of selection for dihydrofolate reductase (dhfr), which confers resistance to methotrexate (Wigler et al., 1980, Natl. Acad. Sci. USA 77:3567; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neomycin phosphotransferase (neo), which confers resistance to the aminoglycoside G-418 (Colberre-Garapin et al., 1981, J. Mol. Biol. 150:1); and hygromycin phosphotransferase (hyg), which confers resistance to hygromycin (Santerre et al., 1984, Gene 30: 147). Other selectable markers, such as but not limited to histidinol and Zeocin™ can also be used.

## 5.2.2 <u>PRODUCTION OF RECOMBINANT PROTEINS</u>

### 5.2.2.1 PEPTIDE TAGGING

Generating a fusion protein comprising a peptide tag and an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or a biologically active fragment thereof can aid the purification of the enzyme. In a particular embodiment, the enzyme in the tRNA splicing pathway used in the methods of the invention is a yeast tRNA splicing enzyme (*e.g.*, a yeast tRNA splicing endonuclease or a yeast tRNA splicing ligase) or a subunit or biologically active fragment thereof. In a specific embodiment, the enzyme in the tRNA splicing pathway used in the methods of the invention is a mammalian tRNA splicing enzyme (*e.g.*, a mammalian tRNA splicing endonuclease, or a mammalian tRNA splicing ligase) or a subunit or biologically active fragment thereof. In a preferred embodiment, the animalia tRNA splicing ligase for use in the methods of the invention is a human tRNA splicing ligase. In another preferred embodiment, the animalia tRNA splicing endonuclease for use in the methods of the invention is a human tRNA splicing endonuclease. In yet another preferred embodiment, the animalia tRNA splicing endonuclease is a human animalia tRNA splicing endonuclease subunit. A fusion protein comprising a peptide and an enzyme in the tRNA splicing pathway can be made by ligating the nucleotide sequence encoding the enzyme (*e.g.*, an animalia tRNA splicing endonuclease, a fungal tRNA splicing endonuclease an animalia tRNA splicing ligase or a fungal tRNA splicing ligase) or a subunit or biologically active fragment thereof to the sequence encoding the peptide tag in the proper reading frame: Care should be taken to ensure that the modified gene remains within the same translational reading frame, uninterrupted by translational stop signals and/or spurious messenger RNA. splicing signals.

The peptide tag may be fused to the amino terminal or to the carboxyl terminal of an enzyme in the tRNA splicing pathway (*e.g.*, an animalia tRNA splicing endonuclease, a fungal tRNA splicing endonuclease, an animalia TUNA splicing ligase or a fungal tRNA splicing ligase). The precise site at which the fusion is made is not critical. The optimal site can be determined by routine experimentation.

A variety of peptide tags known in the art may be conjugated to an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof including, but not limited to, the immunoglobulin constant regions, polyhistidine sequence (Petty, 1996, Metal-chelate affinity chromatography, in Current Protocols in Molecular Biology, Vol. 2, Ed. Ausubel et al., Greene Publish. Assoc. & Wiley Interscience), glutathione S-transferase (GST; Smith, 1993, Methods Mol. Cell Bio. 4:220-229), the *E. coli* maltose binding protein (Guan et al., 1987, Gene 67:21-30), various cellulose binding domains (U.S. patent 5,496,934; 5,202,247; 5,137,819; Tomme et al., 1994, Protein Eng. 7:117-123), and the FLAG epitope (Short Protocols in Molecular Biology, 1999, Ed. Ausubel et al., John Wiley & Sons, Inc., Unit 10.11). Other peptide tags that are well-known to one of skill in the art that are recognized by specific binding partners and thus facilitate isolation by affinity binding to the binding partner (which is preferably immobilized and/or on a solid support) may be conjugated to an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof. As will be appreciated by those skilled in the art, many methods can be used to obtain the coding region of the above-mentioned peptide tags, including but not limited to, DNA cloning, DNA amplification, and synthetic methods. Some of the peptide tags and reagents for their detection and isolation are available commercially.

In a specific embodiment, the polyhistidine tag conjugated to an enzyme in the tRNA splicing pathway (*e.g.*, an animalia tRNA splicing endonuclease, a fungal tRNA splicing endonuclease, an animlia tRNA splicing ligase or a fungal tRNA splicing ligase) or a subunit or a biologically active fragment thereof has at least 6, at least 8, at least 10 or at least 10 histidines. In a preferred embodiment, the polyhistidine tag conj ugated to an enzyme in the tRNA splicing pathway *(e.g.,* an animalia tRNA splicing endonuclease, a fungal tRNA splicing endonuclease, an animlia tRNA splicing ligase or a fungal tRNA splicing ligase) or a subunit or biologically active fragment thereof has 8 histidines.

In a specific embodiment, the polyhistidine tag conjugated to an animalia tRNA splicing endonuclease subunit has at least 6, at least 8, at least 10 or at least 10 histidines. In a preferred embodiment, the polyhistidine tag conjugated to an animalia tRNA splicing endonuclease subunit has 8 histidines.

In another embodiment, an animalia tRNA splicing endonuclease subunit can be labeled with more than one peptide. In a specific embodiment, an animalia tRNA splicing endonuclease subunit is labeled with a peptide tag consisting of 8 histidines and a Flag epitope tag.

In certain embodiments of the invention, different subunits of an animalia tRNA splicing

## 5.2.2.2 EXPRESSION SYSTEMS AND HOST CELLS

Preferred mammalian host cells include, but are not limited to, those derived from humans, monkeys and rodents, (*see,* for example, Kriegler M. in "Gene Transfer and Expression: A Laboratory Manual", New York, Freeman & Co. 1990), such as monkey kidney cell line transformed by SV40 (COS-7, ATCC Accession No. CRL 1651); human embryonic kidney cell lines (293, 293-EBNA, or 293 cells subcloned for growth in suspension culture, Graham et al., J. Gen. Virol., 36:59, 1977; baby hamster kidney cells (BHK, ATCC Accession No. CCL 10); chinese hamster ovary-cells-DHFR (CHO, Urlaub and Chasin. Proc. Natl. Acad. Sci. 77; 4216, 1980); mouse sertoli cells (Mather, Biol. Reprod. 23: 243-251, 1980); mouse fibroblast cells (NIH-3T3), monkey kidney cells (CVI ATCC Accession No. CCL 70); african green monkey kidney cells (VERO-76, ATCC Accession No. CRL-1587); human cervical carcinoma cells (HELA, ATCC Accession No. CCL 2); canine kidney cells (MDCK, ATCC Accession No. CCL 34); buffalo rat liver cells (BRL 3A, ATCC Accession No. CRL 1442); human lung cells (W138, ATCC Accession No. CCL 75); human liver cells (Hep G2, HB 8065); and mouse mammary tumor cells (MMT 060562, ATCC Accession No. CCL51).

A number of viral-based expression systems may also be utilized with mammalian cells to produce an enzyme in the tRNA splicing pathway (*e.g.*, an animalia tRNA splicing endonuclease, a fungal tRNA splicing endonuclease, an animlia tRNA splicing ligase or a fungal tRNA splicing ligase) or a subunit of biologically active fragment thereof. Vectors using DNA virus backbones have been derived from simian vitus 40 (SV40) (Hamer et al., 1979, Cell 17:725), adenovirus (Van Doren et al., 1984, Mol Cell Biol 4:1653), adenoassociated virus (McLaughlin et al., 1988, J Virol 62:1963), and bovine papillomas virus (Zinn et al., 1982, Proc Natl Acad Sci 79:4897). In cases where an adenovirus is used as an expression vector, the donor DNA sequence may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing heterologous products in infected hosts. (*See e.g.*, Logan and Shenk, 1984, Proc. Natl. Acad. Sci. (USA) 81:3655-3659).

Other useful eukaryotic host-vector system may include yeast and insect systems. In yeast, a number of vectors containing constitutive or inducible promoters may be used with *Saccharomyces cerevisiae* (baker's yeast), *Schizosaccharomyces pombe* (fission yeast), *Pichia pastoris,* and *Hansenula polymorpha* (methylotropic yeasts). For a review *see,* Current Protocols in Molecular Biology, Vol. 2, 1988, Ed. Ausubel et al., Greene Publish. Assoc. & Wiley Interscience, Ch. 13; Grant et al., 1987, Expression and Secretion Vectors for Yeast, in Methods in Enzymology, Eds. Wu & Grossman, 1987, Acad. Press, N.Y., Vol. 153, pp. 516-544; Glover, 1986, DNA Cloning, Vol. II, IRL Press, Wash., D.C., Ch. 3; and Bitter, 1987, Heterologous Gene Expression in Yeast, Methods in Enzymology, Eds. Berger & Kimmel, Acad. Press, N.Y., Vol. 152, pp. 673-684; and The Molecular Biology of the Yeast Saccharomyces, 1982, Eds. Strathern et al., Cold Spring Harbor Press, Vols. I and II.

In an insect system, Autographa californica nuclear polyhidrosis virus (AcNPV), a baculovirus, can be used as a vector to express an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof in Spodoptera frugiperda cells. The sequences encoding an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof may be cloned into non-essential regions (for example the polyhedrin gene) of the virus and placed under control of an AcNPV promoter (for example the polyhedrin promoter). These recombinant viruses are then used to infect host cells in which the inserted DNA is expressed. *(See e.g.,* Smith et al., 1983, J Virol 46:584; Smith, U.S. Patent No. 4,215,051.)

Any of the cloning and expression vectors described herein may be synthesized and assembled from known DNA sequences by well known techniques in the art. The regulatory regions and enhancer elements can be of a variety of origins, both natural and synthetic. Some vectors and host cells may be obtained commercially. Non-limiting examples of useful vectors are described in Appendix 5 of Current Protocols in Molecular Biology, 1988, ed. Ausubel et al., Greene Publish. Assoc. & Wiley Interscience, which is incorporated herein by reference; and the catalogs of commercial suppliers such as Clontech Laboratories, Stratagene Inc., and Invitrogen, Inc.

Expression constructs containing a cloned nucleotide sequence encoding an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof can be introduced into the host cell by a variety of techniques known in the art, including but not limited to, for prokaryotic cells, bacterial transformation (Hanahan, 1985, in DNA Cloning, A Practical Approach, 1:109-136), and for eukaryotic cells, calcium phosphate mediated transfection (Wigler et al., 1977, Cell 11:223-232), liposome-mediated transfection (Schaefer-Ridder et al., 1982, Science 215:166-168), electroporation (Wolff et al., 1987, Proc Natl Acad Sci 84:3344), and microinjection (Cappechi, 1980, Cell 22:479-488).

For long term, high yield production of a properly processed enzyme in the tRNA splicing pathway (*e.g.*, an animalia tRNA splicing endonuclease, a fungal tRNA splicing endonuclease, an animalia tRNA splicing ligase or a fungal tRNA splicing ligase), or a subunit or biologically active fragment thereof stable expression in mammalian cells is preferred. Cell lines that stably express an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof may be engineered by using a vector that contains a selectable marker. By way of example but not limitation, following the introduction of the expression constructs, engineered cells may be allowed to grow for 1-2 days in an enriched media, and then are switched to a selective media. The selectable marker in the expression construct confers resistance to the selection and optimally allows cells to stably integrate the expression construct into their chromosomes and to grow in culture and to be expanded into cell lines. Such cells can be cultured for a long period of time while an enzyme in the tRNA splicing pathway (*e.g.*, an animalia tRNA splicing endonuclease, a fungal tRNA splicing endonuclease, an animalia tRNA splicing ligase or a fungal tRNA splicing ligase) or a subunit or biologically active fragment thereof is expressed continuously.

In a preferred embodiment, a human tRNA splicing endonuclease or a subunit thereof or a human tRNA splicing ligase or a biologically active fragment thereof is transfected stably in 293T cells (ATCC Accession No. CRL-11268).

### 5.2.2.3 PROTEIN PURIFICATION

Generally, an enzyme in the tRNA splicing pathway (*e.g.*, an animalia tRNA splicing endonuclease, a fungal tRNA splicing endonulease, an animalia tRNA splicing ligase or a fungal tRNA splicing ligase) or a subunit or biologically active fragment thereof can be recovered and purified from recombinant cell cultures by known methods, including ammonium sulfate precipitation, acid extraction, anion or cation exchange chromatography, phosphocellulose chromatography, immunoaffinity chromatography, hydroxyapatite chromatography, and lectin chromatography. In a specific embodiment, the fungal tRNA splicing endonuclease or subunit thereof is a yeast tRNA splicing endonuclease or a subunit thereof. In another embodiment, the fungal tRNA splicing ligase or biologically active fragment thereof is a yeast tRNA splicing ligase or a biologically active gragment thereof. In a preferred embodiment, the animalia tRNA splicing endonuclease subunit or the animalia tRNA splicing endonuclease for use in the methods of the invention is a mammalian tRNA splicing endonuclease subunit or a mammalian tRNA splicing endonuclease, respectively. In a more preferred embodiment, the animalia tRNA splicing endonuclease subunit or the animalia tRNA splicing endonuclease is a human tRNA splicing endonuclease subunit or a human tRNA splicing endonuclease, respectively. In another preferred embodiment, the animalia tRNA splicing ligase or a biologically active fragment thereof for use in the methods of the invention is a mammalian tRNA splicing ligase or a biologically active fragment thereof. In a more preferred embodiment, the animalia tRNA splicing ligase or a biologically active fragment thereof is a human tRNA splicing ligase or a biologically active fragment thereof. Before the enzyme in the tRNA splicing pathway for use in the methods of the invention can be purified, total protein has to be prepared from the cell culture. This procedure comprises collection, washing and lysis of said cells and is well known to the skilled artisan.

In particular, a recombinant enzyme of the tRNA splicing pathway (*e.g.*, an animalia tRNA splicing endonuclease, a fungal tRNA splicing endonuclease, an animlia tRNA splicing ligase or a fungal tRNA splicing ligase) or a subunit or bioligically active fragment thereof fused to a peptide tag may be purified based on the properties of the peptide: tag. One approach is based on specific molecular interactions between a tag and its binding partner. The other approach relies on the immunospecific binding of an antibody to an epitope present on the tag or on the protein which is to be purified. The principle of affinity chromatography well-known in the art is generally applicable to both of these approaches. Once the tRNA splicing enzyme-peptide tag fusion protein is eluted, fractions can be collected and tested for the presence of the tRNA splicing enzyme and/or for the presence of the peptide tag. In a specific embodiment, the fractions are tested for tRNA splicing endonuclease activity and/or tRNA splicing ligase activity. Subsequently, the fractions with tRNA splicing endonuclease activity and/or tRNA splicing ligase activity levels over a certain threshold level can be pooled. Any method used in the art for measuring the enzymtic activity of tRNA splicing endonuclease and tRNA splicing ligase is contemplated within the present invention.

Described below are several methods based on specific molecular interactions of a tag and its binding partner.

A method that is generally applicable to purifying a tRNA splicing enzyme (*e.g.*, an animalia tRNA splicing endonuclease, a fungal tRNA splicing endonuclease, an animalia tRNA splicing ligase or a fungal tRNA splicing ligase) or a subunit or biologically active fragment thereof that is fused to the constant regions of immunoglobulin is protein A affinity chromatography, a technique that is well known in the art. Staphylococcus protein A is a 42 kD polypeptide that binds specifically to a region located between the second and third constant regions of heavy chain immunoglobulins. Because of the Fc domains of different classes, subclasses and species of immunoglobulins, affinity of protein A for human Fc regions is strong, but may vary among species. Subclasses that are less preferred include human IgG-3, and most rat subclasses. For certain subclasses, protein G (of Streptococci) may be used in place of protein A in the purification. Protein-A sepharose (Pharmacia or Biorad) is a commonly used solid phase for affinity purification of antibodies, and can be used essentially in the same manner for the purification of a tRNA splicing enzyme or a subunit or biologically active fragment thereof fused to an immunoglobulin Fc fragment. Bound tRNA splicing enzyme-Fc fusion protein can be eluted by various buffer systems known in the art, including a succession of citrate, acetate and glycine-HCl buffers which gradually lowers the pH. This method is less preferred if the recombinant cells also produce antibodies which will be co-purified with the tRNA splicing enzyme. *see,* for example, Langone, 1982, J. Immunol. meth. 51:3; Wilchek et al., 1982, Biochem. Intl. 4:629; Sjobring et al., 1991, J. Biol. Chem. 26:399; page 617-618, in Antibodies A Laboratory Manual, edited by Harlow and Lane, Cold Spring Harbor laboratory, 1988,

Alternatively, a polyhistidine tag may be used, in which case, a tRNA splicing enzyme (*e.g.*, an animalia tRNA splicing endonuclease, a fungal tRNA splicing endonuclease, an animalia tRNA splicing ligase or a fungal tRNA splicing ligase) or a subunit or biologically active fragment thereof can be purified by metal chelate chromatography. The polyhistidine tag, usually a sequence of six histidines, has a high affinity for divalent metal ions, such as nickel ions ($Ni^{2+}$), which can be immobilized on a solid phase, such as nitrilotriacetic acid-matrices. Polyhistidine has a well characterized affinity for $Ni^{2+}$-NTA-agarose, and can be eluted with either of two mild treatments: imidazole (0.1-0.2 M) will effectively compete with the resin for binding sites; or lowering the pH just below 6.0 will protonate the histidine sidechains and disrupt the binding. The purification method comprises loading the cell culture lysate onto the $Ni^{2+}$-NTA-agarose column, washing the contaminants through, and eluting the tRNA splicing enzyme (*e.g.*, an animalia tRNA splicing endonuclease, a fungal tRNA splicing endonuclease, an animalia tRNA splicing ligase) a subunit or biologically active fragment thereof with imidazole or weak acid. $Ni^{2+}$-NTA-agarose can be obtained from commercial suppliers such as Sigma (St. Louis) and Qiagen. Antibodies that recognize the polyhistidine tag are also available which can be used to detect and quantitate the tRNA splicing enzyme (*e.g.*, an animalia tRNA splicing endonuclease, a fungal tRNA splicing endocnuclease, an animalia tRNA splicing ligase or a fungal tRNA splicing ligase) or a subunit or biologically active gragment thereof.

Another exemplary peptide tag that can be used is the glutathione-S-transferase (GST) sequence, originally cloned from the helminth, *Schistosoma japonicum.* In general, a tRNA splicing enzyme-GST fusion protein expressed in a prokaryotic host cell, such as *E. coli,* can be purified from the cell culture lysate by absorption with glutathione agarose beads, followed by elution in the presence of free reduced glutathione at neutral pH. Since GST is known to form dimers under certain conditions, dimeric tRNA splicing enzyme (*e.g.*, an animalia tRNA splicing endonuclease, a fungal tRNA splicing endonuclease, an animalia tRNA splicing ligase or a fungal tRNA splicing ligase) may be obtained. *See,* Smith, 1993, Methods Mol. Cell Bio. 4:220-229.

Another useful peptide tag that can be used is the maltose binding protein (MBP) of *E. coli,* which is encoded by the *mal*E gene. An animalia tRNA splicing enzyme (*e.g.*, an animalia tRNA splicing endonuclease, a fungal tRNA splicing endonuclease, an animalia tRNA splicing ligase or a fungal tRNA splicing ligase) or a subunit or biologically active fragment thereof fused to MBP binds to amylose resin while contaminants are washed away. The bound tRNA splicing enzyme-MBP fusion is eluted from the amylose resin by maltose. *See,* for example, Guan et al., 1987, Gene 67:21-30.

The second approach for purifying a tRNA splicing enzyme (*e.g.*, an animalia tRNA splicing endonuclease, a fungal tRNA splicing endonuclease, an animalia RNA splicing ligase or a fungal tRNA splicing ligase) or a subunit or biologically active fragment thereof is applicable to peptide tags that contain an epitope for which polyclonal or monoclonal antibodies are available. It is also applicable if polyclonal or monoclonal antibodies specific to a tRNA splicing enzyme or a subunit or biologically active fragment thereof are available. Various methods known in the art for purification of protein by immunospecific binding, such as immunoaffinity chromatography, and immunoprecipitation, can be used. *See,* for example, Chapter 13 in Antibodies A Laboratory Manual, edited by Harlow and Lane, Cold Spring Harbor laboratory, 1988; and Chapter 8, Sections I and II, in Current Protocols in Immunology, ed. by Coligan et al., John Wiley, 1991; the disclosure of which are both incorporated by reference herein.

In particular the invention relates to the expression and purification of Hs Sen2p and Hs Sen34p (*see* Table 2).

**TABLE 2**

| Gene | Homolog | LocusLink | Genbank Protein | Genome Contig |
|------|---------|-----------|-----------------|---------------|
| Hs Sen2p | Sc Sen2p | 80746 | NP_079541 | NT_005927.12 |
| Hs Sen34p | Sc Sen34p | 79042 | XP_085899 | NT_011225.9 |
| Note: *Sc=Sacromyces cerivisae*; Hs=Human | | | | |

Oligonucleotides complementary to the 5' and 3' ends of the open reading frames of the animalia tRNA splicing endonuclease subunits can be used to PCR amplify the open reading frames encoding the animalia tRNA splicing endonuclease.

The invention also relates to the expression and purification of an Hs Sen 2p variant ("Hs Sen 2 var."). The Hs Sen 2 var. is a splice variant of Hs Sen2 lacking exon 8 of the genomic DNA sequence for Human Sen 2. Figure 2 depicts an amino acid sequence alignment of the amino acid sequences of the two human Sen 2 subunits (*i.e.*, Hs Sen2 and Hs Sen 2 var.) and the amino acid sequence of the yeast subunit Sc Sen 2p. The sequence alignment reveals a high degree of similarity in the YRGGY motif (SEQ ID NO: 4), the active site for the 5' splice site of yeast (Sc Sen 2p) and archael (not shown) tRNA splicing endonuclease. Based upon the sequence alignment, the Hs Sen 2 var. lacks the putative transmembrane domain found in the Hs Sen 2 endonuclease, which may affect the localization of the Hs Sen2 var. in an animalia cell.

In specific embodiments, the Hs Sen2 var. catalyzes the endonucleolytic cleavage of substrates other than those containing tRNA introns. In other embodiments, the Hs Sen2 var catalyzes the endonucleolytic cleavage of substrates containing tRNA introns. In yet other embodiments, the Hs Sen2 var. catalyzes the endonucleolytic cleavage of substrates containing tRNA introns and substrates that do not contain tRNA introns.

The human subunits, including, but not limited to, Hs Sen2, Hs Sen2 var. and Hs Sen 34, can be utilized in accordance with the methods of the invention. In a specific embodiment, the Hs Sen 2 subunit is utilized in accordance with the methods of the invention. In another embodiment, the Hs Sen 2 var. subunit is utilized in accordance with the methods of the invention. In another embodiment, the Hs Sen 34 subunit is utilized in accordance with the methods of the invention. In yet another embodiment, Hs Sen 2, Hs Sen 2 var., Hs Sen 34 or any combination thereof is utilized in accordance with the methods of the invention.

The invention also provides methods for expression and purification of yeast tRNA splicing endonuclease subunits, including but not limited to SEN2, SEN34, SEN54, and SEN15, which have the following accession numbers. SEN2: M32336; SEN34: YAR008w; SEN54: YPL083c; SEN15: YMR059w, respectively.

In a specific embodiment, the yeast tRNA splicing endonuclease is purified according to the procedure described in Trotta et al., 1997, Cell 89:849-858. In another specific embodiment, the yeast tRNA splicing ligase is purified according to the procedure described in Xu et al., 1990, Methods in Enzymology, 181: 463-471, which is incorporated herein by reference in its entirety. Briefly, the purification may comprise polymin P precipitation, followed by Heparin-Agarose chromatography, followed by Blue Trisacryl M chromatography, hydroxyapatite chromatography, and Sehadex G-150 gel filtration chromatography. Alternatively, the yeast tRNA splicing ligase for use in the methods of the invention maybe purified using the methodology described by Phizicky et al, 1986, Journal of Biol. Chem., 261(6): 2978-2986, which is incorporated herein by reference in its entirety.

### 5.3 COMPOUNDS

Libraries screened using the methods of the present invention can comprise a variety of types of compounds. Examples of libraries that can be screened in accordance with the methods of the invention include, but are not limited to, peptoids; random biooligomers; diversomers such as hydantoins, benzodiazepines and dipeptides; vinylogous polypeptides; nonpeptidal peptidomimetics; oligocarbamates; peptidyl phosphonates; peptide nucleic acid libraries; antibody libraries; carbohydrate libraries; and small molecule libraries (preferably, small organic molecule libraries). In some embodiments, the compounds in the libraries screened are nucleic acid or peptide molecules. In a non-limiting example, peptide molecules can exist in a phage display library. In other embodiments, the types of compounds include, but are not limited to, peptide analogs including peptides comprising non-naturally occurring amino acids, *e.g.*, D-amino acids, phosphorous analogs of amino acids, such as a-amino phosphoric acids and $\alpha$-amino phosphoric acids, or amino acids having non-peptide linkages, nucleic acid analogs such as phosphorothioates and PNAs, hormones, antigens, synthetic or naturally occurring drugs, opiates, dopamine, serotonin, catecholamines, thrombin, acetylcholine, prostaglandins, organic molecules, pheromones, adenosine, sucrose, glucose, lactose and galactose. Libraries of polypeptides or proteins can also be used in the assays of the invention.

In a preferred embodiment, the combinatorial libraries are small organic molecule libraries including, but not limited to, benzodiazepines, isoprenoids, thiazolidinones, metathiazanones, pyrrolidines, morpholino compounds, and benzodiazepines. In another embodiment, the combinatorial libraries comprise peptoids; random bio-oligomers; benzodiazepines; diversomers such as hydantoins, benzodiazepines and dipeptides;, vinylogous polypeptides; nonpeptidal peptidomimetics; oligocarbamates; peptidyl phosphonates; peptide nucleic acid libraries; antibody libraries; or carbohydrate libraries. Combinatorial libraries are themselves commercially available *(see, e.g.,* ComGenex, Princeton, New Jersey; Asinex, Moscow, Ru, Tripos, Inc., St. Louis, Missouri; ChemStar, Ltd, Moscow, Russia; 3D Pharmaceuticals, Exton, Pennsylvania; Martek Biosciences, Columbia, Maryland; etc.).

In a preferred embodiment, the library is preselected so that the compounds of the library are more amenable for cellular uptake. For example, compounds are selected based on specific parameters such as, but not limited to, size, lipophilicity, hydrophilicity, and hydrogen bonding, which enhance the likelihood of compounds getting into the cells. In another embodiment, the compounds are analyzed by three-dimensional or four-dimensional computer computation programs.

The combinatorial compound library for use in accordance with the methods of the present invention may be synthesized. There is a great interest in synthetic methods directed toward the creation of large collections of small organic compounds, or libraries, which could be screened for pharmacological, biological or other activity. The synthetic methods applied to create vast combinatorial libraries are performed in solution or in the solid phase, *i.e.*, on a solid support. Solid-phase synthesis makes it easier to conduct multi-step reactions and to drive reactions to completion with high yields because excess reagents can be easily added and washed away after each reaction step. Solid-phase combinatorial synthesis also tends to improve isolation, purification and screening. However, the more traditional solution phase chemistry supports a wider variety of organic reactions than solid-phase chemistry.

Combinatorial compound libraries of the present invention may be synthesized using the apparatus described in U.S. Patent No. 6,190,619 to Kilcoin et al.*,* which is hereby incorporated by reference in its entirety. U.S. Patent No. 6,190,619 discloses a synthesis apparatus capable of holding a plurality of reaction vessels for parallel synthesis of multiple discrete compounds or for combinatorial libraries of compounds.

In one embodiment, the combinatorial compound library can be synthesized in solution. The method disclosed in U.S. Patent No. 6,194,612 to Boger et al.*,* which is hereby incorporated by reference in its entirety, features compounds useful as templates for solution phase synthesis of combinatorial libraries. The template is designed to permit reaction products to be easily purified from unreacted reactants using liquid/liquid or solid/liquid extractions. The compounds produced by combinatorial synthesis using the template will preferably be small organic molecules. Some compounds in the library may mimic the effects of non-peptides or peptides. In contrast to solid phase synthesize of combinatorial compound libraries, liquid phase synthesis does not require the use of specialized protocols for monitoring the individual steps of a multistep solid phase synthesis (Egner et al., 1995, J.Org. Chem. 60:2652; Anderson et al., 1995, J. Org. Chem. 60:2650; Fitch et al., 1994, J. Org. Chem. 59:7955; Look et al., 1994, J. Org. Chem. 49:7588; Metzger et al., 1993, Angew. Chem., Int. Ed. Engl. 32:894; Youngquist et al., 1994, Rapid Commun. Mass Spect. 8:77; Chu et al., 1995, J. Am. Chem. Soc. 117:5419; Brummel et al., 1994, Science 264:399; and Stevanovic et al., 1993, Bioorg. Med. Chem. Lett. 3:431).

Combinatorial compound libraries useful for the methods of the present invention can be synthesized on solid supports. In one embodiment, a split synthesis method, a protocol of separating and mixing solid supports during the synthesis, is used to synthesize a library of compounds on solid supports (see *e.g.,* Lam et al., 1997, Chem. Rev. 97: 41-448; Ohlmeyer et al., 1993, Proc. Natl. Acad. Sci. USA 90:10922-10926 and references cited therein): Each solid support in the final library has substantially one type of compound attached to its surface. Other methods for synthesizing combinatorial libraries on solid supports, wherein one product is attached to each support, will be known to those of skill in the art *(see, e.g.,* Nefzi et al., 1997, Chem. Rev. 97:449-472).

As used herein, the term "solid support" is not limited to a specific type of solid support. Rather a large number of supports are available and are known to one skilled in the art. Solid supports include silica gels, resins, derivatized plastic films, glass beads, cotton, plastic beads, polystyrene beads, alumina gels, and polysaccharides. A suitable solid support may be selected on the basis of desired end use and suitability for various synthetic protocols. For example, for peptide synthesis, a solid support can be a resin such as p-methylbenzhydrylamine (pMBHA) resin (Peptides International, Louisville, KY), polystyrenes (*e.g.*, PAM-resin obtained from Bachem Inc., Peninsula Laboratories, etc.), including chloromethylpolystyrene, hydroxymethylpolystyrene and aminomethylpolystyrene, poly (dimethylacrylamide)-grafted styrene co-divinyl-benzene (*e.g.*, POLYHIPE resin, obtained from Aminotech, Canada), polyamide resin (obtained from Peninsula Laboratories), polystyrene resin grafted with polyethylene glycol (*e.g.*, TENTAGEL or ARGOGEL, Bayer, Tubingen, Germany) polydimethylacrylamide resin (obtained from Milligen/Biosearch, California), or Sepharose (Pharmacia, Sweden).

In some embodiments of the present invention, compounds can be attached to solid supports via linkers. Linkers can be integral and part of the solid support, or they may be nonintegral that are either synthesized on the solid support or attached thereto after synthesis. Linkers are useful not only for providing points of compound attachment to the solid support, but also for allowing different groups of molecules to be cleaved from the solid support under different conditions, depending on the nature of the linker. For example, linkers can be, *inter alia,* electrophilically cleaved, nucleophilically cleaved, photocleavable, enzymatically cleaved, cleaved by metals, cleaved under reductive conditions or cleaved under oxidative conditions. In a preferred embodiment, the compounds are cleaved from the solid support prior to high throughput screening of the compounds.

In certain embodiments of the invention, the test compound is a small molecule.

## 5.4 SCREENING ASSAYS TO IDENTIFY COMPOUNDS THAT MODULATE ONE OR MORE COMPONENTS OF THE tRNA SPLICING PATHWAY

Various assays can be used to identify and verify the ability of a compound to modulate the activity of one or more component (*e.g.*, enzymes) in the tRNA splicing pathway. In some embodiments, the invention encompasses assays to identify and verify the ability of a compound to modulate, *e.g.*, inhibit, the activity of an animalia tRNA splicing endonuclease or a subunit thereof. In other embodiments, the invention encompasses assays to identify and verify the ability of a compound to modulate *e.g.*, inhibit, the activity of a fungal tRNA splicing endonuclease or a subunit thereof. In other embodiments, the invention encompasses assays to identify and verify the ability of a compound to modulate, *e.g.*, inhibit, the activity of an animalia tRNA splicing ligase or a biologically fragment thereof. In yet other embodiments the invention encompasses asays to identify and verify the ability of a compound to modulate, *e.g.*, inhibit the activity of a fungal tRNA splicing ligase in a biologically active fragment thereof.

The invention encompasses *in vitro* based assays as well as *in vivo* based assays for identifying and verifying the ability of a compound to modulate the activity of one or more enzymes in the tRNA splicing pathway. In some embodiments, multiple *in vitro* assays can be performed simultaneously or sequentially to assess the effect of a compound on the activity of one or more components (*e.g.*, enzymes) in the tRNA splicing pathway. In a preferred embodiment, the *in vitro* assays described herein are performed in a high throughput format.

## 5.4.1 REPORTER GENE-BASED ASSAYS

## 5.4.1.1 CELL-BASED ASSAYS

After a vector containing the reporter gene construct is transformed or transfected into a host cell and a compound library is synthesized or purchased or both, the cells are used to screen the library to identify compounds that modulate the activity of one or more components (eg., enzymes) in the tRNA splicing pathway in an indirect cell-based assay. The invention further encompasses characterizing the specific target of the compounds that modulate one or more enzymes in the tRNA splicing pathway by a more direct assay as described herein (*e.g.*, cell-free assay). In some embodiments, the compounds modulate the activity of an animalia and/or fungal tRNA splicing endonuclease. In yet other embodiments, the compounds modulate the activity of an animalia and/or fungal tRNA splicing ligase.

The reporter gene-based assays may be conducted by contacting a compound or a member of a library of compounds with a cell genetically engineered to contain a reporter gene construct comprising a reporter gene and a tRNA intron within the open reading frame of the reporter gene, or within the 5' untranslated region, 3' untranslated region or both the 5' and 3" untranslated regions of the reporter gene construct, or within a mRNA splice site of the reporter gene; and measuring the expression of said reporter gene. The alteration in reporter gene expression relative to a previously determined reference range, the absence of the compound or a control in such reporter-gene based assays indicates that a particular compound modulates the activity of one or more components (*e.g.*, enzymes) in a tRNA splicing pathway. A decrease in reporter gene expression relative to a previously determined reference range, the absence of the compound or a control in such reporter-gene based assays indicates that a particular compound reduces or inhibits the activity of one or more enzymes in a tRNA splicing pathway (*e.g.*, reduces or inhibits the activity of a tRNA splicing endonulease for example the recognition or cleavage of a tRNA intron by a tRNA splicing endonuclease). An increase in reporter gene expression relative to a previously determined reference range, the absence of the compound or a control in such reporter-gene based assays indicates that a particular compound enhances the activity of one or more components (*e.g.*, enzymes) in a tRNA splicing pathway. In a preferred embodiment, a negative control (*e.g.*, phosphate buffered saline ("PBS")) or another agent that is known to have no effect on the expression of the reporter gene) and a positive control (*e.g.*, an agent that is know to have an effect on the expression of the reporter gene, preferably an agent that affects the activity of one or more components (*e.g.*, enzymes) in a tRNA splicing pathway) are included in the cell-based assays described herein.

The step of contacting a compound or a member of a library of compounds with a cell genetically engineered to contain a reporter gene construct comprising a reporter gene and a tRNA intron within the open reading frame of the reporter gene, within the 5' untranslated region, 3' untranslated region or both the 5' and 3' untranslated regions of the reporter gene construct or within a mRNA splice site is preferably conducted under physiologic conditions. In a specific embodiment, a compound or a member of a library of compounds is added to the cells in the presence of an aqueous solution. In accordance with this embodiment, the aqueous solution may comprise a buffer and a combination of salts, preferably approximating or mimicking physiologic conditions. Alternatively, the aqueous solution may comprise a buffer, a combination of salts, and a detergent or a surfactant. Examples of salts which may be used in the aqueous solution include, but are not limited to, KCl, NaCl, and/or $MgCl_2$. The optimal concentration of each salt used in the aqueous solution is dependent on the cells and compounds used and can be determined using routine experimentation. The step of contacting a compound or a member of a library of compounds with a cell genetically engineered to contain the reporter gene construct may be performed for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day.

In one embodiment, the invention provides a method for identifying a compound that modulates the activity of one or more components (*e.g.*, enzymes) in the tRNA splicing pathway, said method comprising: (a) expressing a nucleic acid comprising a reporter gene in a cell, wherein the reporter gene comprises a tRNA intron; (b) contacting said cell with a member of a library of compounds; (c) detecting the expression of said reporter gene, wherein a compound that modulates the activity of one or more components in the tRNA splicing pathway is identified if the expression of said reporter gene in the presence of a compound is altered relative to a previously determined reference range or the expression of said reporter gene in the absence of the compound or the presence of a control; and (d) identifying the component (*e.g.*, enzymes) the tRNA splicing pathway that is modulated by the compound using an assay such as those described herein. In some embodiments, the compounds modulate a tRNA splicing endonuclease activity and/or tRNA splicing ligase activity as determined by direct assays described herein or known to those skilled in the art.

In another embodiment, the invention provides a method for identifying a compound that modulates the activity of one or more components in the tRNA splicing pathway, said method comprising: (a) contacting a member of a library of compounds with a cell containing a nucleic acid comprising a reporter gene, wherein the reporter gene comprises a tRNA intron; and (b) component (*e.g.*, enzymes)detecting the expression of said reporter gene, wherein a compound that modulates the activity of one or more components in the tRNA splicing pathway is identified if the expression of said reporter gene in the presence of a compound is altered relative to a previously determined reference range the expression of said reporter gene in the absence of said test compound or the presence of a control; and (c) identifying the c component (*e.g.*, enzymes) in the tRNA splicing pathway that is modulated by the compound using an assay such as those described in sections XXX.

The expression of a reporter gene in the cell-based reporter-gene assays may be detected by any technique well-known to one of skill in the art. Methods for detecting the expression of a reporter gene expression will vary with the reporter gene used. Assays for the various reporter genes are well-known to one of skill in the art. The expression of a reported gene may be detected by assaying for the RNA and /or protein expression of the reporter gene or asaying for an activity associated with the expressed reporter gene. For example, as described in Section 5.1, luciferase, beta-galactosidase ("b-gal"), beta-glucoronidase ("GUS"), beta-lactamase, chloramphenicol acetyltransferase ("CAT"), and alkaline phosphatase ("AP") are enzymes that can be analyzed in the presence of a substrate and are amenable to high throughput screening. The reaction products of luciferase, beta-galactosidase ("b-gal"), and alkaline phosphatase ("AP") are assayed by, *e.g.*, changes in light imaging (*e.g.*, luciferase), spectrophotometric absorbance (*e.g.*, b-gal), or fluorescence (*e.g.*, AP). Assays for changes in light output, absorbance, and/or fluorescence are easily adapted for high throughput screening. For example, b-gal activity can be measured with a microplate reader. Green fluorescent protein ("GFP") activity can be measured by changes in fluorescence. In the case of mutant GFPs that fluoresce at 488 nm, standard fluorescence activated cell sorting ("FACS") equipment can be used to separate cells based upon GFP activity.

Alterations in the expression of a reporter gene may be determined by comparing the level of expression of the reporter gene to a negative control (*e.g.*, PBS or another agent that is known to have no effect on the expression of the reporter gene) and optionally, a positive control (*e.g.*, an agent that is known to have an effect on the expression of the reporter gene). Alternatively, alterations in the expression of a reporter gene may be determined by comparing the level of expression of the reporter gene to a previously determined reference range.

The expression of a reporter gene can be readily detected, *e.g.*, by quantifying the protein and/or RNA encoded by said gene. Many methods standard in the art can be thus employed, including, but not limited to, immunoassays to detect and/or visualize gene expression (*e.g.*, Western blot, immunoprecipitation followed by sodium dodecyl sulfate polyacrylamide gel electrophoresis (SDS-PAGE), immunocytochemistry, etc) and/or hybridization assays to detect gene expression by detecting and/or visualizing respectively mRNA encoding a gene (*e.g.*, Northern assays, dot blots, *in situ* hybridization, etc), etc. Such assays are routine and well known in the art *(see, e.g.,* Ausubel et al, eds, 1994, Current Protocols in Molecular Biology, Vol. 1, Jolm Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1% sodium deoxycholate, 0.1% SDS, 0.15 M NaCl, 0.01 M sodium phosphate at pH 7.2, 1 % Trasylol) supplemented with protein phosphatase and/or protease inhibitors (*e.g.*, EDTA, PMSF, aprotinin, sodium vanadate), adding the antibody to the cell lysate, incubating for a period of time (*e.g.*, 1 to 4 hours) at 40° C, adding protein A and/or protein G sepharose beads to the cell lysate, incubating for about an hour or more at 40°. C, washing the beads in lysis buffer and resuspending the beads in SDS/sample buffer. The ability of the antibody to immunoprecipitate a particular antigen can be assessed by, *e.g.*, western blot analysis. One of skill in the art would be knowledgeable as to the parameters that can be modified to increase the binding of the antibody to an antigen and decrease the background (*e.g.*, pre-clearing the cell lysate with sepharose beads). For further discussion regarding immunoprecipitation protocols *see, e.g.,* Ausubel et al, eds, 1994, Current Protocols in Molecular Biology, Vol. 1, John Wiley Sons, Inc., New York at 10.16.1.

Western blot analysis generally comprises preparing protein samples, electrophoresis of the protein samples in a polyacrylamide gel (*e.g.*, 8%- 20% SDS-PAGE depending on the molecular weight of the antigen), transferring the protein sample from the polyacrylamide gel to a membrane such as nitrocellulose, PVDF or nylon, blocking the membrane in blocking solution (*e.g.*, PBS with 3% BSA or non-fat milk), washing the membrane in washing buffer (*e.g.*, PBS-Tween 20), blocking the membrane with primary antibody (which recognizes a particular antigen) diluted in blocking buffer, washing the membrane in washing buffer, blocking the membrane with a secondary antibody (which recognizes the primary antibody, *e.g.*, an anti-human antibody) conjugated to an enzymatic substrate (*e.g.*, horseradish peroxidase or alkaline phosphatase) or radioactive molecule (*e.g.*, $^{32}$P or $^{125}$I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols *see, e.g.,* Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding a primary antibody (which recognizes a particular antigen) conjugated to a detectable compound such as an enzymatic substrate (*e.g.*, horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the primary antibody) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs *see, e.g.,* Ausubel et al, eds, 1994, Current Protocols in Molecular Biology, Vol. 1, John Wiley & Sons, Inc., New York at 11.2.1.

### 5.4.1.2 CELL-FREE ASSAYS

After a vector containing the reporter gene construct is produced, a cell-free translation extract is generated or purchased, and a compound library is synthesized or purchased or both, the cell-free translation extract and nucleic acid are used to screen the library to identify compounds that modulate the activity of one or more components (*e.g.*, enzymes) in the tRNA splicing pathway in an indirect cell-free assay. The invention further encompasses characterizing the specific target of the compounds that modulate one or more components (*e.g.*, enzymes) of the tRNA splicing pathway by a more direct assay as described herein.

The reporter gene-based assays may be conducted in a cell-free manner by contacting a compound with a cell-free extract and a reporter gene construct comprising a reporter gene and a tRNA intron within the open reading frame of the reporter gene or within the 5' untranslated region, 3' untranslated region or both the 5' and 3' untranslated regions of the reporter gene construct, or in a mRNA splicing site of the reporter gene, and measuring the expression of said reporter gene. The alteration in reporter gene expression relative to a previously determined reference range, the absence of the compound or a control in such reporter-gene based assays indicates that a particular compound modulates the activity of one or more components (*e.g.*, enzymes) in a tRNA splicing pathway. A decrease in reporter gene expression relative to a previously determined reference range, the absence of the compound or a control in such reporter-gene based assays indicates that a particular compound reduces or inhibits the activity of one or more components (*e.g.*, enzymes) in a tRNA splicing pathway (*e.g.*, reduces or inhibits the activity of a tRNA splicing endonuclease by, for example, inhibiting or reducing the recognition or cleavage of a tRNA intron by a tRNA splicing endonuclease). An increase in reporter gene expression relative to a previously determined reference range, the absence of the compound or a control in such reporter-gene based assays indicates that a particular compound enhances the activity of one or more components (*e.g.*, enzymes) in a tRNA splicing pathway. In a preferred embodiment, a negative control (*e.g.*, FBS or another agent that is known to have no effect on the expression of the reporter gene) and a positive control (*e.g.*, an agent that is known to have an effect on the expression of the reporter gene, preferably an agent that affects the activity of one or more components a tRNA splicang.pathway) are included in the cell-free assays described herein.

In a specific embodiment, the invention provides a method for identifying a compound that modulates the activity of one or more components (*e.g.*, enzymes) in a tRNA splicing pathway, said method comprising: (a) contacting a member of a library of compounds with a cell-free extract and a nucleic acid comprising a reporter gene, wherein the reporter gene comprises a tRNA intron; and (b) detecting the expression of said reporter gene, wherein a compound that modulates the activity of one or more components (*e.g.*, enzymes) in a tRNA splicing pathway is identified if the expression of said reporter gene in the presence of a compound is altered relative to a previously determined reference range or the expression of said reporter gene in the absence of said compound or the presence of a control.

In another embodiment, the invention provides a method for identifying a compound that modulates the activity of one or more components (eg., enzymes) in a tRNA splicing pathway, said method comprising: (a) contacting a member of a library of compounds with a cell-free extract and a nucleic acid c omprising a reporter gene, wherein the reporter gene comprises a tRNA intron; (b) detecting the expression of said reporter gene, wherein a compound that modulates the activity of one or more components (*e.g.*, enzymes) in a tRNA splicing pathway is identified if the expression of said reporter gene in the presence of a compound is altered relative to a previously determined reference range, or the expressions of said reporter gene in the absence of said compound or the presence of a control; and (c) identifying the component (*e.g.*, enzyme) in the tRNA splicing pathway that is modulated by the compound using an assay such as those described in Section 5.4.6.

The activity of a compound in the cell-free extract can be determined by assaying the activity of a reporter protein encoded by a reporter gene, or alternatively, by quantifying the expression of the reporter gene by, for example, labeling the *in vitro* translated protein (*e.g.*, with $^{35}$S-labeled methionine), northern blot analysis, RT-PCR or by immunological methods, such as western blot analysis or immunoprecipitation. Such methods are well-known to one of skill in the art.

### 5.4.2 FRET ASSAY

Fluorescence resonance energy transfer ("FRET") assays can be used to detect alterations in the activity of one or more components (*e.g.*, enzymes) in a tRNA splicing pathways. FRET based assays rely for signal generation on fluorescence resonance energy transfer, according to which a change in fluorescence is caused by a change in the distance separating a first fluorophore from an interacting resonance energy acceptor, either another fluorophore (a donor) or a quencher. Combinations of a fluorophore and an interacting molecule or moiety, including quenching molecules or moieties, are known as "FRET pairs" and are known to those skilled in the art.

The mechanism of FRET-pair interaction requires that the absorption spectrum of one member of the pair overlaps the emission spectrum of the other member, the first fluorophore. If the interacting molecule or moiety is a quencher, its absorption spectrum must overlap the emission spectrum of the fluorophore *(See,* Stryer, L.,Ann. Rev. Biochem. 1978, 47: 819-846; BIOPHYSICAL CHEMISTRY part II, Techniques for the Study of Biological Structure and Function, C. R. Cantor and P. R. Schimmel, pages 448-455 (W. H. Freeman and Co., San Francisco, U.S.A., 1980); Selvin, P. R., 1995, Methods in Enzymology 246: 300-335; all of which are incorporated herein by reference). Efficient, or a substantial degree of, FRET interaction requires that the absorption and emission spectra of the pair have a large degree of overlap. The efficiency of FRET interaction is linearly proportional to that overlap (Haugland et al., P.N.A.S. U.S.A. 63: 24-30 (1969). To obtain a large magnitude of signal, a high degree of overlap is required. FRET pairs, including fluorophore-quencher pairs, have been chosen on that basis.

Any of a number of fluorophore combinations can be selected for use in the present invention *(see* for example, Pesce et al,. eds, Fluorescence Spectroscopy, Marcel Dekker, New York, 1971; White et al., Fluorescence Analysis: A practical Approach, Marcel Dekker, New York, 1970; Handbook of Fluorescent Probes and Research Chemicals, 6th Ed, Molecular Probes, Inc., Eugene, Oreg., 1996; which are incorporated by reference). In general, a preferred donor fluorophore is selected that has a substantial spectrum of the acceptor fluorophore. Furthermore, in may also be desirable in certain applications that the donor have an excitation maximum near a laser frequency such as Helium-Cadmium 442 nM or Argon 488 nM. In such applications the use of intense laser light can serve as an effective means to excite the donor fluorophore. The acceptor fluorophore has a substantial overlap of its excitation spectrum with the emission spectrum of the donor fluorophore. In addition, the wavelength maximum of the emission spectrum of the acceptor moiety is preferably at least 10 nm greater than the wavelength maximum of the excitation spectrum of the donor moiety. The emission spectrum of the acceptor fluorophore is typically in the red portion of the visible spectrum, although, it is believed that acceptor fluorophores having emission at longer wavelengths in the infrared region of the spectrum can be used.

In order to obtain FRET between the fluorescent donor moiety and the fluorescent acceptor moiety or a quencher, the two moieties have to be in spatial proximity with each other. Thus, in certain embodiments, a substrate for an animalia or fungal tRNA splicing endonuclease is labeled such that the fluorescent donor moiety and the fluorescent acceptor moiety or a quencher are at most 0.5 nm, at most 1 nm, at most 5 nm, at most 10 nm, at most 20 nm, at most 30 nm, at most 40 nm, at most 50 nm or at most 100 nm apart from each other.

### 5.4.2.1 CELL-BASED ASSAYS WITH A LABELED SUBSTRATE

FRET-cell based assays may be conducted by microinjecting or transfecting (*e.g.*, using liposomes or electroporation) a substrate for an animalia or fungal tRNA splicing endonuclase into a cell and contacting the cell with a compound, wherein the substrate is labeled at the 5' end with a fluorophore and labeled at the 3' end with a quencher, or the substrate is labeled at the 5' end with a quencher and labeled at the 3' end with a fluorophore, and measuring the fluorescence of the substrate by, *e.g.*, fluorescence microscopy or a fluorescence emission detector such as a Viewlux or Analyst. The endogenous tRNA splicing endonuclease will cleave the substrate and result in the production of a detectable fluorescent signal. The endogenous tRNA splicing ligase will ligate the tRNA half molecules generated from the endonuclease cleavage of the substrate and quench the production of a fluorescent detectable signal. Thus, in order to determine if the compound modulates the tRNA splicing endonuclease activity, the reaction can be carried out in the presence of a known inhibitor of tRNA splicing ligase (*e.g.*, an antibody that specifically binds for the tRNA splicing ligase) or a cell deficient in tRNA splicing ligase activity, so that the reaction does not proceed beyond the endonuclease cleavage of the tRNA substrate. A compound that inhibits or reduces the activity of the endogenous tRNA splicing endonuclease will inhibit or reduce the cleavage of the substrate and thus, inhibit or reduce the production of a detectable fluorescent signal relative to a negative control (*e.g.*, PBS). A compound that enhances the activity of the endogenous endonuclease will enhance the cleavage of the substrate and thus, increase the production of a detectable signal relative to a negative control (*e.g.*, PBS). In order to determine if the compound modulates the tRNA splicing ligase activity, the reaction is allowed to proceed to completion. The endogenous tRNA splicing ligase will join (*i.e.*, ligate) the tRNA half molecules generated into mature tRNA. A compound that inhibits or reduces the activity of the tRNA splicing ligase will inhibit or reduce the ligation of the tRNA half molecules and thus, maintain or enhance the production of a detectable fluorescent signal relative to a negative control (*e.g.*, PBS compound that enhances the activity of a tRNA splicing ligase will enhance the ligation of the tRNA half molecules and thus, decrease the detectable fluorescent signal relative to a negative control (*e.g.*, PBS).

Although not intending to be bound by any mechanism, this assay is based on the theory that the 5' and 3' termini of a tRNA splicing endonuclease substrate are in close spatial proximity such that the fluorescent signal is quenched as a result of the proximity of 5' and 3' termini. It can be appreciated by one skilled in the art that the assay may be modified depending on the particular structural configuration of the substrate.

Alternatively, FRET-cell-based assays may be conducted by microinjecting or transfecting a substrate for an animalia or fungal tRNA splicing endonuclease into a cell and contracting the cell with a compound, wherein the substrate is labeled at the 5' end with a fluorescent donor moiety and labeled at the 3' end with a fluorescent acceptor moiety, or the substrate is labeled at the 5' end with a fluorescent acceptor moiety and labeled at the 3' end with a fluorescent donor moiety, and measuring the fluorescence of the substrate by, *e.g.*, fluorescence microscopy or a fluorescence emission detector such as a Viewlux or Analyst. The endogenous tRNA splicing endonuclease will cleave the substrate and result in the reduction of a detectable fluorscent signal by the fluorescent donor moiety and the fluorescent acceptor moiety at the wavelength of the fluorescent donor moiety. The endogenous tRNA splicing ligase will ligate the tRNA half molecules generated from the endonuclease cleavage of the substrate and induce the production of a detectable fluorescent signal by the fluorescent donor moiety and the fluorescent acceptor moiety at the wavelength of the fluorescent donor moiety. Thus, in order to determine if the compound modulates the tRNA splicing endonuclease activity the reaction can be carried out in the presence of a known inhibitor of tRNA splicing ligase (*e.g.*, an antibody that spcifically binds to the tRNA splicing ligase), or in a cell deficient in tRNA splicing ligase activity, so that reaction does not proceed beyond the endonuclease cleavage of the tRNA substrate. A compound that inhibits or reduces the activity of the endogenous tRNA splicing endonuclease will inhibit or reduce the cleavage of the substrate and thus, maintain or increase the fluorescence emission of the fluorescent acceptor moiety at the wavelength of the fluorescent donor moiety relative to a negative control (*e.g.*, PBS). A compound that enhances the activity of the endogenous tRNA splicing endonuclease will enhance the cleavage of the substrate and thus, reduce the fluorescence emission of the fluorescent acceptor moiety at the wavelength of the fluorescent donor moiety relative to a negative control (*e.g.*, PBS). In order to determine if the compound modulates the tRNA splicing ligase activity, the reaction is allowed to proceed to completion. The endogenous tRNA splicing ligase will join (*i.e.*, ligate) the tRNA half molecules generated into nature tRNA. A compound that inhibits or induces the activity of the tRNA splicing ligase will inhibit the ligation of the tRNA half molecules and thus, reduce the fluorescence emission of the fluorescent acceptor moiety at the wavelength of the fluorescent donor moiety relative to a negative control. A compound that enhances the activity a tRNA splicing ligase will enhance the ligation of the tRNA half molecules and thus, enhance the fluorescence emission of the fluorescent acceptor moiety at the wavelength of the fluorescent donor moiety.

The assay can be conducted in any buffer system that provides conditions conducive to the activity of tRNA splicing pathway. Such buffer systems are well-known to one skilled in the art. In a specific embodiment, the buffer is the medium in which the cell culture is kept. Care should be taken to include magnesium ions in the medium.

In certain embodiments, the assay is conducted for at least 0.2 hours, 0.25 hours, 0.5 hours, 1 hour, 2 hours, 3 hours, 4 hours, 5 hours, 6 hours, 8 hours, 10 hours, 12 hours, 18 hours or at least one day.

Any nucleotide sequence recognized and excised by an animalia and/or fungal tRNA splicing endonuclease may be utilized as a substrate in a FRET assay described herein. For example, a nucleotide sequence comprising a bulge-helix-bulge structure or a mature domain of a precursor tRNA may be utilized as a substrate for an animalia tRNA splicing endonuclease in a FRET assay described herein. A nucleotide sequence comprising a bulge-helix-bulge structure may be utilized as a substrate for a fungal tRNA splicing endonuclease. A nucleotide sequence recognized and excised by an animalia and/or fungal tRNA splicing endonuclease may comprise 10 nucleotides, 15 nucleotides, 20 nucleotides, 25 nucleotides, 25 nucleotides, 30 nucleotides, 40 nucleotides, 45 nucleotides, 50 nucleotides, 55 nucleotides, 60 nucleotides, 65 nucleotides, 75 nucleotides, 100 nucleotides, 125 nucleotides, 150 nucleotides, or more. In a specific embodiment, the substrates for a tRNA splicing endonuclease utilized in the FRET assays described herein comprise a tRNA intron. The substrate may comprise a bulge-helix-bulge conformation. In a specific embodiment, the substrate comprises a tRNA mature domain that contains an intron.

In a preferred embodiment, the tRNA substrate depicted in Figure 1B or Figure 1C are utilized in the FRET assays described herein. The free 5' and 3' ends of the intron of the hybridized tRNA substrate (Figure 1B) or the free 5' and 3' ends of the intron of circularly permuted tRNA substrate (Figure 1C) are labeled with a fluorophore such that the close spatial proximity of the fluorophore on the 5' end with the fluorophore on the 3' end results in fluorescence resonance energy transfer. Cleavage of the substrate by tRNA splicing endonuclease will then result in a spatial separation of the labeled 5' end from the labeled 3' end and thus, in reduced fluorescence resonance energy transfer. Ligation of the tRNA half molecules will result in close proximity of the fluorophore on the 5' end with the fluorophore on the 3' end and thus, in increased fluorescence resonance energy transfer. Alternatively, either the 5' or 3' end of the intron of the hybridized tRNA substrate (Figure 1B) or the intron of circularly permuted tRNA substrate (Figure 1 C) are labeled with a quencher and the other end is labeled with a fluorophore such that the close proximity of the quencher and fluorophore results the inhibition of a detectable fluorescent signal. Cleavage of the substrate by tRNA splicing endonuclease will then result in a spatial separation of the labeled 5' end from the labeled 3' end and thus, in increased production of a detectable fluorescent signal. Ligation of the tRNA half molecules will result in close proximity of the 5' end and the 3' end and thus, in decreased production of a detectable fluorescent signal.

In accordance with the invention, the substrate can be labeled with a single pair of fluorescent donor and acceptor moieties. Alternatively, the substrate can be labeled with different pairs of fluorescent donor moieties and fluorescent acceptor moieties. For example, two, three, four, five or more pairs of fluorescent donor moieties and fluorescent acceptor moieties can be used. In this situation, preferably, at least one of the pairs comprise a fluorescent acceptor moiety that has a different emission spectrum from the fluorescent acceptor moiety of at least one of the other pairs. Alternatively, when at least three pairs are used, the fluorescent acceptor moiety of the first pair, second pair and third pair has a different emission spectrum than the fluorescent acceptor moiety of the other two. Methods for labeling the substrate with a fluorescent acceptor moiety, a fluorescent donor moiety and/or quencher are well-known in the art (see, *e.g.*, U.S. Patent Nos. 6,472,156, 6,451,543, 6,348,322, 6,342,379, 6,323,039, 6,297,018, 6,291,201, 6,280,981, 5,843,658, and 5,439,797, the disclosures of which are incorporated by reference in their entirety). The labeled substrate can be microinjected or transfected into fungal or animalia cells (preferably, mammalian cells and more preferably, human cells) utilizing techniques well-known to one of skill in the art (see, *e.g.*, Adams et al., 1991, Nature 349:694-697).

A substrate of a tRNA splicing endonuclease can be labeled by any method known to the skilled artisan. In certain embodiments, a substrate of an tRNA splicing endonuclease can be labeled using site-specific labeling of RNA with fluorophores. In more specific embodiments, a substrate of a tRNA splicing endonuclease is labeled using the methods: described in Qin and Pyle, 1999 (Methods 18(1):60-70), which is incorporated herein by reference in its entirety. The optimal route for labeling of a substrate of a tRNA splicing endonuclease can be determined by the skilled artisan using routine experimentation. In a specific embodiment, a substrate of a tRNA splicing endonuclease is labeled using different methods, different labels and/or different positions in the tRNA substrate for labeling. The differently labeled substrates are then subjected separately to a splicing assay in the presence and absence, respectively of an inhibitor or an activator of a tRNA splicing endonuclease. The optimal label for the screening assays is the label that is the most easily detectable and allows for the most reproducible detection of the effect of the inhibitor or the activator. Additionally, the sensitivity of the detection of the label may be enhanced using common methods known to one skilled in the art depending on the label used, by routine experimentation. Other labeling procedures, however, may also be used with the methods of the invention, especially, if they provide other desirable advantages.

### 5.4.2.2 <u>CELL-FREE ASSAYS WITH LABELED SUBSTRATE</u>

The FRET cell-free assays may be conducted by contacting a substrate for an animalia or fungal tRNA splicing endonuclease with a cell-free extract and a compound, wherein the substrate is labeled at the 5' end with a fluorophore and labeled at the 3' end with a quencher or, alternatively, the substrate is labeled at the 3' end with a fluorophore and labeled at the 5' end with a quencher, and measuring the fluorescence of the substrate in, *e.g.*, a fluorescence emission detector such as a Viewlux or Analyst. The tRNA splicing endonuclease in the cell-free extract will cleave the substrate and result in the production of a detectable fluorescent signal. The tRNA splicing ligase in the cell-free extract will ligate the tRNA half molecules generated from the endonuclease cleavage of the substrate and quench the production of a detectable fluorescent signal. Thus, in order to determine if the compound modulates the tRNA splicing endonuclease activity, the reaction can be carried out in the presence of a known inhibitor of tRNA splicing ligase (*e.g.*, an antibody that specifically binds to the tRNA splicing ligase), or in a cell-free extract deficient in tRNA splicing ligase activity, so that the reaction does not proceed beyond the cleavage of the tRNA substrate. In some embodiments, the activity of a RNA splicing ligase is inhibited or reduced by excluding ATP from the reaction mixture. Although not intending to be bound by a particular mechanism of action, since the activity of tRNA splicing ligase is dependent on the presence of ATP, excluding ATP from the reaction effectively reduces the activity of the tRNA splicing ligase. A compound that inhibits or reduces the activity of the tRNA splicing endonuclease will inhibit or reduce the cleavage of the substrate and thus, inhibit or reduce the production of a detectable fluorescent signal relative to a negative control (*e.g.*, PBS). A compound that enhances the activity of the tRNA splicing endonuclease will enhance the cleavage of the substrate and thus, increase the production of a detectable signal relative to a negative control (*e.g.*, PBS). In order to determine if the compound modulates the tRNA ligase activity, the reaction is allowed to proceed to completion. The tRNA splicing ligase will join (*i.e.,* ligate) the tRNA half molecules generated into mature tRNA. A compound that inhibits or reduces the activity of the tRNA splicing ligase will inhibit or reduce the ligation of the tRNA half molecules and thus, maintain or enhance the production of a detectable fluorescent signal relative to a negative control (*e.g.*, PBS). A compound that enhances the activity of a tRNA splicing ligase will enhance the ligation of the tRNA half molecules and thus, decrease the detectable fluorescent signal relative to a negative control (*e.g.*, PBS).

Alternatively, the FRET cell-free-based assays may be conducted by contacting a substrate for an animalia or fungal tRNA splicing endonuclease with a cell-free extract and a compound, wherein the substrate is labeled at the 5' end with a fluorescent donor moiety and labeled at the 3' end with a fluorescent acceptor moiety, or alternatively, the substrate is labeled at the 5' end with a fluorescent acceptor moiety and labeled at the 3' end with a fluorescent donor moiety, and measuring the fluorescence of the substrate by, *e.g.*, a fluorescence emission detector such as a Viewlux or Analyst. The tRNA splicing endonuclease will cleave the substrate and result in the reduction of a detectable fluorescent signal by the fluorescent donor moiety and fluorescent acceptor moiety at the wavelength of the fluorescent donor moiety. The tRNA splicing ligase in the cell-free extract will ligate the tRNA half molecules generated from the endonuclease cleavage of the substrate and increase the fluorescence emission of the fluorescent acceptor moiety at the wavelength of the fluorescent donor moiety relative to a negative control (*e.g.*, PBS). Thus, in order to determine if the compound modulates the tRNA splicing endonuclease activity, the reaction can be carried out in the presence of a known inhibitor of tRNA splicing ligase (*e.g.*, an antibody that specifically binds to the tRNA splicing ligase), or in a cell-free extract deficient in tRNA splicing ligase activity, so that the reaction does not proceed beyond the cleavage of the tRNA substrate. A compound that inhibits or reduces the activity of the tRNA splicing endonuclease will inhibit or reduce the cleavage of the substrate and thus, maintain or enhance the fluorescence emission of the fluorescent acceptor moiety at the wavelength of the fluorescent donor moiety relative to a negative control (*e.g.*, PBS). A compound that enhances the activity of the tRNA splicing endonuclease will enhance the cleavage of the substrate and thus, decrease the fluorescence emission of the fluorescent acceptor moiety at the wavelength of the fluorescent donor moiety relative to a negative control (*e.g.*, PBS). In order to determine if the compound modulates the tRNA ligase activity, the reaction is allowed to proceed to completion. The tRNA splicing ligase will join *(i.e.,* ligate) the tRNA half molecules generated into mature tRNA. A compound that inhibits or reduces the activity of the tRNA splicing ligase will inhibit the ligation of the tRNA half molecules and thus, decrease the fluorescence emission of the fluorescent acceptor moiety at the wavelength of the fluorescent donor moiety relative to a negative control (*e.g.*, PBS). A compound that enhances the activity of a tRNA splicing ligase will enhance the ligation of the tRNA half molecules and thus, increase the fluorescence emission of the fluorescent acceptor moiety at the wavelength of the fluorescent donor moiety relative to a negative control (*e.g.*, PBS).

The assay can be conducted in any buffer system that provides conditions conducive to the tRNA splicing pathway. Such buffer systems are well known to the skilled artisan. In a specific embodiment, the buffer comprises 20 mM Tris at a pH of 7.0, 50 mM KCl, 0.1 mM DTT, 5 mM MgCl$_2$, and 0.4% Triton X-100. Care should be taken that pH, salt concentration, detergent concentration etc. of the buffer system do not interfere with FRET.

In certain embodiments, the assay is conducted for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day.

In accordance with the invention, the substrate can be labeled with a single pair of fluorescent donor and acceptor compounds. Alternatively, the substrate can be labeled with different pairs of fluorescent donor moieties and fluorescent acceptor moieties. For example, two, three, four, five or more pairs of fluorescent donor moieties and fluorescent acceptor moieties can be used. In this situation, preferably, at least one of the pairs comprise a fluorescent acceptor moiety that has a different emission spectrum from the fluorescent acceptor moiety of at least one of the other pairs. Alternatively, when at least three pairs are used, the fluorescent acceptor moiety of the first pair, second pair and third pair has a different emission spectrum than the fluorescent acceptor moiety of the other two. Methods for labeling the substrate with a fluorescent acceptor moiety, a fluorescent donor moiety and/or quencher are well-known in the art (see, *e.g.*, U.S. Patent Nos. 6,472,156, 6,451,54.3, 6,348,322, 6,342,379, 6,323,039, 6,297,018, 6, 291,201, 6,280,981, 5,843,658, and 5,439,797, the disclosures of which are incorporated by reference in their entirety).

### 5.4.3 tRNA SUPPRESSION ASSAYS

The effect of a compound or a member of a library of compounds on the activity of one or more components (*e.g.*, enzymes) in a tRNA splicing pathway may be determined using a tRNA endonuclease suppression assay. In such an assay, a host cell is engineered to contain a first reporter gene construct and a suppressor tRNA; the expression of the suppressor tRNA is induced; the host cell is contacted with a compound or a member of a library of compounds; and the expression of the reporter gene and/or the activity of the protein encoded by the reporter gene is measured. The first reporter gene construct comprises a reporter gene with a nonsense codon in its open reading frame such that the open reading frame is interrupted. Standard mutagenesis techniques described, *e.g.*, in Sambrook (Sambrook, 1989, Molecular Cloning, A Laboratory Manual, Second Edition; DNA Cloning, Volumes I and II (Glover, Ed. 1985)) may be used to introduce a nonsense codon into the open reading frame of any reporter gene well-known to one of skill in the art. The first reporter gene construct is transfected into a host cell engineered to contain a suppressor tRNA. Alternatively, the first reporter gene is cotransfected into a host cell with a suppressor tRNA. The suppressor tRNA's expression is regulated by a controllable regulatory element; such as by a tetracycline regulated regulatory element (*see, e.g.,* Buvoli et al, 2000, Molecular and Cellular Biology 20:3116-3124; Park and Bhandary, 1998, Molecular and Cellular Biology 18: 4418-4425) and the suppressor tRNA contains a tRNA intron in the anticodon stem such that only properly spliced suppressor tRNA is functional. Expression of functional suppressor tRNA is dependent on (i) the transcription of the suppressor tRNA, and (ii) tRNA splicing. The expression of functional suppressor tRNA suppresses the nonsense codon in the reporter gene and results in full-length, functional reporter gene expression. Accordingly, the expression of full length, functional reporter gene correlates with the expression of functional suppressor tRNA, which in turn correlates with the level of transcription of the suppressor tRNA and tRNA splicing. The expression of full-length reporter gene and the activity of the protein encoded by the reporter gene can be assayed by any method well-known to the skilled artisan or as described herein.

A compound that inhibits or reduces the activity of one or more components (*e.g.*, enzymes) in a tRNA splicing pathway will inhibit or reduce the production of functional suppressor tRNA and thus, reduce the expression of the reporter gene relative to a previously determined reference range or the expression of reporter gene in the absence of the compound in the presence of a control. A compound that enhances the activity of one or more components (*e.g.*, enzymes) in a tRNA splicing pathway will enhance the production of functional suppressor tRNA and thus, enhance the production of the reporter gene relative to a previously determined reference range, or the expression of the reporter gene in the absence of the compound or the presence of a control.

The step of inducing the expression of the suppressor tRNA may be conducted simultaneously with the step of contacting the host cell with a compound or at least 5 minutes, at least 15 minutes, at least 0.5 hours, at least 1 hour, at least 1.5 hours, at least 2 hours, at least 3 hours, at least 4 hours, at least 5 hours, at least 6 hours, at least 8 hours, at least 10 hours or at least 12 hours before the step of contacting the compound with the host cell. In certain embodiments, the expression of the suppressor tRNA is induced by incubating the host cell with an agent such as, *e.g.*, tetracycline, for approximately 5 minutes, approximately 15 minutes, approximately 0.5 hours, approximately 1 hour, approximately 1.5 hours, approximately 2 hours, approximately 3 hours, approximately 4 hours, approximately 5 hours, 6 approximately hours, 8 approximately hours, approximately 10 hours or approximately 12 hours. In other embodiments, the host cell is contacted with the compound for approximately 5 minutes, approximately 15 minutes, approximately 0.5 hours, approximately 1 hour, approximately 1.5 hours, approximately 2 hours, approximately 3 hours, approximately 4 hours, approximately 5 hours, 6 approximately hours, 8 approximately hours, approximately 10 hours or approximately 12 hours before the addition of an agent such as tetracycline to induce suppressor tRNA expression.

Optionally, the host cell is engineered to contain a second reporter gene construct comprising a reporter gene different from the first reporter gene, wherein the second reporter gene does not contain a nonsense codon. In a specific embodiment, the reporter genes used in the tRNA endonuclease suppression assay are Red and Green Click Beetle luciferase, wherein the Red luciferase contains the nonsense codon. A host cell may be engineered to stably express the two luciferase genes and the suppressor tRNA whose expression is regulated by a controlled regulatory element (such as a tetracycline controlled regulatory element). In the absence of an agent such as tetracycline, the suppressor tRNA is not expressed and thus the red-to-green ratio is low. In the presence of an agent such as tetracycline, the suppressor tRNA is expressed and thus the red-to-green ratio increases. For a high throughput screening, cells are plated in the presence of a compound. After a certain time-period media containing an agent such as tetracycline is added to induce suppressor tRNA expression.

Compounds that inhibit one or more components (*e.g.*, enzymes) in a tRNA splicing pathway will decrease the red-to-green ratio compared to a control without the compound. Once compounds are identified in this assay that modulate the activity of one or more components (*e.g.*, enzymes) in a tRNA splicing pathway, they may be tested using one or more of the assays described herein to identify the specific component (*e.g.*, enzymes) in the tRNA splicing pathway that the compound is targeting and/or to confirm the activity of the compound.

### 5.5 SCREENING ASSAYS TO IDENTIFY COMPOUNDS THAT MODULATE TRNA SPLICING ENDONUCLEASE ACTIVITY

The present invention provides methods for identifying compounds that modulate an activity of an animalia and/or fungal tRNA splicing endonuclease. In particular, the invention provides methods for identifying a compound that inhibits the activity of an animalia and/or fungal tRNA splicing endonuclease.

The invention encompasses various *in vitro* and *in vivo* assays to identify and/or verify the ability of a compound to modulate the activity of an animalia and/or fungal tRNA endonuclease. Multiple *in vitro* assays can be performed simultaneously or sequentially to assess the effect of a compound on the activity of an animalia and/or fungal tRNA splicing endonuclease. In a preferred embodiment, the assays described herein are performed in a high throughput format.

The *in vitro* screening assays described herein can be conducted utilizing any animalia cell or fungal cell, or any purified animalia or fungal tRNA splicing endonuclease. In a specific embodiment, the animalia tRNA splicing endonuclease or animalia cell utilized in *the in vitro* assays is a mammalian tRNA splicing endonuclease (*e.g.*, non-human mammalian tRNA splicing endonuclease and human tRNA splicing endonuclease) or mammalian cell. In a preferred embodiment, the animalia tRNA splicing endonuclease or animal cell utilized in *the in vitro* assays is a human tRNA splicing endonuclease or human cell. In another embodiment, the fungal tRNA splicing endonuclease or fungal cell is a yeast tRNA splicing endonuclease or yeast cell.

### 5.5.1 FRET-BASED ASSAYS

The invention encompasses fluorescence resonance energy transfer ("FRET")-based assays to detect alterations in the activity of an animalia or fungal tRNA splicing endonuclease. In the FRET assays described herein, the subunits of an animalia or fungal tRNA splicing endonuclease or a substrate for an animalia or fungal tRNA splicing endonuclease may be labeled with fluorophores. In circumstances where a subunit(s) of an animalia tRNA splicing endonuclease has not been determined or isolated, the substrate for the animalia or fungal tRNA splicing endonuclease is labeled with fluorophores.

### 5.5.1.1 CELL-FREE ASSAYS WITH A LABELED SUBSTRATE

The FRET cell-free assays may be conducted by contacting a substrate for an animalia or a fungal tRNA splicing endonuclease with a purified animalia or fungal tRNA splicing endonuclease and a compound, wherein the substrate is labeled at the 5' end with a fluorophore and labeled at the 3' end with a quencher or, alternatively, the substrate is labeled at the 5' end with a quencher and labeled at the 3' end with a fluorophore, and measuring the fluorescence of the substrate in, *e.g.*, a fluorescence emission detector such as a Viewlux or Analyst. The tRNA splicing endonuclease will cleave the substrate and result in the production of a detectable fluorescent signal. A compound that inhibits or reduces the activity of the tRNA splicing endonuclease will inhibit or reduce the cleavage of the substrate and thus, inhibit or reduce the production of a detectable fluorescent signal relative to a negative control (*e.g.*, PBS). A compound that enhances the activity of the tRNA splicing endonuclease will enhance the cleavage of the substrate and thus, increase the production of a detectable signal relative to a negative control (*e.g.*, PBS).

Alternatively, the FRET cell-free-based assays may be conducted by contacting a substrate for an animalia or fungal tRNA splicing endonuclease with a purified animalia or fungal tRNA splicing endonuclease and a compound, wherein the substrate is labeled at the 5' end with a fluorescent donor moiety and labeled at the 3' end with a fluorescent acceptor moiety, or, alternatively the substrate is labeled at the 5' end with a fluorescent acceptor moiety and labeled at the 3' end with a fluorescent donor moiety, and measuring the fluorescence of the substrate by, *e.g.*, a fluorescence emission detector such as a Viewlux or Analyst. The tRNA splicing endonuclease will cleave the substrate and result decrease in the fluorescence emission of the fluorescent acceptor moiety at the wavelength of the fluorescent donor moiety. A compound that inhibits or reduces the activity of the tRNA splicing endonuclease will inhibit or reduce cleavage of the substrate and thus, maintain or increase the fluorescence emission of the fluorescent acceptor moiety at the wavelength of the fluorescent donor moiety relative to a negative control (*e.g.*, PBS). A compound that enhances the activity of the tRNA splicing endonuclease will enhance the cleavage of the substrate and thus, reduce the fluorescence emission of the fluorescent acceptor moiety at the wavelength of the fluorescent donor moiety relative to a negative control (*e.g.*, PBS). In a preferred embodiment, a negative control (*e.g.*, PBS or another agent that is known to have no effect on the cleavage of the substrate) and a positive control (*e.g.*, an agent that is known to have an effect on the cleavage of the substrate) are included in the FRET cell-free assays described herein.

The assay can be conducted in any buffer system that provides conditions conducive to the tRNA endonuclease reaction. Such buffer systems are well known to the skilled artisan. In a specific embodiment, the buffer comprises 20 mM Tris at pH of 7.0, 50 mM KCl, 0.1 mM DTT, 5 mM $MgCl_2$ and 0.4% Triton X-100. Care should be taken that pH, salt concentration, detergent concentration etc. of the buffer system do not interfere with FRET.

In certain embodiments, the assay is conducted for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day.

In one embodiment, the invention provides a method of identifying an antiproliferative compound that inhibits or reduces animalia tRNA splicing endonuclease activity, said method comprising: (a) contacting a purified animalia tRNA splicing endonuclease with a substrate of a tRNA splicing endonuclease and a member of a library of compounds, wherein the substrate is labeled at the 5' end with a fluorophore and at the 3' end with a quencher, or, alternatively, the substrate is labeled at the 5' end with a quencher and labeled at the 3' end with a fluorphore; and (b) measuring the activity of the tRNA splicing endonuclease, wherein an antiproliferative compound that inhibits or reduces tRNA splicing endonuclease activity is identified if a fluorescent signal is not detectable or reduced in the presence of the compound relative to the absence of the compound or the presence of a negative control. In another embodiment, the invention provides a method of identifying an antiproliferative compound that inhibits animalia tRNA splicing endonuclease activity, said method comprising: (a) contacting a purified animalia tRNA splicing endonuclease with a substrate of a tRNA splicing endonuclease and a member of a library of compounds, wherein said substrate is labeled at the 5' end with a fluorescent donor moiety and labeled at the 3' end with a fluorescent acceptor moiety, or, alternatively, the substrate is labeled at the 5' end with a fluorescent acceptor moiety and labeled at the 3' end with a fluorescent donor moiety; and (b) measuring the activity of the tRNA splicing endonuclease, wherein an antiproliferative compound that inhibits or reduces tRNA splicing endonuclease activity is identified if the fluorescence emission of the fluorescent acceptor moiety at the wavelength of the fluorescent donor moiety in the presence of the compound is increased relative to the absence of the compound or the presence of a negative control.

In a specific embodiment, the invention provides a method of identifying an antifungal compound that inhibits or reduces fungal tRNA splicing endonuclease activity, said method comprising: (a) contacting a purified fungal tRNA splicing endonuclease with a substrate of a fungal tRNA splicing endonuclease and a member of a library of compounds, wherein the substrate is labeled at the 5' end with a fluorophore and at the 3' end with a quencher, or, alternatively, the substrate is labeled at the 5' end with a quencher and at the 3' end with a fluorophore; and (b) measuring the activity of the tRNA splicing endonuclease, wherein an anti-fungal compound that inhibits or reduces tRNA splicing endonuclease activity is identified if a fluorescent signal is not detectable or reduced in the presence of the compound relative to the absence of the compound or the presence of a negative control. In another embodiment, the invention provides a method of identifying an anti-fungal compound that inhibits fungal tRNA splicing endonuclease activity, said method comprising: (a) contacting a purified fungal tRNA splicing endonuclease with a substrate of a fungal tRNA splicing endonuclease and a member of a library of compounds, wherein the substrate is labeled at the 5' end with a fluorescent donor moiety and at the 3' end with a fluorescent acceptor moiety, or, alternatively, the substrate is labeled at the 5' end with a fluorescent acceptor moiety and at the 3' end with a fluorescent donor moiety; and (b) measuring the activity of the tRNA splicing endonuclease, wherein an anti-fungal compound that inhibits or reduces tRNA splicing endonuclease activity is identified if the fluorescence emission of the fluorescent acceptor moiety at the wavelength of the fluorescent donor moiety in the presence of the compound is increased relative to the absence of the compound or the presence of a negative control. In accordance with these embodiments, the anti-fungal compound identified may be further tested to determine or confirm that it has few, if any, negative effects on animalia cells.

In accordance with the invention, the substrate for tRNA splicing endonuclease may be labeled using a single pair of fluorescent donor and acceptor compounds. The substrate can be labeled with different pairs of fluorescent donor moieties and fluorescent acceptor moieties. For example, two, three, four, five or more pairs of fluorescent donor moieties and fluorescent acceptor moieties can be used. In this situation, preferably, at least one of the pairs comprise a fluorescent acceptor moiety that has a different emission spectrum from the fluorescent acceptor moiety of at least one of the other pairs. Alternatively, when at least three pairs are used, the fluorescent acceptor moiety of the first pair, second pair and third pair has a different emission spectrum than the fluorescent acceptor moiety of the other two. Methods for labeling the substrate with a fluorescent acceptor moiety, a fluorescent donor moiety and/or quencher are well-known in the art (*see, e.g.,* U.S. Patent Nos. 6,472,156, 6,451,543, 6,348,322, 6,342,379, 6,323,039, 6,297,018, 6,291,201, 6,280,981, 5,843,658, and 5,439,797, the disclosures of which are incorporated by reference in their entirety).

The activity of a compound on an animalia or fungal tRNA splicing endonuclease in the FRET cell-free assays can be determined by measuring the fluorescent emission spectra of the substrate utilizing techniques well-known to one of skill in the art. The fluorescent emission spectra measured depends, in part, on the fluorophore used.

### 5.5.1.2 CELL-FREE ASSAYS WITH LABELED tRNA SPLICING ENDONUCLEASE

A FRET cell-free assay may be conducted by contacting a first subunit of an animalia or fungal tRNA splicing endonuclease (*e.g.*, SEN2) labeled with a fluorophore and a second subunit of an animalia or fungal tRNA splicing endonuclease (*e.g.*, SEN34) labeled with a quencher with a compound *in vitro* under conditions conducive to the formation of the endonuclease, and measuring the fluorescence of the animalia or fungal tRNA splicing endonuclease by, *e.g.*, a fluorescence emission detector such as a Viewlux or Analyst. The formation of the animalia or fungal tRNA splicing endonuclease from the labeled subunits will result in a reduction in the detectable fluorescent signal. A compound that inhibits or reduces the formation of the animalia or fungal tRNA splicing endonuclease will enhance the production of detectable fluorescent signal relative to a negative control (*e.g.*, PBS). A compound that enhances the formation of the animalia or fungal tRNA splicing endonuclease will reduce or inhibit the fluorescence detectable relative to a negative control (*e.g.*, PBS).

Alternatively, a FRET cell-free assay may be conducted by contacting a first subunit of an animalia or fungal tRNA splicing endonuclease (*e.g.*, SEN2) labeled with a fluorescent donor moiety and a second, different subunit of an animalia or fungal tRNA splicing endonuclease (*e.g.*, SEN34) labeled with a fluorescent acceptor moiety with a compound *in vitro* under conditions conducive to the formation of the endonuclease, and measuring the fluorescence of the animalia or fungal tRNA splicing endonuclease by, *e.g.*, a fluorescence emission detector such as a Viewlux or Analyst. The formation of the animalia or fungal tRNA splicing endonuclease will result in the production of a detectable fluorescent signal by the fluorescent donor moiety and fluorescent acceptor moiety at the wavelength of the fluorescent donor moiety. A compound that inhibits or reduces the formation of the animalia or fungal tRNA splicing endonuclease will reduce the fluorescence emission of the fluorescent acceptor moiety at the wavelength of the fluorescent donor moiety relative to a negative control (*e.g.*, PBS). A compound that enhances the formation of the animalia or fungal tRNA splicing endonuclease will increase the fluorescence emission of the fluorescent acceptor at the wavelength of the fluorescent donor relative to a negative control (*e.g.*, PBS). In a preferred embodiment, a negative control (*e.g.*, PBS or another agent that is known to have no effect on the cleavage of the substrate) and a positive control (*e.g.*, an agent that is known to have an effect on the cleavage of the substrate) are included in the FRET cell-free assays described herein.

In one embodiment, the invention provides a method of identifying an antiproliferative compound that inhibits or reduces animalia tRNA splicing endonuclease activity, said method comprising: (a) contacting a first subunit of an animalia tRNA splicing endonuclease labeled with a fluorophore and a second, different subunit of an animalia tRNA splicing endonuclease labeled with a quencher with a member of a library of compounds under conditions conducive to the formation of the endonuclease; and (b) measuring the activity of the tRNA splicing endonuclease, wherein an antiproliferative compound that inhibits or reduces the tRNA splicing endonuclease activity is identified if a fluorescent signal is not detectable or reduced in the presence of the compound relative to the absence of the compound or the presence of a negative control. In another embodiment, the invention provides a method of identifying an antiproliferative compound that inhibits or reduces animalia tRNA splicing endonuclease activity, said method comprising: (a) contacting a first subunit of an animalia tRNA splicing endonuclease labeled with a fluorescent donor moiety and a second, different subunit of an animalia tRNA splicing endonuclease labeled with a fluorescent acceptor moiety with a member of a library of compounds under conditions conducive to the formation of the endonuclease; and (b) measuring the activity of the tRNA splicing endonuclease, wherein an antiproliferative compound that inhibits or reduces the tRNA splicing endonuclease activity is identified if the fluorescence emission of the fluorescent acceptor moiety at the wavelength of the fluorescent donor moiety in the presence of the compound is increased relative to the absence of the compound or the presence of a negative control.

In a specific embodiment, the invention provides a method of identifying an antifungal compound that inhibits or reduces fungal tRNA splicing endonuclease activity, said method comprising: (a) contacting a first subunit of a fungal tRNA splicing endonuclease labeled with a fluorophore and a second, different subunit of a fungal tRNA splicing endonuclease labeled with a quencher with a member of a library of compounds under conditions conducive to the formation of the endonuclease; and (b) measuring the activity of the tRNA splicing endonuclease, wherein an anti-fungal compound that inhibits or reduces the tRNA splicing endonuclease activity is identified if a fluorescent signal is not detectable or reduced in the presence of the compound relative to the absence of the compound or the presence of a negative control. In another embodiment, the invention provides a method of identifying an anti-fungal compound that inhibits or reduces fungal tRNA splicing endonuclease activity, said method comprising: (a) contacting a first subunit of a fungal tRNA splicing endonuclease labeled with a fluorescent donor moiety and a second, different subunit of a fungal tRNA splicing endonuclease labeled with a fluorescent acceptor moiety with a member of a library of compounds under conditions conducive to the formation of the endonuclease; and (b) measuring the activity of the tRNA splicing endonuclease, wherein an anti-fungal compound that inhibits or reduces the tRNA splicing endonuclease activity is identified if the fluorescence emission of the fluorescent acceptor moiety at the wavelength of the fluorescent donor moiety in the presence of the compound is increased relative to the absence of the compound or the presence of a negative control. In accordance with these embodiments, the anti-fungal compound identified may be further tested to determine or confirm that it has few, if any, negative effects on animalia cells.

### 5.5.1.3 CELL-BASED ASSAYS WITH <u>LABELED tRNA SPLICING ENDONUCLEASE</u>

A FRET cell-based assay may be conducted by microinjecting or transfecting a first subunit of an animalia or fungal tRNA splicing endonuclease (*e.g.*, SEN2) labeled with a fluorophore and a second, different subunit of an animalia or fungal tRNA splicing endonuclease (*e.g.*, SEN34) labeled with a quencher into a cell and contacting the cell with a compound, and measuring the fluorescence of the animalia or fungal tRNA splicing endonuclease by, *e.g.*, fluorescence microscopy or a fluorescence emission detector such as a Viewlux or Analyst. Preferably, the cell microinjected or transfected is deficient in one or more of the subunits of the animalia or fungal tRNA splicing endonuclease. The formation of the animalia or fungal tRNA splicing endonuclease from the labeled subunits will result in a reduction in the fluorescence detectable. A compound that inhibits or reduces the formation of the animalia or fungal tRNA splicing endonuclease will enhance the production of detectable fluorescent signal relative to a negative control (*e.g.*, PBS). A compound that enhances the formation of the animalia or fungal tRNA splicing endonuclease will reduce or inhibit the fluorescence detectable relative to a negative control (*e.g.*, PBS).

Alternatively, a FRET cell-based assay may be conducted by microinjecting a first subunit of an animalia or fungal tRNA splicing endonuclease (*e.g.*, SEN2) labeled with a fluorescent donor moiety and a second, different subunit of an animalia or fungal tRNA splicing endonuclease (*e.g.*, SEN34) labeled with a fluorescent acceptor moiety into a cell and contacting the cell with a compound, and measuring the fluorescence of the animalia or fungal tRNA splicing endonuclease by, *e.g.*, fluorescence microscopy or a fluorescence emission detector such as a Viewlux or Analyst. The formation of the animalia or fungal tRNA splicing endonuclease will result in the production of a detectable fluorescent signal by the fluorescent donor moiety and fluorescent acceptor moiety at the wavelength of the fluorescent donor moiety. A compound that inhibits or reduces the formation of the animalia or fungal tRNA splicing endonuclease will reduce the fluorescence emission of the fluorescent acceptor at the wavelength of the fluorescent donor moiety relative to a negative control (*e.g.*, PBS). A compound that enhances the formation of the animalia or fungal tRNA splicing endonuclease will increase the fluorescence emission of the fluorescent acceptor moiety at the wavelength of the fluorescent donor moiety relative to a negative control (*e.g.*, PBS). In a preferred embodiment, a negative control (*e.g.*, PBS or another agent that is known to have no effect on the cleavage of the substrate) and a positive control (*e.g.*, an agent that is known to have an effect on the cleavage of the substrate) are included in the FRET cell-based assays described herein.

In one embodiment, the invention provides a method of identifying an antiproliferative compound that inhibits or reduces animalia tRNA splicing endonuclease activity, said method comprising: (a) contacting a cell microinjected or transfected with a first subunit of an animalia tRNA splicing endonuclease labeled with a fluorophore and a second, different subunit of an animalia tRNA splicing endonuclease labeled with a quencher with a member of a library of compounds; and (b) measuring the fluoroscence of the tRNA splicing endonuclease, wherein an antiproliferative compound that inhibits or reduces the tRNA splicing endonuclease activity is identified if the fluorescent signal detectable in the presence of the compound is increased relative to the absence of the compound or the presence of a negative control. In another embodiment, the invention provides a method of identifying an antiproliferative compound that inhibits or reduces animalia tRNA splicing endonuclease activity, said method comprising: (a) contacting a cell microinjected or transfected with a first subunit of an animalia tRNA splicing endonuclease labeled with a fluorescent donor moiety and a second, different subunit of an animalia tRNA splicing endonuclease labeled with a fluorescent acceptor moiety with a member of a library of compounds; and (b) measuring the fluorescence of the tRNA splicing endonuclease, wherein an antiproliferative compound that inhibits or reduces the tRNA splicing endonuclease activity is identified if the fluorescence emission of the fluorescent acceptor moiety at the wavelength of the fluorescent donor moiety in the presence of the compound is decreased relative to the absence of the compound or the presence of a negative control.

In a specific embodiment, the invention provides a method of identifying an antifungal compound that inhibits or reduces fungal tRNA splicing endonuclease activity, said method comprising: (a) contacting a cell microinjected or transfected with a first subunit of a fungal tRNA splicing endonuclease labeled with a fluorophore and a second, different subunit of a fungal tRNA splicing endonuclease labeled with a quencher with a member of a library of compounds; and (b) measuring the fluorescence of the tRNA splicing endonuclease, wherein an anti-fungal compound that inhibits or reduces the tRNA splicing endonuclease activity is identified if the fluorescent signal detectable in the presence of the compound is increased relative to the absence of the compound or the presence of a negative control. In another embodiment, the invention provides a method of identifying an anti-fungal compound that inhibits or reduces fungal tRNA splicing endonuclease activity, said method comprising: (a) contacting a first subunit of a fungal tRNA splicing endonuclease labeled with a fluorescent donor moiety and a second, different subunit of a fungal tRNA splicing endonuclease labeled with a fluorescent acceptor moiety with a member of a library of compounds; and (b) measuring the activity of the tRNA splicing endonuclease, wherein an anti-fungal compound that inhibits or reduces the tRNA splicing endonuclease activity is identified if the fluorescence emission of the fluorescent acceptor moiety at the wavelength of the fluorescent in the presence of the compound is decreased relative to the absence of the compound or the presence of a negative control. In accordance with these embodiments, the anti-fungal compound identified may be further tested to determine or confirm that it has few, if any, negative effects on animalia cells.

In certain embodiments, the compound and the cell are incubated for at least 0.2 hours, 0.25 hours, 0.5 hours, 1 hour, 2 hours, 3 hours, 4 hours, 5 hours, 6 hours, 8 hours, 10 hours, 12 hours, 18 hours, or at least 1 day.

Methods for labeling a subunit of an animalia tRNA splicing endonuclease with a fluorescent acceptor moiety, a fluorescent donor moiety and/or quencher are well-known in the art (*see, e.g.,* U.S. Patent Nos. 6,472,156, 6,4.51,543, 6,34.8,322, 6,342,379, 6,323,039, 6,297,018, 6,291,201, 6,280,981, 5,843,658, and 5,439,797, the disclosures of which are incorporated by reference in their entirety).

### 5.5.2 <u>BINDING ASSAYS</u>

In specific embodiments, compounds that modulate the activity of an animalia or fungal tRNA splicing endonuclease can be identified by direct binding assays. In particular, compounds that inhibit the activity of an animalia or fungal tRNA splicing endonuclease by directly or indirectly reducing or inhibiting the interaction between a substrate for an animalia or fungal tRNA splicing endonuclease and an animalia or fungal tRNA splicing endonuclease can be identified using direct binding assays. Such assays are described in International Patent Publication Nos. WO 02/083837 and WO 02/083953, the disclosures of which are hereby incorporated by reference in their entireties. Briefly, direct binding assays may be conducted by attaching a library of compounds to solid supports, *e.g.*, polymer beads, with each solid support having substantially one type of compound attached to its surface. The plurality of solid supports of the library is exposed in aqueous solution to a substrate for an animalia or fungal tRNA splicing endonuclease having a detectable label, forming a dye-labeled substrate:support-attached compound complex. Binding of a substrate to a particular compound labels the solid support, *e.g.*, bead, comprising the compound, which can be physically separated from other, unlabeled solid supports. Once labeled solid supports are identified, the chemical structures of the compounds thereon can be determined by, *e.g.*, reading a code on the solid support that correlates with the structure of the attached compound.

Alternatively, binding assays may be conducted by contacting a substrate for an animalia or fungal tRNA splicing endonuclease having a detectable label with a member of a library of compounds free in solution, in labeled tubes or microtiter wells, or a microarray. Compounds in the library that bind to the labeled substrate of an animalia or fungal tRNA splicing endonuclease will form a detectably labeled complex that can be identified and removed from the uncomplexed, unlabeled compounds in the library, and from uncomplexed, labeled substrate of an animalia or fungal tRNA splicing endonuclease, by a variety of methods including, but not limited to, methods that differentiate changes in the electrophoretic, chromatographic, or thermostable properties of the complexed substrate.

In a specific embodiment, compounds that bind to an animalia or fungal tRNA splicing endonuclease are identified. In accordance with this embodiment, any assay well-known to one of skill in the art for the detection of an interaction between a compound with a protein, polypeptide or peptide can be to identify compounds that bind to an animalia or fungal tRNA splicing endonuclease. For example, the assay can be conducted by attaching a library of compounds to solid supports, *e.g.*, polymer beads, with each solid support having substantially one type of compound attached to its surface. The plurality of solid supports of the library is exposed in aqueous solution to an animalia or fungal tRNA splicing endonuclease having a detectable label, forming a dye-labeled endonuclease:support-attached compound complex. Binding of the endonuclease to a particular compound labels the solid support, *e.g.* , bead, comprising the compound, which can be physically separated from other, unlabeled solid supports. Once labeled solid supports are identified, the chemical structures of the compounds thereon can be determined by, *e.g.*, reading a code on the solid support that correlates with the structure of the attached compound.

Alternatively, the assays may be conducted by contacting an animalia or fungal tRNA splicing endonuclease with a member of a library of labeled compounds free in solution, in labeled tubes or microtiter wells, or a microarray. Compounds in the library that bind to the animalia or fungal tRNA splicing endonuclease will form a detectably labeled complex that can be identified and removed from the uncomplexed, labeled compounds in the library, and from uncomplexed, unlabeled animalia or fungal tRNA splicing endonuclease, by a variety of methods.

### 5.5.3 <u>FLUORESCENCE POLARIZATION ASSAYS</u>

The effect of a compound on the activity of an animalia or fungal tRNA splicing endonuclease may be determined utilizing a fluorescence polarization-based assay. In such an assay, a fluorescently labeled substrate for an animalia or fungal tRNA splicing endonuclease is contacted with an animalia cell-free extract (preferably, an animalia or fungal tRNA splicing endonuclease extract) or a purified animalia or fungal tRNA splicing endonuclease and a compound or a member of a library of compounds; and the fluorescently polarized light emitted is measured. An important aspect of this assay is that the size of the substrate used in the assay is large enough to distinguish a change in fluorescent polarized light emitted following cleavage of the substrate. The or fungal tRNA splicing endonuclease in the cell-free extract or the purified animalia or fungal tRNA splicing endonuclease will cleave the substrate and result in a change in intensity of emitted polarized light. Fluorescently labeled substrates when excited with plane polarized light will emit light in a fixed plane only if they do not rotate during the period between excitation and emission. The extent of depolarization of the emitted light depends upon the amount of rotation of the substrate, which is dependent on the size of the substrate. Small substrates rotate more than larger substrates between the time they are excited and the time they emit fluorescent light. A small fluorescently labeled substrate rotates rapidly and the emitted light is depolarized. A large fluorescently labeled substrate rotates more slowly and results in the emitted light remaining polarized. A compound that inhibits the activity of the tRNA splicing endonuclease will inhibit or reduce the cleavage of the substrate and thus, decrease the rotation of the substrate relative to a negative control (*e.g.*, PBS) or the absence of the compound, which will result in the emitted light remaining polarized. A compound that enhances the activity of the tRNA splicing endonuclease will enhance the cleavage of the substrate and thus, increase the rotation of the substrate relative to a negative control (*e.g.*, PBS) or the absence of the compound, which will result in more of the emitted light being depolarized.

The intensities of the light are measured in planes 90° apart and are many times designated the horizontal and vertical intensities. In some instruments the excitation filter is moveable while the emission filter is fixed. In certain other machines the horizontal and vertical intensities are measured simultaneously via fiber optics. Research grade fluorescence polarization instruments are commercially available from, *e.g.*, Pan Vera, BMG Lab Technologies, and LJL Biosystems. Abott provides clinical laboratory instrumentation. The value of fluorescence polarization is determined by the following equation:

$$\text{polarization} = \frac{\text{intensity}_{\text{vertical}} - \text{intensity}_{\text{horizontal}}}{\text{intensity}_{\text{veritcal}} + \text{intensity}_{\text{horizontal}}}.$$

Fluorescence polarization values are most often divided by 1000 and expressed as millipolarization units (mP).

In a specific embodiment, the hybridized tRNA substrate or circularly permitted tRNA substrate depicted in Figures 1B and 1C, respectively, are used as a substrate in the fluorescence polarization assay and the 5' end, the 3' end or both the 5' and 3' ends of the circularly permited tRNA substraess are labeled with a flurophene.

### 5.5.4 <u>FISH Assays</u>

The activity of an animalia or fungal tRNA splicing endonuclease may be determined in an assay in which the persistence and quantity of tRNA intron is detected in an animalia cell or fungal cell. The amount of tRNA intron is quantified at different time points after or during the incubation of the cell with the compound. The tRNA intron can be detected by means of fluorescence *in situ* hybridization ("FISH") using a tRNA intronspecific probe. In certain embodiments, a control experiment is conducted in parallel wherein the animalia cell or fungal cell is not contacted with a compound.

In the absence of an inhibitor of an animalia or fungal tRNA splicing endonuclease, the splicing reaction is fast and the concentration of intron in the cell is low. Without being bound by theory, because the spliced intron is normally degraded the concentration of tRNA intron in the animalia cell or fungal cell is below the detection threshold. In the presence of an inhibitor of an animalia or fungal tRNA splicing endonuclease, the splicing reaction is slowed down and the amount of tRNA intron increases. Thus, a compound that inhibits animalia or fungal tRNA splicing endonuclease can be identified by its ability to increase the level of tRNA intron in the animalia cell or fungal cell.

Methods for conducting FISH are well-known to the skilled artisan and can be used with the invention. Exemplary methods for FISH are described in Sarkar and Hopper, 1998 (Mol. Biol. Cell 9:3041-3055), which is incorporated herein in its entirety.

In certain embodiments, a FISH assay is used to determine the effect of a compound on the activity of an animalia or fungal tRNA splicing endonuclease in a high-throughput screen. In particular a 96-lens microscope can be used for a high-throughput screen based on FISH. In a specific embodiment, 96 cell cultures are incubated in a 96-well plate with different compounds. Subsequently, the cells are subjected to a FISH analysis using a tRNA intron specific probe and analyzed using the 96-lens microscope. The presence of a signal or the presence of a significantly stronger signal demonstrates that tRNA intron was present in the cells at elevated levels and thus, the compound is a candidate inhibitor of tRNA splicing endonuclease.

Without being bound by theory, the FISH assay identifies the compound as inhibitor of the tRNA splicing endonuclease directly. Thus, in certain embodiments, a compound that was identified in a FISH assay as an inhibitor of tRNA splicing is a *prima facie* candidate for an inhibitor of tRNA splicing endonuclease.

### 5.5.5 OTHER SCREENING ASSAYS

The activity of an animalia or fungal tRNA splicing endonuclease may be determined in an assay in which the amount of substrate for a tRNA splicing endonuclease cleaved by the endonuclease in the presence of a compound relative to a control (preferably, a negative control and more preferably, a negative control and a positive control) is detected. Such an assay may be conducted by contacting or incubating a compound with a labeled substrate for an animalia or fungal tRNA splicing endonuclease and a cell-free extract or purified animalia or fungal tRNA splicing endonuclease under conditions conducive for tRNA splicing endonuclease activity, and measuring the amount of cleaved substrate. The substrate for the animalia or fungal tRNA splicing endonuclease can be labeled with any detectable agent (including, but not limited to, spectroscopic labels such as fluorescent dyes (*e.g.*, fluorescein and derivatives such as fluorescein isothiocyanate (FITC) and Oregon Green™, rhodamine and derivatives (*e.g.*, Texas red, tetramethylrhodimine isothiocynate (TRITC), bora-3a,4a-diaza-s-indacene (BODIPY®) and derivatives, *etc.*), digoxigenin, biotin, phycoerythrin, AMCA, CyDye™, and the like), radiolabels (*e.g.*, $^3$H, $^{125}$I, $^{35}$S, $^{14}$C, $^{32}$P, $^{33}$P, *etc.*), enzymes (*e.g.*, horse radish peroxidase, alkaline phosphatase etc.), spectroscopic colorimetric labels such as colloidal gold or colored glass or plastic (*e.g.*, polystyrene, polypropylene, latex, etc.) beads, or nanoparticles - nanoclusters of inorganic ions with defined dimension from 0.1 to 1000 nm) utilizing techniques known to one of skill in the art. In certain embodiments, a compound is contacted or incubated with a labeled substrate for an animalia or fungal tRNA splicing endonuclease and a cell-free extract or purified animalia or fungal tRNA splicing endonuclease for at least 5 minutes, at least 10 minutes, at least 15 minutes, at least 30 minutes, at least 1 hour, at least 2 hours, or more. The amount of cleaved substrate is proportional to the activity of the tRNA splicing endonuclease. The amount of cleaved tRNA splicing endonuclease can be measured by any technique known to one skilled in the art.

In certain embodiments, the cleaved tRNA splicing endonuclease substrate is separated from the uncleaved tRNA splicing endonuclease substrate by gel-electrophoresis. The amount of cleaved tRNA splicing endonuclease substrate can be quantified by measuring the intensity of the signal of the cleaved tRNA splicing endonuclease substrate. The stronger the signal produced by the cleaved tRNA splicing endonuclease substrate relative to the uncleaved tRNA splicing endonuclease substrate the more active is the tRNA splicing endonuclease. The signal intensity can be quantified using autoradiography or a phosphoimager. If the activity of the tRNA splicing endonuclease is decreased in the presence of a compound, i.e., if the signal of the cleaved tRNA splicing endonuclease substrate relative to the uncleaved tRNA splicing endonuclease substrate is decreased compared to the reaction without the compound or in the presence of a negative control, the compound is identified as an inhibitor of the tRNA splicing endonuclease.

In other embodiments, the amount of cleaved tRNA is determined using mass spectrometry.

### 5.6 SCREENING ASSAYS TO IDENTIFY COMPOUNDS THAT MODULATE TRNA SPLICING LIGASE ACTIVITY

The invention encompasses various *in vitro* assays to identify and/or verify the ability of a compound to modulate the activity of an animalia and/or fungal tRNA splicing ligase. Multiple *in vitro* assays can be performed simultaneously or sequentially to assess the effect of a compound on the activity of an animalia and/or fungal tRNA splicing ligase. One or more of the activities associated with an animalia and/or fungal tRNA splicing ligase can be measured utilizing the techniques described herein or well known to one skilled in the art to determine the effect of a compound on an animaila and/or fungal tRNA splicing ligase. Examples of activities associated with animalia and/or fungal tRNA splicing ligase include, but are not limited to, kinase activity, cyclic phosphodiesterase activity, adenylate synthetase activity, and 2' phosphotransferase activity. *See, e.g.,* Abelson et al., 1998, Journal of Biol. Chem., 273(21): 2685-88; Belfort et al., 1997, Cell, 89: 1003-6; Xu et al., 1990, Methods in Enzymology, 181: 463-471; Phizicky et al., 1986, Journal of Biol. Chem. 261(6): 2978-2986; Belford et al., 1993, Journal of Biological Chem., 268(4): 2444-2450; Gomes et al., 1997, Biochemical and Biophysical Res. Comm. 237: 588-94; Greer et al., 1982, Cell, 32: 537-46. An activity specific for fungal tRNA splicing ligase can be measured to identify compounds that specifically or differentially affect the activity of a fungal tRNA splicing ligase. Alternatively, an activity specific for an animalia tRNA splicing ligase can be measured to identify compounds that specifically or differentially affect the activity of an animalia or fungal tRNA splicing ligase. In a preferred embodiment, the *in vitro* assays described herein are performed in a high through put format.

The *in vitro* screening assays described herein can be conducted utilizing any animalia cell or fungal cell or any purified animalia or fungal tRNA splicing ligase. In a specific embodiment, the animalia tRNA splicing ligase utilized in the *in vitro* assays is a mammalian tRNA splicing ligase (*e.g.*, non-human mammalian tRNA splicing ligase and human tRNA splicing ligase). In a preferred embodiment, the animalia tRNA splicing ligase utilized in the *in vitro* assays is a human tRNA splicing ligase. In another embodiment, the fungal tRNA splicing ligase utilized in the *in vitro* assays is a yeast tRNA splicing ligase. The species of the tRNA splicing ligase utilized in an *in vitro* assay will vary depending on the purpose for conducting the assay.

The invention encompasses methods for identifying a compound that modulates (*e.g.*, reduces or enhances) an animalia or fungal tRNA splicing ligase activity, said methods comprising: (a) contacting a substrate of tRNA splicing ligase, *e.g.*, tRNA half molecules, with a compound or a member of a library of compounds and an animalia or fungal tRNA splicing ligase in an amount sufficient to generate a population of mature tRNA molecules; and (b) measuring the formation of mature tRNA molecules, wherein a compound that modulates the tRNA splicing ligase activity is identified if the amount of mature tRNA molecules generated in the presence of the compound is altered relative to a previously determined reference range, or the amount in the presence of a control (*e.g.*, phosphate buffered saline ("PBS")), or the absence of the compound. A compound that enhances the activity of an animalia or fungal tRNA splicing the ligase increases the amount of mature tRNA molecules generated. A compound that inhibits or reduces the activity of an animalia or fungal tRNA splicing ligase decreases the amount of mature tRNA molecules generated. The formation or generation of mature tRNA molecules can be measured utilizing techniques well-known to one of skill in the art, including, but not limited to, electrophoresis. In some embodiments, the tRNA ligase is from a crude extract. In other embodiments, the tRNA ligase is a recombinant tRNA ligase, purified to homogeneity by standard methods known to those skilled in the art or described herein (*See, e.g.,* Xu et al., 1990, Methods in Enzymology, 181: 463-71; Phyizicky et al., 1986, Journal of Biol. Chem., 261 (6): 2978-86).

The invention also encompasses methods for identifying a compound that modulates (*e.g.*, reduces or enhances) an animalia or fungal tRNA splicing ligase activity, said methods comprising: (a) contacting a substrate of an animalia or fungal tRNA splicing endonuclease with an animalia or fungal tRNA splicing endonuclease in an amount sufficient to cleave the substrate and generate tRNA half molecules; (b) contacting the tRNA half molecules with a compound or a member of a library of compounds and an animalia or fungal tRNA splicing ligase in an amount sufficient to generate a population of mature tRNA molecules; and (c) measuring the formation of mature tRNA molecules, wherein a compound that modulates the tRNA splicing ligase activity is identified if said the amount of mature tRNA molecules generated in the presence of the compound is altered relative to a previously determined reference range, or the amount in the presence of a control (*e.g.*, phosphate buffered saline ("PBS")), or the absence of the compound.

**[PLEASE PROVIDE SPECIFIC PROTOCOLS FOR PREFERRED LIGASE ASSAYS]**

The invention encompasses methods for identifying a compound that modulates the kinase activity of an animalia or fungal tRNA splicing ligase utilizing a polynucleotide kinase assay well-known to known to one of the skill in the art. Although not intending to be bound by any mechanism of action, this assay is based on the ability of an animalia or fungal tRNA splicing ligase to transfer the g-phosphate of ATP to a variety of polynucleotides. One exemplary assay for measuring a polynucleotide kinase activity may comprise the following: providing a poly(A) nucleotide of, for example, 600 nucleotides in length; providing $[\gamma-^{32}P]ATP$; providing a sufficient amount of an animalia or fungal tRNA splicing ligase in an appropriate buffer including, for example, a buffer containing a DTT and $MgCl_2$, which is either pre-incubated with a library of the compounds or not; allowing the reactants to incubate for 15 minutes at 37°C; phenol chloroform extracting the reaction; adding bovine serum albumin and cold trichloroacetic acid to the aqueous phase; collecting the insoluble material by centrifugation; adding cold trichloroacetic acid and sodium pyrophosphate; collecting the acid insoluble material on glass fiber filter; measuring the radioactivity on the glass filter, wherein the amount of measured radioactivity is proportional to the kinase activity of the tRNA splicing ligase. If the compound inhibits or reduces the kinase activity of the tRNA splicing ligase, the amount of radioactivity measured is decreased relative to the amount of radioactivity measured in the absence of the compound or the presence of a negative control (*e.g.*, PBS). On the other hand, if the compound increase the kinase activity of the tRNA splicing ligase, the amount of radioactivity measured is decreased relative to the amount of radioactivity measured in the absence of the compound or the presence of a negative control (*e.g.*, PBS). Polynucleotide kinases assays are well-known in the art. *See e.g.,* Xu et al., 1990, Methods in Enzymology, 181: 463-471; Phizicky et al., 1986, Journal of Biol. Chem. 261(6): 2978-2986; Pick et al., 1986, J. Biol. Chem., 261: 6684. **[PLEASE CONFIRM AND PROVIDE PREFERRED KINASE ASSAY]**

The invention encompasses methods for identifying a compound that modulates the cyclic phosphodiesterase activity of an animalia or fungal tRNA splicing ligase. Any of the assays known to those skilled in the art for measuring cyclic phospodiesterase activity are within the scope of the present invention, see, *e.g.*, Phizicky et al., 1986, Journal of Biol. Chem. 261(6): 2978-2986; Greer et al., 1983, Cell, 537, both of which are incorporated herein by reference in their entireties. One exemplary assay for measuring a cyclic phospodiesterase activity is based on an earlier protocol described by Xu et al. (1990, Methods in Enzymology, 181: 463-471) and comprises the following: incubating a substrate for the assay, *e.g.*, UnG>P labeled with $^{32}$P in the 3'-terminal cyclic phosphate, with an animalia or fungal tRNA ligase; at 30°C for 20 minutes; stopping the reaction by boiling and adding ribonuclease A; analyzing the reaction mixture on cellulose plates. The amounts of cGMP and 2'-GMP are quantitated by cutting the spots from the thin-layer plate and counting in a scintillation counter. This assay may be conducted in the presence and absence of the compounds in order to assess the effect of the compounds on the cyclic phosphodiesterase activity of the tRNA splicing ligase. In addition, the covalent addition of AMP to a tRNA splicing ligase can be demonstrated by incubating the protein with [a$^{32}$P-ATP] in a reaction mixture containing 100mM HEPES(pH 7.5); 10 mM MgCl$_2$, 3mM DTT, and tRNA ligase. After incubation for 10 minutes at 30°C, an equal volume of 125 mM Tris (pH 6.8) 1.5% SDS, 135 mM b-mercaptoethanol; 20% glycerol and 0.1% bromopheonl blue is added and the mixture is boiled and analyzed to identify the presence of the adenylated protein based on the mobility on an SDS-gel.

### 5.6.1 FRET-BASED ASSAYS

The invention encompasses "FRET" based assays to detect alterations in the activity of an animalia and/or fungal tRNA splicing ligase. The invention encompasses FRET based assays wherein the tRNA splicing ligase or a substrate of the tRNA splicing ligase is labeled with a fluorophore.

### 5.6.1.1 FRET-LABELED SUBSTRATE

The invention encompasses a FRET cell-free assay for identifying a compound that modulates the activity of an animalia or fungal tRNA splicing ligase, comprising: contacting a substrate for an animalia or fungal tRNA splicing ligase comprising a population of 5' tRNA half molecules and a population of 3' tRNA half molecules, with a cell-free extract (preferably, an animalia or fungal tRNA splicing ligase extract), or a purified animalia or fungal tRNA splicing ligase and a compound or a member of a library of compounds, wherein the termini of each population of tRNA half molecule is labeled such that one population is labeled with fluorophor and the other population is labeled with a quencher; and measuring the fluorescence of the substrate. The tRNA splicing ligase will join the tRNA half molecules and result in the loss of the fluorescent signal. A compound that inhibits or reduces the activity of the tRNA splicing ligase will inhibit or reduce the ligation of the tRNA half molecules and thus, maintain or enhance the detectable fluorescent signal relative to the signal in ansence of the compopund or the presence of a negative control (*e.g.*, PBS). A compound that enhances the activity of the tRNA splicing ligase will enhance the ligation of the tRNA half molecules and thus, decrease the detectable fluorescent signal relative to the signal in absence of the compound or the presence of a negative control (*e.g.* PBS).

A substrate of an animalia tRNA splicing ligase can be labeled by any method known to the skilled artisan. In certain embodiments, a substrate of an animalia tRNA splicing ligase can be labeled using site-specific labeling of RNA with fluorophores. In more specific embodiments, a substrate of an animalia tRNA splicing ligase is labeled using the methods described in Qin and Pyle, 1999 (Methods 18(1):60-70), which is incorporated herein by reference in its entirety. The optimal protocol for labeling of a substrate of an animalia tRNA splicing ligase can be determined by the skilled artisan using routine experimentation. In a specific embodiment, the 5' termini of the 5' tRNA half molecule is labeled using RNA labeling schemes known to one skilled in the art. In another specific embodiment, the 3' termini of the 3' tRNA half molecule is labeled using RNA labeling schemes known to one skilled in the art. It will be appreciated by one skilled in the art that the site of labeling is chosen so that the site that is being ligated is not labeled. Additionally, the sensitivity of the detection of the label may be enhanced using common methods known to one skilled in the art depending on the label used, by routine experimentation.

In a specific embodiment, a substrate of an animalia tRNA splicing ligase is labeled using different methods, different labels and/or different positions in the tRNA substrate for labeling. The differently labeled substrates are then subjected separately to a splicing assay in the presence and absence, respectively of an inhibitor or an activator of an animalia tRNA splicing ligase. The optimal label for the screening assays is the label that is the most easily detectable and allows for the most reproducible detection of the effect of the inhibitor or the activator. Other labeling procedures, however, may also be used with the methods of the invention, especially if they provide other desirable advantages.

The invention also encompasses a FRET cell-free assay for identifying a compound that modulates the activity of an animalia or fungal tRNA splicing ligase, comprising: contacting a substrate for an animalia or fungal tRNA splicing ligase comprising a population of 5' tRNA half molecules and a population of 3' tRNA half molecules with a cell-free extract (preferably, a tRNA splicing ligase extract) or a purified animalia or fungal tRNA splicing ligase and a compound or a member of a library of compounds, wherein the terminus of one population is labeled with a fluorescent donor moiety and the terminus of the other population is labeled with a fluorescent acceptor moiety; and measuring the fluorescence of the substrate at the wavelength of the fluorescent donor moiety. The tRNA splicing ligase join the tRNA half molecules and result in the fluorescence of the fluorescent acceptor moiety at the wavelength of the fluorescent donor moiety. A compound that inhibits the activity of the tRNA splicing ligase will inhibit or reduce the ligation of the tRNA half molecules and thus, decrease the fluorescence emission of the fluorescent acceptor moiety at the wavelength of the fluorescent donor moiety relative to the fluorescence emission in the absence of the compound or the presence of a negative control (*e.g.*, PBS). A compound that enhances the activity of the tRNA splicing ligase will enhance the ligation of the tRNA half molecules and thus, maintain or increase the fluorescence emission of the fluorescent acceptor moiety at the wavelength of the fluorescent donor moiety relative to the fluorescence emission in the absence of the compound or the presence of a negative control (*e.g.*, PBS).

### 5.6.1.2 <u>FRET-LABELED ENZYME</u>

A FRET cell-free assay may be conducted by contacting a tRNA splicing ligase (*e.g.*, an animalia or fungal tRNA splicing ligase) labeled with a fluorophore and a tRNA splicing ligase substrate labeled with a quencher with a compound *in vitro* under conditions conducive to the formation of a complex beteween the tRNA splicing ligase and the substrate (since the ligase is believed to have a high affinity for the substrate; a stable complex will be formed), and measuring the fluorescence of the tRNA splicing ligase by, *e.g.*, a fluorescence emission detector such as a Viewlux or Analyst. The formation of a complex beteween the tRNA splicing ligase and the substrate will result in a reduction in the detectable fluorescence. A compound that inhibits or reduces the formation of the complex will enhance the production of detectable fluorescent signal relative to the absence of the compound or the presence of a negative control (*e.g.*, PBS). A compound that enhances the formation of the complex will reduce or inhibit the fluorescence detectable relative to the absence of the compound or a negative control (*e.g.*, PBS).

Alternatively, a FRET cell-free assay may be conducted by contacting a tRNA splicing ligase labeled with a fluorescent donor moiety and a substrate of tRNA splicing ligase labeled with a fluorescent acceptor moiety with a compound *in vitro* under conditions conducive to the formation of a complex beteween the tRNA splicing ligase and the substrate, and measuring the fluorescence of the tRNA splicing ligase by, *e.g.*, a fluorescence emission detector such as a Viewlux or Analyst. The formation of the complex will result in the production of a detectable fluorescent signal by the fluorescent donor moiety and fluorescent acceptor moiety at the wavelength of the fluorescent donor. A compound that inhibits or reduces the formation of the complex will reduce the fluorescence emission of the fluorescent acceptor moiety at the wavelength of the fluorescent donor moiety relative to the absence of the compound or the presence of a negative control (*e.g.*, PBS). A compound that enhances the formation of the complex will increase the fluorescence emission of the fluorescent acceptor moiety at the wavelength of the fluorescent donor moiety relative to the absence of the compound or the presence of a negative control (*e.g.*, PBS). In a preferred embodiment, a negative control (e.g., PBS or another agent that is known to have no effect on the cleavage of the substrate) and a positive control (*e.g.*, an agent that is known to have an effect on the cleavage of the substrate) are included in the FRET cell-free assays described herein.

### 5.6.2 <u>FLUORESCENCE POLARIZATION-BASED ASSAYS</u>

The effect of a compound on the activity of animalia and/or fungal tRNA splicing ligase may be determined using a fluorescence polarization based assay. In such an assay, a fluorescently labeled substrate for an animalia and/or fungal tRNA splicing ligase, *e.g.*, fluorescently labeled tRNA half molecules, is contacted with a purified animalia and/or fungal tRNA splicing ligase and a compound or a member of a library of compounds, and the fluoresenctly polarized light emitted is measured. An important aspect of this assay is that the size of the substrate used in this assay is large enough to distinguish a change in fluorescent polarized light emitted following ligation of the substrate. The tRNA splicing ligase will ligate the substrate and result in a change in the intensity of the emitted polarized light. A compound that inhibits the activity of a tRNA splicing ligase will inhibit or reduce the ligation of the tRNA half molecules and thus, increase the rotation of the substrate relative to a negative control (*e.g.*, PBS) of the absence of the compound, which will result in more of the light being depolarized. A compound that enhances the activity of a tRNA splicing ligase will enhance the ligation of the tRNA half molecules and thus, decrease the rotation of the substrate relative to a negative control or the absence of the compound, which will result in the emitted light remaining polarized.

### 5.6.3 <u>FISH Assays</u>

The activity of an animalia or fungal tRNA splicing ligase may be determined in an assay in which the quantity of a mature tRNA molecule generated from two tRNA half molecules is detected in an animalia cell or fungal cell. The amount of mature tRNA molecule may be quantified at different time points after or during the incubation of the cell witch the compound. The mature tRNA molecule can be detected by means of fluorescence *in situ* hybridization ("FISH") using a mature tRNA molecule specific probe. In certain embodiments, a control experiment is conducted in parallel wherein the animalia cell or fungal cell is not contacted with a compound or is contacted with a negative control (*e.g.*, PBS). In the absence of an inhibitor of an animalia or fungal tRNA splicing ligase, the ligase will join tRNA half molecules to form mature tRNA molecules. In the presence of an inhibitor of an animalia or fungal tRNA splicing endonuclease, the ligase is slowed down and the amount of mature tRNA molecule decreases. Thus, a compound that inhibits animalia or fungal tRNA splicing ligase can be identified by its ability to decrease the level of mature tRNA molecule in the animalia cell or fungal cell. Methods for conducting FISH are well-known to the skilled artisan and can be used with the invention. Exemplary methods for FISH are described in Sarkar and Hopper, 1998 (Mol. Biol. Cell 9:3041-3055), which is incorporated herein in its entirety.

In certain embodiments, a FISH assay is used to determine the effect of a compound on the activity of an animalia or fungal tRNA splicing ligase in a high-throughput screen. In particular a 96-lens microscope can be used for a high-throughput screen based on FISH. In a specific embodiment, 96 cell cultures are incubated in a 96-well plate with different compounds. Subsequently, the cells are subjected to a FISH analysis using a mature tRNA molecule specific probe and analyzed using the 96-lens microscope. A decrease in the signal detected demonstrates that mature tRNA molecule was present in the cells at decreased levels and thus, the compound is a candidate inhibitor of tRNA splicing ligase.

### 5.6.4 GEL-ELECTROPHORESIS BASED ASSAYS

The invention encompasses characterizing the compounds for their effect on tRNA splicing ligase activity using gel electrophoresis based assays. Gel electrophoresis based assays for measuring tRNA splicing ligase activity are well-known in the art, and any of the standard methods can be used in conjunction with the methods of the invention (*see e.g.,* Gomes et al., 1997 Biochemical and Biophysical Res. Comm., 237(3): 588-594, which is incorporated herein by reference in its entirety).

The invention provides a method for identifying a compound that modulates the activity of an animalia or fungal tRNA splicing ligase based on an assay described by Belford et al. (1993, Journal of Biological Chem., 268(4): 2444-2450), said method comprises: contacting a radiolabeled pre-tRNA substrate (*e.g.*, radiolabeled pre-tRNA Phe prepared using an *in vitro* transcription reaction known to those skilled in the art, *e.g.*, (Reyes et al., 1987, Anal. Biochem, 166: 90-106; all of which is incorporated herein by reference in its entirety) with a sufficient amount of a purified animalia or fungal tRNA splicing endonuclease and under conditions such that the pre-tRNA is cleaved; incubating the cleaved substrate with a purified animalia or fungal tRNA splicing ligase, which has been pre-incubated with a compound or a member of a library of the compounds, and analyzing the products of the ligase reaction by electrophoresis in *e.g.*, an 8M urea gel or 12% polyacrylamide gel and visualizing the gel by autoradiography. Alternatively, the invention provides a method for identifying a compound that modulates the activity of an animalia or fungal tRNA splicing ligase, said method comprising: contacting a radiolabeled pre-tRNA substrate with a sufficient amount of a purified animalia or fungal tRNA splicing endonuclease and under conditions that the pre-tRNA is cleaved; incubating, under appropriate conditions for ligation, the cleaved substrate with a purified animalia or fungal tRNA splicing ligase and a compound or a member of a library of compounds; and analyzing the products of the ligase reaction by electrophoreses, *e.g.*, an 8 m urea gel or 12% polyacrylamide gel and visualizing the gel by autoradiography. The products reaction can be quantitated by, *e.g.*, Cerenkov counts/minute of the gel slices. A compound that inhibits or reduces the activity of the tRNA splicing ligase will inhibit or reduce the ligation of the cleaved substrate and thus, decrease the amount of ligase reaction products (*i.e.*, mature tRNA molecule) detected relative to the amount of products detected in the absence of the compound or the presence of a negative control (*e.g.*, PBS). A compound that enhances the activity of the tRNA splicing ligase will enhance the ligation of the cleaved substrate and, thus, maintain or increase the amount of ligase reaction products detected relative to the amount of products detected in the absence of the compound or the presence of a negative control (*e.g.*, PBS).

The invention provides a method for identifying a compound that modulates the activity of an animalia or fungal tRNA splicing ligase, said method comprising: contacting a cell-free extract or a purified animalia or fungal tRNA splicing ligase with tRNA half molecules under conditions sufficient to promote the ligation of the half-molecules in the presence of a compound or a member of a library of compounds; and analyzing the products of the ligase reaction by, *e.g.*, electrophoresis and autoradiography if the half molecules are radiolabeled or by, *e.g.*, northern blot analysis using a probe specific for the ligated product if the half-molecules are unlabeled. A compound that inhibits or reduces the activity of the tRNA splicing ligase will inhibit or reduce the ligation of the tRNA half molecules and thus, decrease the amount of ligase reaction product detected relative to the amount of products detected in the absence of the compound or the presence of a negative control. A compound that enhances the activity of the tRNA splicing ligase will enhance the ligation of the tRNA half molecules and thus, maintain or increase the amount of ligase reaction products detected in the absence of the compound or the presence of a negative control (*e.g.*, PBS).

Gel electrophoresis based assays are used qualitatively and quantitatively to determine the amount of products formed from a tRNA splicing ligase reaction in the presence and absence of a compound or a member of a library of compounds in order to assess the effect of the compounds on a tRNA splicing ligase activity. The pre-tRNA molecule, the mature tRNA, the 5' and 3' tRNA half molecules have different electrophoretic mobilities and may be separated by electrophoresis; visualized and/or quantitated using standard methods known to those skilled in the art.

Methods for separation of products of a tRNA splicing ligase reaction from the reactants comprises any method of electrophoretic separation, including but not limited to, denaturing and non-denaturing polyacrylamide gel electrophoresis, urea gel electrophoresis, gel filtration, pulsed field gel electrophoresis, two dimensional gel electrophoresis, continuous flow electrophoresis, zone electrophoresis, agarose gel electrophoresis, and capillary electrophoresis. Other embodiments of electrophoretic separation include, but are not limited to urea gel electrophoresis, gel filtration, pulsed field gel electrophoresis, two dimensional gel electrophoresis, continuous flow electrophoresis, zone electrophoresis, and agarose gel electrophoresis.

In a preferred embodiment, an automated electrophoretic system comprising a capillary cartridge having a plurality of capillary tubes is used for high-throughput screening of compounds for their effect on an animalia and/or fungal tRNA splicing ligase activity. Such an apparatus for performing automated capillary gel electrophoresis is disclosed in U.S. Patent Nos. 5,885,430; 5,916,428; 6,027,627; and 6,063,251, the disclosures of which are incorporated by reference in their entireties.

The device disclosed in U.S. Patent No. 5,885,430, which is incorporated by reference in its entirety, allows one to simultaneously introduce samples into a plurality of capillary tubes directly from microtiter trays having a standard size. U.S. Patent No. 5,885,430 discloses a disposable capillary cartridge which can be cleaned between electrophoresis runs, the cartridge having a plurality of capillary tubes. A first end of each capillary tube is retained in a mounting plate, the first ends collectively forming an array in the mounting plate. The spacing between the first ends corresponds to the spacing between the centers of the wells of a microtiter tray having standard size. Thus, the first ends of the capillary tubes can simultaneously be dipped into the samples present in the tray's wells. The cartridge is provided with a second mounting plate in which the second ends of the capillary tubes are retained. The second ends of the capillary tubes are arranged in an array which corresponds to the wells in the microtiter tray, which allows for each capillary tube to be isolated from its neighbors and therefore free from cross-contamination, as each end is dipped into an individual well.

Plate holes may be provided in each mounting plate and the capillary tubes inserted through these plate holes. In such a case, the plate holes are sealed airtight so that the side of the mounting plate having the exposed capillary ends can be pressurized. Application of a positive pressure in the vicinity of the capillary openings in this mounting plate allows for the introduction of air and fluids during electrophoretic operations and also can be used to force out gel and other materials from the capillary tubes during reconditioning. The capillary tubes may be protected from damage using a needle comprising a cannula and/or plastic tubes, and the like when they are placed in these plate holes. When metallic cannula or the like are used, they can serve as electrical contacts for current flow during electrophoresis. In the presence of a second mounting plate, the second mounting plate is provided with plate holes through which the second ends of the capillary tubes project. In this instance, the second mounting plate serves as a pressure containment member of a pressure cell and the second ends of the capillary tubes communicate with an internal cavity of the pressure cell. The pressure cell is also formed with an inlet and an outlet. Gels, buffer solutions, cleaning agents, and the like may be introduced into the internal cavity through the inlet, and each of these can simultaneously enter the second ends of the capillaries.

In another preferred embodiment, the automated electrophoretic system can comprise a chip system consisting of complex designs of interconnected channels that perform and analyze enzyme reactions using part of a channel design as a tiny, continuously operating electrophoresis material, where reactions with one sample are going on in one area of the chip while electrophoretic separation of the products of another sample is taking place in a different part of the chip. Such a system is disclosed in U.S. Patent Nos. 5,699,157; 5,842,787; 5,869,004; 5,876,675; 5,942,443; 5,948,227; 6,042,709; 6,042,710; 6,046,056; 6,048,498; 6,086,740; 6,132,685; 6,150,119; 6,150,180; 6,153,073; 6,167,910; 6,171,850; and 6,186,660, the disclosures of which are incorporated by reference in their entireties.

The system disclosed in U.S. Patent No. 5,699,157, which is hereby incorporated by reference in its entirety, provides for a microfluidic system for high-speed electrophoretic analysis. of subj ect materials for applications in the fields of chemistry, biochemistry, biotechnology, molecular biology and numerous other areas. The system has a channel in a substrate, a light source and a photoreceptor. The channel holds subject materials in solution in an electric field so that the materials move through the channel and separate into bands according to species. The light source excites fluorescent light in the species bands and the photoreceptor is arranged to receive the fluorescent light from the bands. The system further has a means for masking the channel so that the photoreceptor can receive the fluorescent light only at periodically spaced regions along the channel. The system also has an unit connected to analyze the modulation frequencies of light intensity received by the photoreceptor so that velocities of the bands along the channel are determined, which allows the materials to be analyzed.

The system disclosed in U.S. Patent No. 5,699,157 also provides for a method of performing high-speed electrophoretic analysis of subject materials, which comprises the steps of holding the subject materials in solution in a channel of a microfluidic system; subjecting the materials to an electric field so that the subject materials move through the channel and separate into species bands; directing light toward the channel; receiving light from periodically spaced regions along the channel simultaneously; and analyzing the frequencies of light intensity of the received light so that velocities of the bands along the channel can be determined for analysis of said materials. The determination of the velocity of a species band determines the electrophoretic mobility of the species and its identification.

U.S. Patent No. 5,842,787, which is hereby incorporated by reference in its entirety, is generally directed to devices and systems employ channels having, at least in part, depths that are varied over those which have been previously described (such as the device disclosed in U.S. Patent No. 5,699,157), wherein said channel depths provide numerous beneficial and unexpected results such as but not limited to, a reduction in sample perturbation, reduced non-specific sample mixture by diffusion, and increased resolution.

In another embodiment, the electrophoretic method of separation comprises polyacrylamide gel electrophoresis. In a preferred embodiment, the polyacrylamide gel electrophoresis is non-denaturing, so as to differentiate the mobilities of the different RNA molecules.

### 5.6.5 DIRECT BINDING ASSAYS

The invention encompasses direct binding assays for identifying compounds that modulate the activity of an animalia and/or fungal tRNA splicing ligase. In particular, compounds that inhibit the activity of an animalia or fungal tRNA splicing ligase by directly or indirectly reducing or inhibiting the interaction between a substrate for an animalia or fungal tRNA splicing ligase and an animalia or fungal tRNA splicing ligase. Such assays are described in International Patent Publication Nos. WO 02/083837 and WO 021083953, the disclosures of which are hereby incorporated by reference in their entireties. Briefly, direct binding assays may be conducted by attaching a library of compounds to solid supports, *e.g.*, polymer beads, with each solid support having substantially one type of compound attached to its surface. The plurality of solid supports of the library is exposed in aqueous solution to a substrate for an animalia or fungal tRNA splicing ligase having a detectable label, forming a dye-labeled substrate:support-attached compound complex. Binding of a substrate to a particular compound labels the solid support, *e.g.*, bead, comprising the compound, which can be physically separated from other, unlabeled solid supports. Once labeled solid supports are identified, the chemical structures of the compounds thereon can be determined by, *e.g.*, reading a code on the solid support that correlates with the structure of the attached compound.

Alternatively, direct binding assays may be conducted by contacting a substrate for an animalia or fungal tRNA splicing ligase having a detectable label with a member of a library of compounds free in solution, in labeled tubes or microtiter wells, or a microarray. Compounds in the library that bind to the labeled substrate of an animalia or fungal tRNA splicing ligase will form a detectably labeled complex that can be identified and removed from the uncomplexed, labeled compounds in the library, and from uncomplexed, unlabeled substrate of an animalia or fungal tRNA splicing ligase, by a variety of methods including, but not limited to, methods that differentiate changes in the electrophoretic, chromatographic, or thermostable properties of the complexed substrate.

In a specific embodiment, compounds that bind to an animalia or fungal tRNA splicing ligase are identified. In accordance with this embodiment, any assay well-known to one of skill in the art for the detection of an interaction between a compound with a protein, polypeptide or peptide can be to identify compounds that bind to an animalia or fungal tRNA splicing ligase. For example, the assay can be conducted by attaching a library of compounds to solid supports, *e.g.*, polymer beads, with each solid support having substantially one type of compound attached to its surface. The plurality of solid supports of the library is exposed in aqueous solution to an animalia or fungal tRNA splicing ligase having a detectable label, forming a dye-labeled endonuclease:support-attached compound complex. Binding of the ligase to a particular compound labels the solid support, *e.g.*, bead, comprising the compound, which can be physically separated from other, unlabeled solid supports. Once labeled solid supports are identified, the chemical structures of the compounds thereon can be determined by, *e.g.*, reading a code on the solid support that correlates with the structure of the attached compound.

Alternatively, the assays may be conducted by contacting an animalia or fungal tRNA splicing ligase with a member of a library of labeled compounds free in solution, in labeled tubes or microtiter wells, or a microarray. Compounds in the library that bind to the animalia or fungal tRNA splicing ligase will form a detectably labeled complex that can be identified and removed from the uncomplexed, labeled compounds in the library, and from uncomplexed, unlabeled animalia or fungal tRNA splicing ligase, by a variety of methods.

### 5.6.6 OTHER SCREENING ASSAYS

The activity of an animalia and/or fungal tRNA splicing ligase may be determined in an assay in which the amount of substrate for a tRNA splicing ligase, to be ligated in to a mature tRNA in the presence of a compound relative to a control (preferably, a negative control and more preferably, a negative control and a positive control) is detected. Such an assay may be conducted by contacting or incubating a compound with a labeled substrate for an animalia and/or tRNA splicing ligase and a cell-free extract or purified animalia and/or tRNA splicing ligase under conditions conducive for tRNA splicing ligase activity, and measuring the amount of mature tRNA produced.

The substrate for the animalia and/or fungal tRNA splicing ligase can be labeled with any detectable agent known to one skilled in the art. Useful labels in the present invention are presented above.

In certain embodiments, a compound is contacted or incubated with a labeled substrate for an animalia and/or fungal tRNA splicing ligase and a cell-free extract or purified animalia and/or fungal tRNA splicing ligase for at least 5 minutes, at least 10 minutes, at least 15 minutes, at least 30 minutes, at least 1 hour, at least 2 hours, or more. The amount of mature tRNA produced (*e.g.*, the loss of tRNA half-molecules) is proportional to the activity of the tRNA splicing ligase. The amount of mature tRNA produced by a tRNA splicing ligase can be measured by any technique known to one skilled in the art.

In certain embodiments, the ligated product, *e.g.*, the mature tRNA is separated from the unligated tRNA splicing ligase substrate, *e.g.*, the 5' and 3' tRNA half molecules, by gel-electrophoresis. The amount of mature tRNA formed by the tRNA splicing ligase can be quantified by measuring the intensity of the signal of the mature tRNA produced relative to the signal intensity of the tRNA half-molecules. The stronger the signal produced by the in the mature tRNA and the weaker the signal of the tRNA half molecules the more active is the tRNA splicing ligase. The signal intensity can be quantified using autoradiography or a phosphoimager. If the activity of the tRNA splicing ligase is decreased in the presence of a compound, *i.e.*, if the signal of the mature tRNA relative to the tRNA half molecules is decreased compared to the reaction without the compound or in the presence of a negative control, the compound is identified as an inhibitor of the tRNA splicing ligase. In other embodiments, the amount of mature tRNA is determined using mass spectrometry.

## 5.7 CHARACTERIZATION OF THE STRUCTURE OF COMPOUNDS

The structure of the compounds identified utilizing the methods of the present invention may be determined using any method known to one skilled in the art or described herein. It will be appreciated by one skilled in the art, that depending on the type of the compound identified the optimal method for determining the structure of the compound will be used, *e.g.*, if the compound identified using the methods of the invention is a polypeptide with a molecular weight greater than 60 KDa, it is preferable to determine the structure using x-ray crystallography rather than NMR.

If the library comprises arrays or microarrays of compounds, wherein each compound has an address or identifier, the compound can be deconvoluted, *e.g.*, by crossreferencing the positive sample to original compound list that was applied to the individual test assays.

If the library is a peptide or nucleic acid library, the sequence of the compound can be determined by direct sequencing of the peptide or nucleic acid. Such methods are well known to one of skill in the art.

A number of physico-chemical techniques can be used for the *de novo* characterization of compounds bound to the target RNA. Examples of such techniques include, but are not limited to, mass spectrometry, NMR spectroscopy, X-ray crytallography and vibrational spectroscopy.

### 5.7.1 MASS SPECTROMETRY

Mass spectrometry (*e.g.*, electrospray ionization ("ESI"), matrix-assisted laser desorption-ionization ("MALDI"), and Fourier-transform ion cyclotron resonance ("FT-ICR") can be used for elucidating the structure of a compound.

MALDI uses a pulsed laser for desorption of the ions and a time-of-flight analyzer, and has been used for the detection of noncovalent tRNA:amino-acyl-tRNA synthetase complexes (Gruic-Sovulj et al., 1997, J. Biol. Chem. 272: 32084-32091). However, covalent cross-linking between the target nucleic acid and the compound is required for detection, since a non-covalently bound complex may dissociate during the MALDI process.

ESI mass spectrometry ("ESI-MS") has been of greater utility for studying noncovalent molecular interactions because, unlike the MALDI process, ESI-MS generates molecular ions with little to no fragmentation (Xavier et al., 2000, Trends Biotechnol. 18(8):349-356). ESI-MS has been used to study the complexes formed by HIV Tat peptide and protein with the TAR RNA (Sannes-Lowery et al., 1997, Anal. Chem. 69:5130-5135).

Fourier-transform ion cyclotron resonance ("FT-ICR") mass spectrometry provides high-resolution spectra, isotope-resolved precursor ion selection, and accurate mass assignments (Xavier et al., 2000, Trends Biotechnol. 18 (8):349-356). FT-ICR has been used to study the interaction of aminoglycoside antibiotics with cognate and non-cognate RNAs (Hofstadler et al., 1999, Anal. Chem. 71:3436-3440; and Griffey et al., 1999, Proc. Natl. Acad. Sci. USA 96: 10129-10133). As true for all of the mass spectrometry methods discussed herein, FT-ICR does not require labeling a compound.

An advantage of mass spectroscopy is not only the elucidation of the structure of the compound, but also the determination of the structure of the compound bound to an RNA. Such information can enable the discovery of a consensus structure of a compound that specifically binds to an RNA.

### 5.7.2 NMR SPECTROMETRY

NMR spectroscopy is a valuable technique for identifying complexed target nucleic acids by qualitatively determining changes in chemical shift, specifically from distances measured using relaxation effects, and NMR-based approaches have been used in the identification of small molecule binders of protein drug targets (Xavier et al., 2000, Trends Biotechnol. 18(8):349-356). The determination of structure-activity relationships ("SAR") by NMR is the first method for NMR described in which small molecules that bind adjacent subsites are identified by two-dimentional $^1$H-$^{15}$N spectra of the target protein (Shuker et al., 1996, Science 274:1531-1534). The signal from the bound molecule is monitored by employing line broadening, transferred NOEs and pulsed field gradient diffusion measurements (Moore, 1999, Curr. Opin. Biotechnol. 10:54-58). A strategy for lead generation by NMR using a library of small molecules has been recently described (Fejzo et al., 1999, Chem. Biol. 6:755-769).

SAR by NMR can be used to elucidate the structure of a compound.

As described above, NMR spectroscopy is a technique for identifying binding sites in target nucleic acids by qualitatively determining changes in chemical shift, specifically from distances measured using relaxation effects. Examples of NMR that can be used for the invention include, but are not limited to, one-dimentional NMR, two-dimentional NMR, correlation spectroscopy ("COSY"), and nuclear Overhauser effect ("NOE") spectroscopy. Such methods of structure determination of compounds are well-known to one of skill in the art.

Similar to mass spectroscopy, an advantage of NMR is the not only the elucidation of the structure of the compound, but also the determination of the structure of the compound bound to the RNA. Such information can enable the discovery of a consensus structure of a compound that specifically binds to an RNA.

### 5.7.3 X-RAY CRYSTALLOGRAPHY

X-ray crystallography can be used to elucidate the structure of a compound. For a review of x-ray crystallography see, *e.g.,* Blundell et al. 2002, Nat Rev Drug Discov 1(1):45-54. The first step in x-ray crystallography is the formation of crystals. The formation of crystals begins with the preparation of highly purified and soluble samples. The conditions for crystallization is then determined by optimizing several solution variables known to induce nucleation, such as pH, ionic strength, temperature, and specific concentrations of organic additives, salts and detergent. Techniques for automating the crystallization process have been developed to automate the production of high-quality protein crystals. Once crystals have been formed, the crystals are harvested and prepared for data collection. The crystals are then analyzed by diffraction (such as multi-circle diffractometers, high-speed CCD detectors, and detector off-set). Generally, multiple crystals must be screened for structure determinations.

### 5.7.4 VIBRATIONAL SPECTROSCOPY

Vibrational spectroscopy (*e.g.* infrared (IR) spectroscopy or Raman spectroscopy) can be used for elucidating the structure of a compound.

Infrared spectroscopy measures the frequencies of infrared light (wavelengths from 100 to 10,000 nm) absorbed by the compound as a result of excitation of vibrational modes according to quantum mechanical selection rules which require that absorption of light cause a change in the electric dipole moment of the molecule. The infrared spectrum of any molecule is a unique pattern of absorption wavelengths of varying intensity that can be considered as a molecular fingerprint to identify any compound.

Infrared spectra can be measured in a scanning mode by measuring the absorption of individual frequencies of light, produced by a grating which separates frequencies from a mixed-frequency infrared light source, by the compound relative to a standard intensity (double-beam instrument) or pre-measured ('blank') intensity (single-beam instrument). In a preferred embodiment, infrared spectra are measured in a pulsed mode ("FT-IR") where a mixed beam, produced by an interferometer, of all infrared light frequencies is passed through or reflected off the compound. The resulting interferogram, which may or may not be added with the resulting interferograms from subsequent pulses to increase the signal strength while averaging random noise in the electronic signal, is mathematically transformed into a spectrum using Fourier Transform or Fast Fourier Transform algorithms.

Raman spectroscopy measures the difference in frequency due to absorption of infrared frequencies of scattered visible or ultraviolet light relative to the incident beam. The incident monochromatic light beam, usually a single laser frequency, is not truly absorbed by the compound but interacts with the electric field transiently. Most of the light scattered off the sample will be unchanged (Rayleigh scattering) but a portion of the scatter light will have frequencies that are the sum or difference of the incident and molecular vibrational frequencies. The selection rules for Raman (inelastic) scattering require a change in polarizability of the molecule. While some vibrational transitions are observable in both infrared and Raman spectrometry, must are observable only with one or the other technique. The Raman spectrum of any molecule is a unique pattern of absorption wavelengths of varying intensity that can be considered as a molecular fingerprint to identify any compound.

Raman spectra are measured by submitting monochromatic light to the sample, either passed through or preferably reflected off, filtering the Rayleigh scattered light, and detecting the frequency of the Raman scattered light. An improved Raman spectrometer is described in US Patent No. 5,786,893 to Fink et al., which is hereby incorporated by reference.

Vibrational microscopy can be measured in a spatially resolved fashion to address single beads by integration of a visible microscope and spectrometer. A microscopic infrared spectrometer is described in U.S. Patent No. 5,581,085 to Reffner et al., which is hereby incorporated by reference in its entirety. An instrument that simultaneously performs a microscopic infrared and microscopic Raman analysis on a sample is described in U.S. Patent No. 5,841,139 to Sostek et al., which is hereby incorporated by reference in its entirety.

In one embodiment of the method, compounds are synthesized on polystyrene beads doped with chemically modified styrene monomers such that each resulting bead has a characteristic pattern of absorption lines in the vibrational (IR or Raman) spectrum, by methods including but not limited to those described by Fenniri et al., 2000, J. Am. Chem. Soc. 123:8151-8152. Using methods of split-pool synthesis familiar to one of skill in the art, the library of compounds is prepared so that the spectroscopic pattern of the bead identifies one of the components of the compound on the bead. Beads that have been separated according to their ability to bind target RNA can be identified by their vibrational spectrum. In one embodiment of the method, appropriate sorting and binning of the beads during synthesis then allows identification of one or more further components of the compound on any one bead. In another embodiment of the method, partial identification of the compound on a bead is possible through use of the spectroscopic pattern of the bead with or without the aid of further sorting during synthesis, followed by partial resynthesis of the possible compounds aided by doped beads and appropriate sorting during synthesis.

In another embodiment, the IR or Raman spectra of compounds are examined while the compound is still on a bead, preferably, or after cleavage from bead, using methods including but not limited to photochemical, acid, or heat treatment. The compound can be identified by comparison of the IR or Raman spectral pattern to spectra previously acquired for each compound in the combinatorial library.

## 5.8 SECONDARY ASSAYS

The compounds identified in the assays described *supra* that modulate the activity of one or more components (*e.g.*, enzymes) of an animalia and/or fungal tRNA splicing pathway (for convenience referred to herein as a "lead" compound) can be further tested for both direct binding to RNA and biological activity. In one embodiment, the compounds are tested for biological activity *in in vitro* assays and/or animal models. In another embodiment, the lead compound assessed for its effect on animalia tRNA splicing endonuclease and/or animalia tRNA splicing ligase and animalia cellular proliferation. In another embodiment, the lead compound is used to design congeners or analogs. In another embodiment, the lead compound assessed for its effect on fungal tRNA splicing endonuclease and/or fungal tRNA splicing ligase and fungal replication. In yet another embodiment, mutagenesis studies can be conducted to assess the mechanism by which a lead compound is modulating the activity of one or more components (*e.g.*, enzymes) of an animalia or fungal tRNA splicing pathway.

## 5.8.1 SPECIFICITY OF A LEAD COMPOUND

The specificity of a compound for a component (*e.g.*, an enzyme) of an animalia tRNA splicing pathway such as an animalia tRNA splicing endonuclease and/or an animalia tRNA splicing ligase can be determined by conducting one or more of the assays described in Sections 5.4, 5.5 and 5.6 utilizing a fungal cell, a fungal cell-free extract, or a puriifed fungal enzyme in the fungal tRNA splicing pathway (*e.g.*, a purified fungal tRNA splicing endonuclease or a purified fungal tRNA splicing ligase). The specificity of a compound for a component (*e.g.*, an enzyme) of a fungal tRNA splicing pathway such as a fungal tRNA splicing endonuclease and/or a fungal tRNA splicing ligase can be determined by conducting one or more of the assays described in Sections 5.4, 5.5 and 5.6 utilizing an animalia cell, an animalia cell-free extract, or a purified animalia enzyme in the animalia tRNA splicing pathway (*e.g.*, a purified animalia tRNA splicing endonuclease or a purified tRNA splicing ligase. Compounds that affect a component (*e.g.*, an enzyme) of both the animalia tRNA splicing pathway and the fungal tRNA splicing pathway may be used to prevent, treat, manage or ameliorate a proliferative disorder or one or more symptoms thereof and/or a fungal infection in the patient with a proliferative disorder. Compounds that affect only a component (*e.g.*, an enzyme) of an fungal tRNA splicing pathway may be used to prevent, treat, manage or ameliorate a fungal infection or a symptom or condition associated therewith. Compounds that affect only a component (*e.g.*, an enzyme) of an animalia tRNA splicing pathway may be used to prevent, treat, manage or ameliorate a proliferative disorder or a symptom or condition associated therewith.

## 5.8.2 ANTI-PROLIFERATIVE ASSAYS

The compounds identified in the assays described *supra* (for convenience referred to herein as a "lead" compound) can be tested for biological activity using host cells containing or engineered to contain animalia tRNA splicing enzymes including, but not limited to, tRNA splicing endonuclease and tRNA splicing ligase coupled to a functional readout system. For example, a phenotypic or physiological readout can be used to assess activity of an animalia tRNA splicing enzyme, *e.g.*, a tRNA splicing endonuclease and a tRNA splicing ligase, in the presence and absence of the lead compound.

In one embodiment, a phenotypic or physiological readout can be used to assess activity of an animalia tRNA splicing enzyme in the presence and absence of the lead compound. The animalia tRNA splicing enzyme may be overexpressed in a cell in which the animalia tRNA splicing enzyme is endogenously expressed. The effect of a lead compound can be assayed by measuring the cell growth or viability of the target cell. Such assays can be carried out with representative cells of cell types involved in a particular proliferative disorder. A lower level of proliferation or survival of the contacted cells indicates that the lead compound is effective to treat a condition in the patient characterized by uncontrolled cell growth. Alternatively, instead of culturing cells from a patient, a lead compound may be screened using cells of a tumor or malignant cell line or an endothelial cell line. Specific examples of cell culture models include, but are not limited to, for lung cancer, primary rat lung tumor cells (Swafford et al., 1997, Mol. Cell. Biol., 17:1366-1374) and large-cell undifferentiated cancer cell lines (Mabry et al, 1991, Cancer Cells, 3:53-58); colorectal cell lines for colon cancer (Park and Gazdar, 1996, J. Cell Biochem. Suppl. 24:131-141); multiple established cell lines for breast cancer (Hambly et al., 1997, Breast Cancer Res. Treat. 43:247-258; Gierthy et al., 1997, Chemosphere 34:1495-1505; Prasad and Church, 1997, Biochem. Biophys. Res. Commun. 232:14-19); a number of well-characterized cell models for prostate cancer (Webber et al., 1996, Prostate, Part 1, 29:386-394; Part 2, 30:58-64; and Part 3, 30:136-142; Boulikas, 1997, Anticancer Res. 17:1471-1505); for genitourinary cancers, continuous human bladder cancer cell lines (Ribeiro et al., 1997, Int. J. Radiat. Biol. 72:11-20); organ cultures of transitional cell carcinomas (Booth et al., 1997, Lab Invest. 76: 843-857) and rat progression models (Vet et al., 1997, Biochim. Biophys Acta 1360:39-44); and established cell lines for leukemias and lymphomas (Drexler, 1994, Leuk. Res. 18:919-927, Tohyama, 1997, Int. J. Hematol. 65:309-317).

Many assays well-known in the art can be used to assess the survival and/or growth of a patient cell or cell line following exposure to a lead compound; for example, cell proliferation can be assayed by measuring Bromodeoxyuridine (BrDU) incorporation (*see, e.g.,* Hoshino et al., 1986, Int. J. Cancer 38, 369; Campana et al., 1988, J. Immunol. Meth. 107:79) or ($^3$H)-thymidine incorporation (*see, e.g.,* Chen, J., 1996, Oncogene 13:1395-403; Jeoung, J., 1995, J. Biol. Chem. 270:18367-73), by direct cell count, by detecting changes in transcription, translation or activity of known genes such as proto-oncogenes (*e.g., fos, myc*) or cell cycle markers (Rb, cdc2, cyclin A, D1, D2, D3, E, etc). The levels of such protein and mRNA and activity can be determined by any method well known in the art. For example, protein can be quantitated by known immunodiagnostic methods such as Western blotting or immunoprecipitation using commercially available antibodies. mRNA can be quantitated using methods that are well known and routine in the art, for example, using northern analysis, RNase protection, the polymerase chain reaction in connection with the reverse transcription. Cell viability can be assessed by using trypan-blue staining or other cell death or viability markers known in the art. In a specific embodiment, the level of cellular ATP is measured to determined cell viability. Differentiation can be assessed, for example, visually based on changes in morphology.

The lead compound can also be assessed for its ability to inhibit cell transformation (or progression to malignant phenotype) *in vitro.* In this embodiment, cells with a transformed cell phenotype are contacted with a lead compound, and examined for change in characteristics associated with a transformed phenotype (a set of *in vitro* characteristics associated with a tumorigenic ability *in vivo*), for example, but not limited to, colony formation in soft agar, a more rounded cell morphology, looser substratum attachment, loss of contact inhibition, loss of anchorage dependence, release of proteases such as plasminogen activator, increased sugar transport, decreased serum requirement, or expression of fetal antigens, etc. (see Luria et al., 1978, General Virology, 3d Ed., John Wiley & Sons, New York, pp. 436-446).

Loss of invasiveness or decreased adhesion can also be assessed to demonstrate the anti-cancer effects of a lead compound. For example, an aspect of the formation of a metastatic cancer is the ability of a precancerous or cancerous cell to detach from primary site of disease and establish a novel colony of growth at a secondary site. The ability of a cell to invade peripheral sites reflects its potential for a cancerous state. Loss of invasiveness can be measured by a variety of techniques known in the art including, for example, induction of E-cadherin-mediated cell-cell adhesion. Such E-cadherin-mediated adhesion can result in phenotypic reversion and loss of invasiveness (Hordijk et al., 1997, Science 278:1464-66).

Loss of invasiveness can further be examined by inhibition of cell migration. A variety of 2-dimensional and 3-dimensional cellular matrices are commercially available (Calbiochem-Novabiochem Corp. San Diego, CA). Cell migration across or into a matrix can be examined using microscopy, time-lapsed photography or videography, or by any method in the art allowing measurement of cellular migration. In a related embodiment, loss of invasiveness is examined by response to hepatocyte growth factor (HGF). HGF-induced cell scattering is correlated with invasiveness of cells such as Madin-Darby canine kidney (MDCK) cells. This assay identifies a cell population that has lost cell scattering activity in response to HGF (Hordijk et al., 1997, Science 278:1464-66).

Alternatively, loss of invasiveness can be measured by cell migration through a chemotaxis chamber (Neuroprobe/ Precision Biochemicals Inc. Vancouver, BC). In such assay, a chemo-attractant agent is incubated on one side of the chamber (*e.g.*, the bottom chamber) and cells are plated on a filter separating the opposite side (*e.g.*, the top chamber). In order for cells to pass from the top chamber to the bottom chamber, the cells must actively migrate through small pores in the filter. Checkerboard analysis of the number of cells that have migrated can then be correlated with invasiveness (*see e.g.*, Ohnishi, T., 1993, Biochem. Biophys. Res. Commun.193:518-25).

In a specific embodiment, the effect of a lead compound or the proliferation of a normal animalia cell (*i.e.*, a cell not undergoing abnormal proliferation; not a cancer cell or cell line) versus the proliferation of an abnormal cell undergoing abnormal proliferation (*e.g.*, a cancer cell or cell line). The compounds that selectively exhibit cells undergoing abnormal proliferation (*e.g.*, transformed, malignant cells or cell lines), while aliening for normal cellular growth and metabolism are preferably utilized as anti-proliferative compounds in the treatment, prevention, management or amelioration of a proliferative disorder or one or more symptoms thereof.

### 5.8.3 ANIMAL MODELS AND TOXICITY STUDIES

The lead compounds identified in the assays described herein can be tested for biological activity using animal models for a proliferative disorder. These include animals engineered to contain an animalia tRNA splicing endonuclease and/or an animalia tRNA splicing ligase coupled to a functional readout system, such as a transgenic mouse. Such animal model systems include, but are not limited to, rats, mice, chicken, cows, monkeys, pigs, dogs, rabbits, etc. In a specific embodiment of the invention, a compound identified in accordance with the methods of the invention is tested in a mouse model system. Such model systems are widely used and well-known to the skilled artisan such as the SCID mouse model or transgenic mice.

The anti-angiogenic activity of a compound identified in accordance with the invention can be determined by using various experimental animal models of vascularized tumors. The anti-tumor activity of a compound identified in accordance with the invention can be determined by administering the compound to an animal model and verifying that the compound is effective in reducing the proliferation or spread of cancer cells in said animal model. An example of an animal model for human cancer in general includes, but is not limited to, spontaneously occurring tumors of companion animals (see, *e.g.*, Vail & MacEwen, 2000, Cancer Invest 18(8):781-92).

Examples of animal models for lung cancer include, but are not limited to, lung cancer animal models described by Zhang & Roth (1994, In Vivo 8(5):755-69) and a transgenic mouse model with disrupted p53 function (see, *e.g.,* Morris et al., 1998, J La State Med Soc 150(4):179-85). An example of an animal model for breast cancer includes, but is not limited to, a transgenic mouse that overexpresses cyclin D1 (see, *e.g.*, Hosokawa et al., 2001, Transgenic Res 10(5):471-8). An example of an animal model for colon cancer includes, but is not limited to, a TCRbeta and p53 double knockout mouse (see, *e.g.*, Kado et al., 2001, Cancer Res 61(6):2395-8). Examples of animal models for pancreatic cancer include, but are not limited to, a metastatic model of Panc02 murine pancreatic adenocarcinoma (see, *e.g.,* Wang et al., 2001, Int J Pancreatol 29(1):37-46) and nu-nu mice generated in subcutaneous pancreatic tumours (see, *e.g.*, Ghaneh et al., 2001, Gene Ther 8(3):199-208). Examples of animal models for non-Hodgkin's lymphoma include, but are not limited to, a severe combined immunodeficiency ("SCID") mouse (see, *e.g.*, Bryant et al., 2000, Lab Invest 80 (4):553-73) and an IgHmu-HOX11 transgenic mouse (see, *e.g.,* Hough et al., 1998, Proc Natl Acad Sci USA 95(23): 13853-8). An example of an animal model for esophageal cancer includes, but is not limited to, a mouse transgenic for the human papillomavirus type 16 E7 oncogene (see, *e.g.*, Herber et al., 1996, J Virol 70(3):1873-81). Examples of animal models for colorectal carcinomas include, but are not limited to, Apc mouse models (see, *e.g.*, Fodde & Smits, 2001, Trends Mol Med 7(8):369-73 and Kuraguchi et al., 2000, Oncogene 19(50):5755-63).

The lead compounds identified in the assays described herein for biological activity using animal models for a fungal infection. Animal models for fungal infections, such as *Candida* infections, zygomycosis, *Candida* mastitis, progressive disseminated trichosporonosis with latent trichosporonemia, disseminated candidiasis, pulmonary paracoccidioidomycosis, pulmonary aspergillosis, *Pnuemocystis carinii* pneumonia, cryptococcal meningitis, coccidioidal meningoencephalitis and cerebrospinal vasculitis, *Aspergillus niger* infection, *Fusarium* keratitis, paranasal sinus mycoses, *Aspergillus fumigatus* endocarditis, tibial dyschondroplasia, *Candida glabrata* vaginitis, oropharyngeal candidiasis, X-linked chronic granulomatous disease, tinea pedis, cutaneous candidiasis, mycotic placentitis, disseminated trichosporonosis, allergic bronchopulmonary aspergillosis, mycotic keratitis, *Cryptococcus neoformans* infection, fungal peritonitis, *Curvularia* geniculata infection, staphylococcal endophthalmitis, sporotrichosis, and dermatophytosis have been developed (see, *e.g.*, Arendrup et al., 2002, Infection 30(5):286-291; Kamie, 2001, Mycopathologia 152(1):5-13; Guhad et al., 200, FEMS Microbiol Lett. 192(1):27-31; Yamagata et al., 200, J Clin Microbiol. 38(9):32606; Andrutis et al., 2000, J Clin Microbiol. 38(6):2317-2323; Cock et al., 2000, Rev Inst Med Trop Sao Paulo 42(2):59-66; Shibuya et al., 1999, Microb Pathog. 27(3):123-131; Beers et al., 1999, J Lab Clin Med. 133(5):423-33; Najvar et al., 1999, Antimicrob Agents Chemother. 43(2):413-414; Williams et al., 1988, J Infect Dis. 178(4):1217-1221; Yoshida, 1988, Kansenshogaku Zasshi 72(6):621-630; Alexandrakis et al., 1998, Br J Ophthalmol. 82(3):306-11; Chakrabarti et al., 1997, J Med Vet Mycol. 35 (4):295-97; Martin et al., 1997, Antimicrob Agents Chemother. 41(1):13-16; Chu et al., 1996, Avian Dis. 40(3):715-19; Fidel et al, 1996, J Infect Dis. 173(2):425-31; Cole et al., 1995, FEMS Microbiol Lett. 15; 126(2):177-80; Pollock et al., 1995, Nat Genet. 9(2):202-09; Uchida et al., 1994, Jpn J Antibiot. 47(10):1407-12; Maebashi et al., 1994, J Med Vet Mycol. 32(5):349-59; Jensen & Schonheyder, 1993, J Exp Anim Sci. 35(4):155-60; Goksalan & Anaissie, 1992, Infect Immun. 60(8):3339-44; Kurup et al., 1992, J Immunol. 148(12):3783-88; Singh et al., 1990, Mycopatholgia 112(3):127-37; Salkowski & Balish, 1990, Infect Immun. 58(10):3300-06; Ahmad et al., 1985, Am J Kidney Dis. 7(2):153-56; Alture-Werber E, Edberg SC, 1985, Mycopathologia 89(2):69-73; Kane et al., 1981, Antimicrob Agents Chemother. 20(5): 595-99; Barbee et al., 1977, Am J Pathol. 86(1):281-84; and Maestrone et al., 1973, Am J Vet Res. 34(6):833-36.

The toxicity and/or efficacy of a compound identified in accordance with the invention can be determined by standard pharmaceutical procedures in cell cultures or experimental animals, *e.g.*, for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. A compound identified in accordance with the invention that exhibits large therapeutic indices is preferred. While a compound identified in accordance with the invention that exhibits toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of a compound identified in accordance with the invention for use in humans. The dosage of such agents lies preferably within a range of circulating concentrations that include the $ED_{50}$ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the $IC_{50}$ (*i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography.

### 5.8.4 <u>ANTI-FUNGAL ASSAYS</u>

The compounds identified in the assays described supra (for convenience referred to herein as a "lead" compound) can be tested for anti-fungal activity. Any of the standard anti-fungal assays well-known in the art can be used to assess the anti-fungal activity of a lead compound. The anti-fungal effect on different species of fungus can be tested. The tests recommended by the National Committee for Clinical Laboratories (NCCLS) (*See* National Committee for Clinical Laboratories Standards. 1995, Proposed Standard M27T. Villanova, Pa., all of which is incorporated herein by reference in its entirety) and other methods known to those skilled in the art (Pfaller et al., 1993, Infectious Dis. Clin. N. Am. 7: 435-444) can be used to assess the anti-fungal effect of a lead compound. The anti-fungal activities of lead compounds can be tested using macrodilution methods and/or microdilution methods using protocols well-known to those skilled in the art (*see, e.g.,* Clancy et al., 1997 Journal of Clinical Microbiology, 35(11): 2878-82; Ryder et al., 1998, Antimicrobial Agents and Chemotherapy, 42(5): 1057-61; U.S. 5,521,153; U.S. 5,883,120, U.S. 5,521,169, all of which are incorporated by reference in their entirety). Briefly, a fungal strain is cultured in an appropriate liquid media, and grown at an appropriate temperature, depending on the particular fungal strain used for a determined amount of time, which is also depends on the particular fungal strain used. An innoculum is then prepared photometrically and the turbidity of the suspension is matched to that of a standard, *e.g.*, a McFarland standard. The effect of the lead compound on the turbidity of the inoculum is determined visually or spectrophotometrically. The minimal inhibitory concentration of the lead compound (MIC) is determined, which is defined as the lowest concentration of the lead compound which prevents visible growth of an inoculum as measured by determining the culture turbidity.

The anti-fungal activity of a lead compound can also be determined utilizing colorimetric based assays well-known to one of skill in the art. One exemplary colorimetric assay that can be used to assess the anti-fungal activity of a lead compound is described by Pfaller et al. (1994, Journal of Clinical Microbiology, 32(8): 1993-6, which is incorporated herein by reference in its entirety; also *see* Tiballi et al., 1995, Journal of Clinical Microbiology, 33(4): 915-7). This assay employs a colorimetric endpoint using an oxidation-reduction indicator (Alamar Biosciences, Inc., Sacramento CA).

The anti-fungal activity of a lead compound can also be determined utilizing photometric assays well-known to one of skill in the art (*see, e.g.,* Clancy et al., 1997 Journal of Clinical Microbiology, 35(11): 2878-82; Jahn et al., 1995, Journal of Clinical Microbiology, 33(3): 661-667, each of which is incorporated herein by reference in its entirety). This photometric assay is based on quantifying mitochondrial respiration by viable fungi through the reduction of 3-(4,5-dimethyl-2thiazolyl)-2,5,-diphenyl-2H-tetrazolium bromide (MTT) to formazan. MIC's determined by this assay are defined as the highest concentration of the test compound associated with the first precipitous drop in optical density. In some embodiments, the lead compounds are assayed for anti-fungal activity using macrodilution, microdilution and MTT assays in parallel.

The antifungal properties of the lead compounds may also be determined from a fungal lysis assay, as well as by other methods, including, *inter alia,* growth inhibition assays, fluorescence-based fungal viability assays, flow cytometry analyses, and other standard assays known to those skilled in the art.

In some embodiments, the assays for growth inhibition of a fungal strain by a lead compound are used to derive an "end.$_{50}$" value for the compound, which is defined as the concentration of the compound required to kill 50% of the fungal cell. Alternatively, growth inhibition by a lead compound may also be characterized in terms of the minimum inhibitory concentration (MIC), which is the concentration of compound required to achieve inhibition of fungal cell growth. Such values are well known to those in the art as representative of the effectiveness of a particular antifungal agent against a particular organism or group of organisms. For instance, cytolysis of a fungal population by an antifungal compound can also be characterized, as described above by the minimum inhibitory concentration, which is the concentration required to reduce the viable fungal population by 99.9%. The value of MIC.$_{50}$ can also be used, defined as the concentration of a compound required to reduce the viable fungal population by 50%. In preferred embodiments, the compounds of the present invention are selected for use based, *inter alia,* on having MIC values of less than 25 ug/mL, more preferably less than 7 ug/mL, and even more preferably less than 1 ug/mL against a desired fungal target, *e.g.*, Candida albicans.

Another parameter useful in identifying and measuring the antifungal effectiveness of a lead compound is the determination of the kinetics of the antifungal activity of the lead compound. Such a determination can be made by determining antifungal activity as a function of time. In a preferred embodiment, a lead compound displays kinetics which result in efficient lysis of a fungal cell. In another preferred embodiment, a lead compound is fungicidal.

In a most preferred embodiment, a lead compound displays selective toxicity to target microorganisms (in particular, fungi) and minimal toxicity to animalia (preferably mammalian cells, and most preferably human cells). Determination of the toxic dose (or "LD.$_{50}$ ") can be carried out using protocols well-known in the field of pharmacology. Ascertaining the effect of a lead compound on animalia (preferably, mammalian cells and most preferably human cells) is preferably performed using tissue culture assays, well-known to those skilled in that art (*see,* for example, Gootz, T. D. (1990) Clin. Microbiol. Rev. 3:13-31, which is incorporated herein by reference in its entirety). For mammalian cells, such assay methods include, *inter alia,* trypan blue exclusion and MTT assays (Moore et al. (1994) Compound Research 7:265-269 which is incorporated herein by reference in its entirety). Where a specific cell type may release a specific metabolite upon changes in membrane permeability, that specific metabolite may be assayed, *e.g.*, the release of hemoglobin upon the lysis of red blood cells (Srinivas et al. (1992) J. Biol. Chem. 267:7121-7127 which is incorporated herein by reference in its entirety). The lead compounds are preferably tested against primary cells, *e.g.*, using human skin fibroblasts (HSF) or fetal equine kidney (FEK) cell cultures, or other primary cell cultures routinely used by those skilled in the art. Permanent cell lines may also be used, *e.g.*, Jurkat cells. In some embodiments, the lead compounds are tested in cancer lines, including but not limited to Caco-2 (human colon carcinoma cell line) and Huh7 (human hepatoma cell line). In yet other embodiments, the lead compounds of the inventiom may be tested in peripheral blood mononuclear cells (PBMC). In preferred embodiments, the lead compounds are selected for use in animals, or animal cell/tissue culture based, at least in part, on having LD.$_{50}$'s at least one order of magnitude greater than the MIC or ED.sub.$_{50}$ as the case may be, and even more preferably at least two, three and even four orders of magnitude greater. That is, in preferred embodiments, where the lead compounds are to be administered to an animal, a suitable therapeutic index is preferably greater than 10, and more preferably greater than 100, 1000 or even 10,000.

### 5.8.5 DESIGN OF CONGENERS OR ANALOGS

The compounds which display the desired biological activity can be used as lead compounds for the development or design of congeners or analogs having useful pharmacological activity. For example, once a lead compound is identified, molecular modeling techniques can be used to design variants of the compound that can be more effective. Examples of molecular modeling systems are the CHARM and QUANTA programs (Polygen Corporation, Waltham, MA). CHARM performs the energy minimization and molecular dynamics functions. QUANTA performs the construction, graphic modelling and analysis of molecular structure. QUANTA allows interactive construction, modification, visualization, and analysis of the behavior of molecules with each other.

A number of articles review computer modeling of drugs interactive with specific proteins, such as Rotivinen et al., 1988, Acta Pharmaceutical Fennica 97:159-166; Ripka, 1998, New Scientist 54-57; McKinaly & Rossmann, 1989, Annu. Rev. Pharmacol. Toxiciol. 29:111-122; Perry & Davies, OSAR: Quantitative Structure-Activity Relationships in Drug Design pp. 189-193 (Alan R. Liss, Inc. 1989); Lewis & Dean, 1989, Proc. R. Soc. Lond. 236:125-140 and 141-162; Askew et al., 1989, J. Am. Chem. Soc. 111:1082-1090. Other computer programs that screen and graphically depict chemicals are available from companies such as BioDesign, Inc. (Pasadena, California), Allelix, Inc. (Mississauga, Ontario, Canada), and Hypercube, Inc. (Cambridge, Ontario). Although these are primarily designed for application to drugs specific to particular proteins, they can be adapted to design of drugs specific to any identified region. The analogs and congeners can be tested for binding to an animalia tRNA splicing endonuclease using the above-described screens for biologic activity. Alternatively, lead compounds with little or no biologic activity, as ascertained in the screen, can also be used to design analogs and congeners of the compound that have biologic activity.

### 5.8.6 MUTAGENESIS STUDIES

The amino acid residue of an animalia or fungal tRNA splicing ligase and/or the nucleotide sequence of a substrate for an animalia or fungal tRNA splicing ligase that are necessary for a compound identified in accordance with the invention to modulate the activity of an animalia and/or fungal tRNA splicing ligase can be determined utilizing standard mutagenesis techniques well-known to one of skill in the art. Further, the subunit(s) of an animalia or fungal tRNA splicing endonuclease and/or the nucleotide sequence of a substrate for an animalia or fungal tRNA splicing endonuclease that are necessary for a compound identified in accordance with the methods of the invention to modulate the activity of an animalia or fungal tRNA splicing endonuclease can be determined utilizing standard mutagenesis techniques well-known to one of skill in the art. One or more mutations (*e.g.*, deletions, additions and/or substitutions) may be introduced into an enzyme of the tRNA splicing pathway (*e.g.*, a RNA splicing endonuclease or a tRNA splicing ligase) or a subunit or bioligically active fragment thereof and the effect of the mutations on the activity of the enzyme in the presence or absence of a compound can be determined using an assay described herein. Moreover, one or more mutations (*e.g.*, deletions, additions, and/or substitutions) may also be introduced into a substrate for an enzyme in the tRNA splicing pathway (*e.g.*, a tRNA splicing endonuclease or a tRNA splicing ligase) and the effect of the mutations on the activity of the enzyme in the presence or absence of a compound can be determined using an assay described herein. In particular, one or more mutations (*e.g.*, deletions, additions and/or substitutions) may be introduced into the nucleotide sequence for a tRNA intron within the open frame reading of a reporter gene and the effect on the expression of a reporter gene in a reporter gene-based assay described herein can be determined. If the mutation in the tRNA intron affects the ability of the compound to modulate the expression of the reporter gene, then the mutated sequence plays a role in the activity of the tRNA splicing endonuclease.

Standard techniques known to those of skill in the art can be used to introduce mutations in the nucleotide sequence of an enzyme in the tRNA splicing pathway and/or the nucleotide sequence of a substrate for an enzyme in the tRNA splicing pathway, including, for example, site-directed mutagenesis and PCR-mediated mutagenesis. In a specific embodiment, less than 75 nucleic acid residue substitutions, less than 50 nucleic acid residue substitutions, less than 45 nucleic acid residue substitutions, less than 40 nucleic acid residue substitutions, less than 35 nucleic acid residue substitutions, less than 30 nucleic acid residue substitutions, less than 25 nucleic acid residue substitutions, less than 20 nucleic acid residue substitutions, less than 15 nucleic acid residue substitutions, less than 10 nucleic acid residue substitutions, or less than 5 nucleic acid residue substitutions are introduced into the nucleotide sequence of an enzyme in the tRNA splicing pathway and/or the nucleotide sequence of a substrate for an enzyme in the tRNA splicing pathway.

### 5.9 USE OF IDENTIFIED COMPOUNDS TO PREVENT, TREAT OR MANAGE A PROLIFERATIVE DISORDER

The present invention provides methods of preventing, treating, managing or ameliorating a proliferative disorder or one or more symptoms thereof, said methods comprising administering to a subject in need thereof one or more compounds identified in accordance with the methods of the invention that inhibit or reduce the activity of one or more enzymes in animalia tRNA splicing pathway. In one embodiment, the invention provides a method of preventing, treating or ameliorating a proliferative disorder or one or more symptoms thereof, said method comprising administering to a subject in need thereof a dose of a prophylactically or therapeutically effective amount of one or more compounds identified in accordance with the methods of the invention that inhibit or reduce the activity of one or more enzymes in an animalia tRNA splicing pathway. In another embodiment, a compound identified in accordance with the methods of the invention is not administered to prevent, treat, manage or ameliorate a proliferative disorder or one or more symptoms thereof, if such compound has been used previously to prevent, treat, manage or ameliorate said proliferative disorder.

The invention also provides methods of preventing, treating, managing or ameliorating a proliferative disorder or one or more symptoms thereof, said methods comprising administering to a subject in need thereof one or more of the compounds identified utilizing the screening methods described herein that inhibit or reduce the activity of one or more enzymes in an animalia tRNA splicing pathway, and one or more other therapies (*e.g.*, prophylactic or therapeutic agents), which are currently being used, have been used or are known to be useful in the prevention, treatment, management or amelioration of said proliferative disorder of one or more symptoms thereof (including, but not limited to the prophylactic or therapeutic agents listed in Section 5.9.1 hereinbelow and agents useful in preventing, treating, managing or amelining psoriasis or fibrosis. The therapies (*e.g.*, prophylactic or therapeutic agents) of the combination therapies of the invention can be administered sequentially or concurrently. In a specific embodiment, the combination therapies of the invention comprise a compound of the invention that inhibit or reduce the activity of one or more enzymes in an animalia tRNA splicing pathway and at least one other therapy (eg., a prophylactic ortherapeutic agen) which has the same mechanism of action as said compound. In another embodiment, the combination therapies of the invention comprise a compound of the invention that inhibit or reduce the activity of one or more enzymes in an animalia tRNA splicing pathway and at least one other therapy (*e.g.*, a prophylactic or therapeutic agent) which has a different mechanism of action than said compound. The combination therapies of the present invention improve the prophylactic or therapeutic effect of a compound of the invention by functioning together with the compound to have an additive or synergistic effect. The combination therapies of the present invention reduce the side effects associated with each therapy (*e.g.*, each prophylactic or therapeutic agent).

The prophylactic or therapeutic agents of the combination therapies can be administered to a subject in the same pharmaceutical composition. Alternatively, the prophylactic or therapeutic agents of the combination therapies can be administered concurrently to a subj ect in separate pharmaceutical compositions. The prophylactic or therapeutic agents may be administered to a subject by the same or different routes of administration.

In specific embodiment, a pharmaceutical composition comprising one or more compounds identified in a screening assay described herein that inhibit or reduce the activity of an enzyme in an animalia tRNA splicing pathway is administered to a subject, preferably a human, to prevent, treat, manage or ameliorate a proliferative disorder or one or more symptoms thereof. In accordance with this embodiment, the pharmaceutical composition of may also comprise one or more other therapies (*e.g.*, prophylactic or therapeutic agents) which are currently being used, have been used or are known to be useful in the prevention, treatment, management or amelioration of a proliferative disorder or one or more symptoms thereof.

A compound identified in accordance with the methods of the invention that inhibits or reduces the activity of an enzyme in an animalia tRNA splicing pathway may be used as a first, second, third, fourth or fifth line of therapy for the prevention, treatment, management or amelioration of a proliferative disorder or one or more symptoms thereof. The invention provides methods for treating, managing or ameliorating a proliferative disorder (*e.g.*, cancer) or one or more symptoms thereof in a subject refractory to conventional therapies for such a proliferative disorder, said methods comprising administering to said subject a dose of a prophylactically or therapeutically effective amount of a compound identified in accordance with the methods of the invention that inhibits or reduces the activity of an enzyme in an animalia tRNA splicing pathway. A cancer may be determined to be refractory to a therapy means when at least some significant portion of the cancer cells are not killed or their cell division arrested in response to the therapy. Such a determination can be made either *in vivo or in vitro* by any method well-known to one of skill in the art for assaying the effectiveness of a therapy on cancer cells, using the art-accepted meanings of "refractory" in such a context. In a specific embodiment, a cancer is refractory where the number of cancer cells has not been significantly reduced, or has increased.

The invention provides methods for treating, managing or ameliorating a proliferative disorder or one or more symptoms thereof in a subject refractory to existing single agent therapies for such a proliferative disorder, said methods comprising administering to said subject a dose of a prophylactically or therapeutically effective amount of a compound identified in accordance with the methods of the invention that inhibits or reduces the activity of an enzyme in an animalia tRNA splicing pathway and a dose of a prophylactically or therapeutically effective amount of one or more other therapies (*e.g.*, one or more other prophylactic or therapeutic agents). The invention also provides methods for treating, managing or ameliorating a proliferative disorder or one or more symptoms thereof, said method comprising administering a compound identified in accordance with the methods of the invention that inhibits or reduces the activity of an enzyme in an animalia tRNA splicing pathway in combination with any other therapy (*e.g.*, radiation therapy, chemotherapy or surgery) to patients who have proven refractory to other therapies but are no longer on these therapies. The invention also provides methods for the treatment or management of a patient having a proliferative disorder (*e.g.*, cancer) and immunosuppressed by reason of having previously undergone other therapies. The invention also provides alternative methods for the treatment or management of a proliferative disorder such as cancer where chemotherapy, radiation therapy, hormonal therapy, and/or biological therapy/immunotherapy has proven or may prove too toxic, *i.e.*, results in unacceptable or unbearable side effects, for the subject being treated or managed. Further, the invention provides methods for preventing the recurrence of a proliferative disorder such as cancer in patients that have been treated and have no disease activity by administering a compound identified in accordance with the methods of the invention that inhibits or reduces the activity of an enzyme in an animalia tRNA splicing pathway.

Proliferative disorders that can be prevented, treated, managed or ameliorated by the methods encompassed by the invention include, but are not limited to, neoplasms, tumors, metastases, or any disease or disorder characterized by uncontrolled cell growth (*e.g.*, psriasis). In a specific embodiment, the proliferative disorder that is prevented treated, managed or ameliorative in accordance with the methods of the invention is a non-cancerous disorder characterized or associated with hyper-proliferation of cells. Examples of such disorders include, but are not limtied to, psoraisis, and pulminary fibrosis. In another embodiment, the proliferative disorder that is prevented, treated, managed or ameliorated in accordance with the methods of the invention is a benign or malignant cancer. The cancer may be a primary or metastatic cancer.

Specific examples of cancers that can be prevented, treated, managed or ameliorated by the methods encompassed by the invention include, but are not limited to, cancer of the head, neck, eye, mouth, throat, esophagus, chest, bone, lung, colon, rectum, stomach, prostate, breast, ovaries, kidney, liver, pancreas, and brain. Additional cancers include, but are not limited to, the following: leukemias such as but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cyclic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma, gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell cancer, adenocarcinoma, hypernephroma, fibrosarcoma, transitional cell cancer (renal pelvis and/or uterer); Wihns' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, *see* Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America). It is also contemplated that cancers caused by aberrations in apoptosis can also be prevented, treated, managed or ameliorated by the methods and compositions of the invention. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes.

## 5.9.1 ANTI-CANCER THERAPIES

The present invention provides methods of preventing, treating, managing or ameliorating cancer or one or more symptoms thereof, said methods comprising administering to a subject in need thereof one or more compounds identified in accordance with the methods of the invention that inhibit or reduce the activity of an enzyme in an animalia tRNA splicing pathway and one or more other therapies (*e.g.*, prophylactic or therapeutic agents). Therapeutic or prophylactic agents include, but are not limited to, peptides, polypeptides, fusion proteins, nucleic acid molecules, small molecules, mimetic agents, synthetic drugs, inorganic molecules, and organic molecules. Any agent or therapy (*e.g.*, chemotherapies, radiation therapies, hormonal therapies, and/or biological therapies/immunotherapies) which is known to be useful, or which has been used or is currently being used for the prevention, treatment, management or amelioration of cancer or one or more symptoms thereof can be used in combination with a compound identified in accordance with the methods of the invention that inhibit or reduce the activity of an enzyme in an animalia tRNA splicing pathway. Examples of such agents (*i.e.*, anti-cancer agents) include, but are not limited to, angiogenesis inhibitors, topoisomerase inhibitors and immunomodulatory agents (such as chemotherapeutic agents and agents other than chemotherapeutic agents that have an immunomodulatory effect).

Angiogenesis inhibitors (*i.e.*, anti-angiogenic agents) include, but are not limited to, angiostatin (plasminogen fragment); antiangiogenic antithrombin III; angiozyme; ABT-627; Bay 12-9566; Benefin; Bevacizumab; BMS-275291; cartilage-derived inhibitor (CDI); CAI; CD59 complement fragment; CEP-7055; Col 3; combretastatin A-4; endostatin (collagen XVIII fragment); fibronectin fragment; Gro-beta; Halofuginone; Heparinases; Heparin hexasaccharide fragment; HMV833; human chorionic gonadotropin (hCG); IM-862; Interferon alpha/beta/gamma; Interferon inducible protein (IP-10); Interleukin-12; Kringle 5 (plasminogen fragment); Marimastat; Metalloproteinase inhibitors (TIMPs); 2-methoxyestradiol; MMI 270 (CGS 27023A); MoAb IMC-1C11; Neovastat; NM-3; Panzem; PI-88; Placental ribonuclease inhibitor; plasminogen activator inhibitor; platelet factor-4 (PF4); Prinomastat; Prolactin 16kDa fragment; Proliferin-related protein (PRP); PTK 787/ZK 222594; retinoids; solimastat; squalamine; SS 3304; SU 5416; SU6668; SU11248; tetrahydrocortisol-S; tetrathiomolybdate; thalidomide; thrombospondin-1 (TSP-1); TNP-470; transforming growth factor-beta; vasculostatin; vasostatin (calreticulin fragment); ZD6126; ZD 64744; and farnesyl transferase inhibitors (FTI). Chemotherapeutic agents include, but are not limited to, methothrexate, leflunomide, cyclophosphamide, cytoxan, Immuran, cyclosporine A, minocycline, azathioprine, antibiotics (*e.g.*, FK506 (tacrolimus)), methylprednisolone (MP), corticosteroids, steriods, mycophenolate mofetil, rapamycin (siro limus), mizoribine, deoxyspergualin, brequinar, and malononitriloamindes (*e.g.*, leflunamide). Immunomodulatory agents other than chemotherapeutics include but are not limited to, anti-T cell receptor antibodies (*e.g.*, anti-CD4 antibodies (*e.g.*, cM-T412 (Boeringer), IDEC-CE9.1® (IDEC and SKB), mAB 4162W94, Orthoclone and OKTcdr4a (Janssen-Cilag)), anti-CD3 antibodies (*e.g.*, Nuvion (Product Design Labs), OKT3 (Johnson & Johnson), or Rituxan (IDEC)), anti-CD5 antibodies (*e.g.*, an anti-CD5 ricin-linked immunoconjugate), anti-CD7 antibodies (*e.g.* , CHH-380 (Novartis)), anti-CD8 antibodies, anti-CD40 ligand monoclonal antibodies (*e.g.*, IDEC-131 (IDEC)), anti-CD52 antibodies (*e.g.*, CAMPATH 1H (Ilex)), anti-CD2 antibodies (*e.g.*, MEDI-507 (MedImmune, Inc., International Publication Nos. WO 02/098370 and WO 02/069904), anti-CD11a antibodies (*e.g.*, Xanelim (Genentech)), and anti-B7 antibodies (*e.g.*, IDEC-114) (IDEC))); CTLA4-immunoglobulin; LFA-3TIP (Biogen, International Publication No. WO 93/08656 and U.S. Patent No. 6,162,432); soluble cytokine receptors (*e.g.*, the extracellular domain of a TNF-alpha receptor or a fragment thereof, the extracellular domain of an IL-1beta receptor or a fragment thereof, and the extracellular domain of an IL-6 receptor or a fragment thereof), cytokines or fragments thereof (*e.g.*, interleukin (IL)-2, IL-3, IL-4, IL-5, IL-6, IL-7, IL-8, IL-9, IL-10, IL-11, IL-12, IL-15, IL-23, TNF-alpha, TNF-beta, interferon (IFN)-alpha, IFN-beta, IFN-gamma, and GM-CSF), anti-cytokine receptor antibodies (*e.g.*, anti-IFN receptor antibodies, anti-IL-2 receptor antibodies (*e.g.*, Zenapax (Protein Design Labs)), anti-IL-4 receptor antibodies, anti-IL-6 receptor antibodies, anti-IL-10 receptor antibodies, and anti-IL-12 receptor antibodies), and anti-cytokine antibodies (*e.g.*, anti-IFN antibodies, anti-TNF-alpha antibodies, anti-IL-lbeta antibodies, anti-IL-6 antibodies, anti-IL-8 antibodies (*e.g.*, ABX-IL-8 (Abgenix)), and anti-IL-12 antibodies).

Specific examples of anti-cancer agents which can be used in accordance with the methods of the invention include, but not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium;

gemcitabine; gemcitabine hydrochloride; hydroxyurea; idarubicin hydrochloride; ifosfamide; ilmofosine; interleukin-2 (including recombinant interleukin-2, or rIL2), interferon alpha-2a; interferon alpha-2b; interferon alpha-n1; interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin, loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; vorozole; zanoterone; zeniplatin; zilascorb; and zinostatin stimalamer.

The invention also encompasses the administration of one or more compounds identified in accordance with the methods of the invention in combination with radiation therapy comprising the use of x-rays, gamma rays and other sources of radiation to destroy the cancer cells. In preferred embodiments, the radiation treatment is administered as external beam radiation or teletherapy wherein the radiation is directed from a remote source. In other preferred embodiments, the radiation treatment is administered as internal therapy or brachytherapy wherein a radioactive source is placed inside the body close to cancer cells or a tumor mass.

Cancer therapies and their dosages, routes of administration and recommended usage are known in the art and have been described in such literature as the Physician's Desk Reference (56th ed., 2002).

**5.10 USE OF THE IDENTIFIED COMPOUNDS TO PREVENT, MANAGE OR TREAT FUNGAL INFECTION**

The present invention provides methods of preventing, treating, managing or ameliorating a fungal infection or one or more symptoms thereof, said method comprising administering to a subject in need thereof, one or more compounds identified in accordance with the invention that inhibit or reduce the activity of an enzyme in a fungal tRNA splicing pathway. In a specific embodiment, the invention provides a method of preventing, treating, managing or ameliorating a fungal infection or one or more symptoms thereof, said method comprising administering to a subject in need thereof a dose of a prophylactically or therapeutically effective amount of one or more compounds that inhibit or reduce the activity of an enzyme in a fungal tRNA splicing pathway. In another embodiment, the invention provides a method of preventing, treating, managing or ameliorating a fungal infection refractory to standard or experimental therapies used in the art to prevent, treat, manage or ameliorate a fungal infection or one or more symptoms thereof, said method comprising administering to a subject in need thereof, an prophylactically or therapeutically effective amount of one or more compounds that inhibit or reduce the activity of an enzyme in a fungal tRNA splicing pathway.

The invention also provides methods of preventing, treating, managing or ameliorating a fungal infection or one or more symptoms thereof, said methods comprising administering to a subject in need thereof one or more of the compounds identified utilizing the screening methods described herein that inhibit or reduce the activity of an enzyme in a fungal tRNA splicing pathway, and one or more other therapies (*e.g.*, prophylactic or therapeutic agents), which therapies are currently being used, have been used or are known to be useful in the prevention, treatment, management or amelioration of said fungal infection or one or more symptoms thereof (including, but not limited to the prophylactic or therapeutic agents listed in Section 5.10.1 herein below). The therapies (*e.g.*, prophylactic or therapeutic agents) of the combination therapies of the invention can be administered sequentially or concurrently. In a specific embodiment, the combination therapies of the invention comprise a compound of the invention that inhibits or reduces the activity of an enzyme in a fungal tRNA splicing pathway and at least one other therapy (*e.g.*, prophylactic or therapeutic agent) which has the same mechanism as saud compound. In another embodiment, the combination therapies of the invention comprise a compound of the invention that inhibits or reduces the activity of an enzyme in a fungal tRNA splicing pathway and at least one other therapy (*e.g.*, prophylactic or therapeutic agent) which has a different mechanism of action than said compound. The combination therapies of the present invention improve the prophylactic or therapeutic effect of a compound of the invention by functioning together with the compound to have an additive or synergistic effect. The combination therapies of the present invention reduce the side effects associated with each terapy (*e.g.*, each prophylactic or therapeutic agent).

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

In specific embodiment, a pharmaceutical composition comprising one or more compounds identified in a screening assay described herein that inhibit or reduce the activity of an enzyme in a fungal tRNA splicing pathway is administered to a subject, preferably a human, to prevent, treat, manage or ameliorate a fungal infection or one or more symptoms thereof. In accordance with this embodiment, the pharmaceutical composition may also comprise one or more other therapies (*e.g.*, prophylactic or therapeutic agents) which are currently being used, have been used or are known to be useful in the prevention, treatment, management or amelioration of a fungal infection or one or more symptoms thereof.

The compounds identified in accordance with the methods of the invention that inhibit or reduce the activity of an enzyme in a fungal tRNA splicing pathway can be used to prevent, treat, manage or ameliorate a fungal infection by any fungal organism known in the art which causes a superficial infection, (*e.g.*, an infection that affect the skin), to a deeply invasive and lethal infection. The compounds identified in accordance with the methods of the invention that inhibit or reduce the activity of an enzyme in a fungal tRNA splicing pathway have anti-fungal activity against a broad range of fungal organisms, including but not limited to dermatophytes, trichophyton, microsporum, trichoderma, cryptococcus, fusarium, epidermophyton, Candida species (mainly Candida albicans, followed by Candida tropicalis), Aspergillus species, Cryptococcus neoformans, Histoplasma capsulatum, Coccidioides immitis, Pneumocystis carinii and some zygomycetes.

The compounds identified in accordance with the methods of the invention that inhibit or reduce the activity of an enzyme in a fungal tRNA splicing pathway of the invention are particularly useful in immunocompromised subjects who are susceptible to fungal infections. Immunocompromised patients include, for example, those infected with HIV, those undergoing chemotherapy, transplant recipients, or cancer patients receiving immunosuppressive medications. Fungal organisms which attack imunocompromised patients are often called opportunistic fungi including but not limited to Candida, trichosporon, cryptococcus. The compounds identified in accordance with the methods of the invention that inhibit or reduce the activity of an enzyme in a fungal tRNA splicing pathway are also useful in subject whose immune system is compromised due to therapy with broad spectrum anti-bacterial agents or who have been subject to invasive procedures (*e.g.*, catheters, prosthetic devices).

In some embodiments, the anti-fungal compounds identified by the methods of the invention have a comparable therapeutic efficacy as other antifungal agents known to those skilled in the art for the treatment, management and/or prevention of a fungal infection in a subject, preferably a mammal, unsubject most preferably a human subject, as determined by standard antifungal assays known to those skilled in the art. In a specific embodiment, the anti-fungal compounds identified by the methods of the invention have the same anti-fungal potency as amphotericin B, a polyene compound with a broad anti-fungal spectrum, including most species of *Candida.* In a specific preferred embodiment, the anti-fungal compounds identified in accordance with the invention have a reduced toxicity (*e.g.*, reduced renal toxicity, liver toxicity) while maintaining a similar clinical efficacy as antifungal agents commonly used in the art for the treatment, management and/or prevention of fungal infections in a mammal, preferably a human. In another preferred embodiment, the anti-fungal compounds identified in accordance with the invention have reduced side effects, including but not limited to, nausea and vomiting relative to other anti-fungal agents commonly used for the treatment and/or prevention of fungal infection, while maintaining a similar clinical efficacy as anti-fungal agents commonly used in the art for the treatment and/or prevention of fungal infections in a mammal, preferably a human.

In other embodiments, the antifungal compounds identified by the methods of the invention not only have antifungal activity themselves but also enhance the antifungal activity of other anti-fungal agents commonly used for the treatment management and/or prevention of fungal infections in a host, preferably a mammal. In other embodiments, the antifungal compounds identified by the methods of the invention not only have antifungal activity themselves but also enhance the therapeutic efficacy of other anti-fungal agents, commonly used for the treatment and/or management of fungal infections in a host, preferably a mammal, when they are used in combination with said anti-fungal agents.

In a preferred embodiment, the anti-fungal compounds identified by the methods of the invention are useful for treating, preventing, managing or ameliorating a fungal infection or one or more symptoms thereof of in subject, (preferably a mammal, unsubject and most preferably a human subject) with developed primary and/or secondary drug resistance to anti-fungal agents, including but not limited to polyene antifungal drugs (*e.g.,* amphotericin B), azole anti-fungal drugs, allylamine and morpholine anti-fungal drugs, antimetabolite anti-fungal drugs, that are commonly used.for the treatment, management and/or prevention of a fungal infection. In a specific embodiment, the anti-fungal compounds identified by the methods of the invention are useful for preventing, treating, managing or ameliorating a fungal infection or one or more symptoms thereof in a subject, preferably a mammal, a subject and most preferably a human subject), wherein the fungal species is resistant to amphotericin B, including but not limited to, *Psuedallescherica boydii, Fusarium* spp., *Trichosporon* spp., and *Candida guilliermondii.*

### 5.10.1 ANTI-FUNGAL AGENTS

Anti-fungal agents and therapies well known to one of skill in the art for prevention, treatment, management or amelioration of fungal infections can be used in the compositions and methods of the invention. Non-limiting examples of anti-fungal agents include proteins, polypeptides, peptides, fusion proteins, antibodies, nucleic acid molecules, organic molecules, inorganic molecules, and small molecules that inhibit or reduce fungal infection, inhibit or reduce the replication of fungi, or inhibit or reduce the spread of fungi to other subjects. Examples of anti-fungal agents include, but are not limited to, azole drugs *(e.g.,* miconazole, ketoconazole (NIZORAL®), imidazole, triazoles *(e.g.,* fluconazole (DIFLUCAN®)), and itraconazole (SPORANOX®)), polyene *(e.g.,* nystatin, amphotericin B (FUNGIZONE®), amphotericin B lipid complex ("ABLC")(ABELCET®), amphotericin B colloidal dispersion ("ABCD")(AMPHOTEC®), liposomal amphotericin B (AmBisome®)), potassium iodide (KI), and pyrimidine *(e.g.,* flucytosine (ANCOBON®)).

Anti-fungal therapies and their dosages, routes of administration, and recommended usage are known in the art and have been described in such literature as the Physician's Desk Reference (56th ed., 2002).

### 5.11 COMPOSITIONS AND METHODS OF ADMINISTERING COMPOUNDS

The present invention provides compositions comprising compounds identified in accordance with the methods of the invention or a pharmaceutically acceptable salt thereof. In a specific embodiment, the invention provides a composition comprising a compound identified in accordance with the methods of the invention that inhibits or reduces the activity of an animalia and/or fungal tRNA splicing endonuclease or a pharmaceutically acceptable salt thereof. In another embodiment, the invention provides a compositions comprising a compound identified in accordance with the methods of the invention that inhibits or reduces the activity or animalia and/or fungal tRNA splicing ligase. In accordance with these embodiments, the composition may also comprise one or more other prophylactic or therapeutic agents, said prophylactic or therapeutic agents known to be useful for, has been or is currently being used in the prevention, treatment, management or amelioration of a fungal infection or a proliferative disorder, or one or more symptoms thereof.

The compositions of the invention may be non-sterile, or sterile and suitable for administration to a subject. In addition to a prophylactic or therapeutic agent, the compositions of the invention may comprise a pharmaceutically acceptable vehicle in a suitable amount so as to provide the form for proper administration to a subject. In a preferred embodiment, the compositions of the invention are pharmaceutical compositions suitable for administration to a subject and comprise an effective amount of a compound identified in accordance with the methods of the invention or a pharmaceutically acceptable salt thereof and/or an effective amount of one or more other prophylactic or therapeutic agents, and a pharmaceutically acceptable vehicle.

Biologically active compounds identified using the methods of the invention or a pharmaceutically acceptable salt thereof can be administered to a patient, preferably a mammal, more preferably a human, suffering from a proliferative disorder or a fungal infection. In a specific embodiment, a compound or a pharmaceutically acceptable salt thereof is administered to a patient, (preferably a mammal and most preferably a human) as a preventative measure against a proliferative disorder or a fungal infection. When administered to a patient, the compound or a pharmaceutically acceptable salt thereof is preferably administered as component of a composition that optionally comprises a pharmaceutically acceptable vehicle.

The compositions of the invention can be administered orally, or by any other convenient route, for example, by infusion or bolus injection, by absorption through epithelial or mucocutaneous linings *(e.g.,* oral mucosa, rectal, and intestinal mucosa, etc.) and may be administered together with another biologically active agent. Administration can be systemic or local. Various delivery systems are known, *e.g.,* encapsulation in liposomes, microparticles, microcapsules, capsules, *etc.,* and can be used to administer the compound and pharmaceutically acceptable salts thereof.

Methods of administration include, but are not limited to, intradermal, intramuscular, intraperitoneal, intravenous, subcutaneous, intranasal, epidural, oral, sublingual, intranasal, intracerebral, intravaginal, transdermal, rectally, by inhalation, or topically, particularly to the ears, nose, eyes, or skin. The mode of administration is left to the discretion of the practitioner. In most instances, administration will result in the release of the compound or a pharmaceutically acceptable salt thereof into the bloodstream.

In specific embodiments, it may be desirable to administer the compound or a pharmaceutically acceptable salt thereof locally. This may be achieved, for example, and not by way of limitation, by local infusion during surgery, topical application, *e.g.,* in conjunction with a wound dressing after surgery, by injection, by means of a catheter, by means of a suppository, or by means of an implant, said implant being of a porous, nonporous, or gelatinous material, including membranes, such as sialastic membranes, or fibers.

In certain embodiments, it may be desirable to introduce the compound or a pharmaceutically acceptable salt thereof into the central nervous system by any suitable route, including intraventricular, intrathecal and epidural injection. Intraventricular injection may be facilitated by an intraventricular catheter, for example, attached to a reservoir, such as an Ommaya reservoir.

Pulmonary administration can also be employed, *e.g.*, by use of an inhaler or nebulizer, and formulation with an aerosolizing agent, or via perfusion in a fluorocarbon or synthetic pulmonary surfactant. In certain embodiments, the compound and pharmaceutically acceptable salts thereof can be formulated as a suppository, with traditional binders and vehicles such as triglycerides.

In another embodiment, the compound and pharmaceutically acceptable salts thereof can be delivered in a vesicle, in particular a liposome *(see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; *see* generally *ibid.*).

In yet another embodiment, the compound and pharmaceutically acceptable salts thereof can be delivered in a controlled release system (*see, e.g.,* Goodson, in Medical Applications of Controlled Release, supra, vol. 2, pp. 115-138 (1984)). Other controlled-release systems discussed in the review by Langer, 1990, Science 249:1527-1533 may be used. In one embodiment, a pump may be used *(see* Langer, *supra;* Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (*see* Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; *see* also Levy et al., 1985, Science 228:190; During et al., 1989, Ann. Neurol. 25:351; Howard et al., 1989, J. Neurosurg. 71:105). In yet another embodiment, a controlled-release system can be placed in proximity of a target RNA of the compound or a pharmaceutically acceptable salt thereof, thus requiring only a fraction of the systemic dose.

As discussed *supra,* the compositions of the invention may comprise a pharmaceutically acceptable vehicle which provides the form for administration to a subject. In a specific embodiment, the term "pharmaceutically acceptable" means approved by a regulatory agency of the Federal or a state government or listed in the U.S. Pharmacopeia or other generally recognized pharmacopeia for use in animals, mammals, and more particularly in humans. The term "vehicle" refers to a diluent, adjuvant, excipient, or carrier with which a compound of the invention is administered. Such pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilizing, thickening, lubricating and coloring agents may be used. When administered to a patient, the pharmaceutically acceptable vehicles are preferably sterile. Water is a preferred vehicle when the compound of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Compound compositions, if desired, can also contain minor amounts of wetting or emulsifying agents, or pH buffering agents.

Compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use. In one embodiment, the pharmaceutically acceptable vehicle is a capsule (*see e.g.,* U.S. Patent No. 5,698,155). Other examples of suitable pharmaceutical vehicles are described in Remington's Pharmaceutical Sciences, Alfonso R. Gennaro, ed., Mack Publishing Co. Easton, PA, 19th ed., 1995, pp. 1447 to 1676, incorporated herein by reference.

In a preferred embodiment, the compound or a pharmaceutically acceptable salt thereof is formulated in accordance with routine procedures as a pharmaceutical composition adapted for oral administration to human beings. Compositions for oral delivery may be in the form of tablets, lozenges, aqueous or oily suspensions, granules, powders, emulsions, capsules, syrups, or elixirs, for example. Orally administered compositions may contain one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving compound are also suitable for orally administered compositions. In these later platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, and the like. Such vehicles are preferably of pharmaceutical grade. Typically, compositions for intravenous administration comprise sterile isotonic aqueous buffer. Where necessary, the compositions may also include a solubilizing agent.

In another embodiment, the compound or a pharmaceutically acceptable salt thereof can be formulated for intravenous administration. Compositions for intravenous administration may optionally include a local anesthetic such as lignocaine to lessen pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the compound or a pharmaceutically acceptable salt thereof is to be administered by infusion, it can be dispensed, for example, with an infusion bottle containing sterile pharmaceutical grade water or saline. Where the compound or a pharmaceutically acceptable salt thereof is administered by injection, an ampoule of sterile water for injection or saline can be provided so that the ingredients may be mixed prior to administration.

The amount of a compound or a pharmaceutically acceptable salt thereof that will be effective in the treatment of a particular disease will depend on the nature of the disease, and can be determined by standard clinical techniques. In addition, *in vitro* or *in vivo* assays may optionally be employed to help identify optimal dosage ranges. The precise dose to be employed will also depend on the route of administration, and the seriousness of the disease, and should be decided according to the judgment of the practitioner and each patient's circumstances. However, suitable dosage ranges for oral administration are generally about 0.001 milligram to about 500 milligrams of a compound or a pharmaceutically acceptable salt thereof per kilogram body weight per day. In specific preferred embodiments of the invention, the oral dose is about 0.01 milligram to about 100 milligrams per kilogram body weight per day, more preferably about 0.1 milligram to about 75 milligrams per kilogram body weight per day, more preferably about 0.5 milligram to 5 milligrams per kilogram body weight per day. The dosage amounts described herein refer to total amounts administered; that is, if more than one compound is administered, or if a compound is administered with a therapeutic agent, then the preferred dosages correspond to the total amount administered. Oral compositions preferably contain about 10% to about 95% active ingredient by weight.

Suitable dosage ranges for intravenous (i.v.) administration are about 0.01 milligram to about 100 milligrams per kilogram body weight per day, about 0.1 milligram to about 35 milligrams per kilogram body weight per day, and about 1 milligram to about 10 milligrams per kilogram body weight per day. Suitable dosage ranges for intranasal administration are generally about 0.01 pg/kg body weight per day to about 1 mg/kg body weight per day. Suppositories generally contain about 0.01 milligram to about 50 milligrams of a compound of the invention per kilogram body weight per day and comprise active ingredient in the range of about 0.5% to about 10% by weight.

Recommended dosages for intradermal, intramuscular, intraperitoneal, subcutaneous, epidural, sublingual, intracerebral, intravaginal, transdermal administration or administration by inhalation are in the range of about 0.001 milligram to about 200 milligrams per kilogram of body weight per day. Suitable doses for topical administration are in the range of about 0.001 milligram to about 1 milligram, depending on the area of administration. Effective doses may be extrapolated from dose-response curves derived from *in vitro* or animal model test systems. Such animal models and systems are well known in the art.

The compound and pharmaceutically acceptable salts thereof are preferably assayed *in vitro* and *in vivo,* for the desired therapeutic or prophylactic activity, prior to use in humans. For example, *in vitro* assays can be used to determine whether it is preferable to administer the compound, a pharmaceutically acceptable salt thereof, and/or another therapeutic agent. Animal model, systems can be used to demonstrate safety and efficacy.

**Equivalents:**

The present invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

Various publications, patents and patent applications are cited herein, the disclosures of which are incorporated by reference in their entireties.

Preferred embodiments

1. A method of identifying an anti-fungal compound that inhibits or reduces the activity of a fungal tRNA splicing ligase, said method comprising:

(a) contacting a population of 5' tRNA half molecules and a population of 3' tRNA half molecules with a fungal cell-free extract or a purified fungal tRNA splicing ligase and a compound or a member of a library of compounds; and

(b) measuring the ligation of the half molecules, wherein an anti-fungal compound that inhibits or reduces the activity of a fungal tRNA splicing ligase is identified if an increase in ligated half molecules is detected relative to the signal in the absence of the compound or the presence of a negative control.

2. A method of identifying an anti-fungal compound that inhibits or reduces the activity of a fungal tRNA splicing ligase, said method comprising:

(a) contacting a population of 5' tRNA half molecules and a population of 3' tRNA half molecules with a fungal cell-free extract or a purified fungal tRNA splicing ligase and a compound or a member of a library of compounds, wherein the termini of one of the populations of tRNA molecules is labeled with a fluorophore and the other is labeled with a quencher; and

(b) measuring the fluorescence of the half molecules, wherein an antifungal compound that inhibits or reduces the activity of a fungal tRNA splicing ligase is identified if an increase in the fluorescent signal is detectable relative to the signal in the absence of the compound or the presence of a negative control.

3. A method of identifying an anti-fungal compound that inhibits or reduces the activity of a fungal tRNA splicing ligase, said method comprising:

(a) contacting a population of 5' tRNA half molecules and a population of 3' tRNA half molecules with a fungal cell-free extract or a purified fungal tRNA splicing ligase and a compound or a member of a library of compounds, wherein the termini of one of the populations of tRNA molecules is labeled with a fluorescent acceptor moiety and the other is labeled with a fluorescent donor moiety; and

(b) measuring the fluorescence of the half molecules, wherein an anti-fungal compound that inhibits or reduces the activity of a fungal tRNA splicing ligase is identified if a decrease the fluorescence emission of the fluorescent acceptor moiety at the wavelength of the fluorescent donor moiety relative to the fluorescence emission in the absence of the compound or the presence of a negative control is detected.

4. A method of identifying an anti-proliferative compound that inhibits or reduces the activity of an animalia tRNA splicing ligase, said method comprising:

(a) contacting a population of 5' tRNA half molecules and a population of 3' tRNA half molecules with an animalia cell-free extract or a purified animalia tRNA splicing ligase and a compound or a member of a library of compounds; and

(b) measuring the ligation of the half molecules, wherein an anti-proliferative compound that inhibits or reduces the activity of an animalia tRNA splicing ligase is identified if an increase in ligated half molecules is detected relative to the signal in the absence of the compound or the presence of a negative control.

5. A method of identifying an anti-proliferative compound that inhibits or reduces the activity of an animalia tRNA splicing ligase, said method comprising:

(a) contacting a population of 5' tRNA half molecules and a population of 3' tRNA half molecules with an animalia cell-free extract or a purified animalia tRNA splicing ligase and a compound or a member of a library of compounds, wherein the termini of one of the populations of tRNA molecules is labeled with a fluorophore and the other is labeled with a quencher; and

(b) measuring the fluorescence of the half molecules, wherein an antiproliferative compound that inhibits or reduces the activity of an animalia tRNA splicing ligase is identified if an increase in the fluorescent signal is detectable relative to the signal in the absence of the compound or the presence of a negative control.

6. A method of identifying an anti-proliferative compound that inhibits or reduces the activity of an animalia tRNA splicing ligase, said method comprising:

(a) contacting a population of 5' tRNA half molecules and a population of 3' tRNA half molecules with an alimalia cell-free extract or a purified animalia tRNA splicing ligase and a compound or a member of a library of compounds, wherein the termini of one of the populations of tRNA molecules is labeled with a fluorescent acceptor moiety and the other is labeled with a fluorescent donor moiety; and

(b) measuring the fluorescence of the half molecules, wherein an antiproliferative compound that inhibits or reduces the activity of an animalia tRNA splicing ligase is identified if a decrease the fluorescence emission of the fluorescent acceptor moiety at the wavelength of the fluorescent donor moiety relative to the fluorescence emission in the absence of the compound or the presence of a negative control is detected.

7. The method of any one of items 1-6, wherein said method further comprises a step wherein the structure of the compound that modulates tRNA splicing ligase activity is determined.

8. The method of item 1, 2 or 3, wherein said cell-free extract is a yeast cell-free extract.

9. The method of item 4, 5 or 6, wherein said cell-free extract is a mammalian cell-free extract.

10. The method of item 4, 5 or 6, wherein said cell-free extract is a human cell-free extract.

11. The method of any one of items 1-6, wherein said compound is selected from a combinatorial library of compounds comprising peptoids; random biooligomers; diversomers such as hydantoins, benzodiazepines and dipeptides; vinylogous polypeptides; nonpeptidal peptidomimetics; oligocarbamates; peptidyl phosphonates; peptide nucleic acid libraries; antibody libraries; carbohydrate libraries; and small organic molecule libraries.

12. The method of item 11, wherein said small organic molecule libraries are libraries of benzodiazepines, isoprenoids, thiazolidinones, metathiazanones, pyrrolidines, morpholino compounds, or diazepindiones.

13. The method of any one of items 1-6, wherein said method further comprises a step wherein the structure of the compound that modulates tRNA splicing endonuclease activity is determined.

14. The method of item 13, wherein said structure of the compound is determined by mass spectroscopy, NMR, vibrational spectroscopy, or X-ray crystallography.

15. The method of item 1, 2 or 3, wherein said compound directly binds said fungal tRNA splicing ligase.

16. The method of item 4, 5 or 6, wherein said compound directly binds said fungal tRNA splicing ligase.

17. The method of any one of items 1-6, wherein said compound binds to the substrate.

18. A method of treating, preventing or ameliorating a fungal infection or a symptom thereof, comprising administering an effective amount of a compound identified according to the method of item 1, 2 or 3, or a pharmaceutically acceptable salt thereof.

19. The method of item 18, wherein said fungal infection is a yeast infection.

20. A method of treating, managing, or ameliorating a proliferative disorder or a symptom thereof, comprising the administering to a subject an effective amount of a compound identified according to the method of item 4, 5 or 6, or a pharmaceutically acceptable salt thereof.

21. The method of item 20, wherein the proliferative disorder is cancer, psoriasis or pulmonary fibrosis.

22. The method of item 1, 2 or 3 further comprising assessing the effect of the compound on animalia tRNA splicing ligase.

23. The method of any one of items 1-6 further comprising a step wherein the cytotoxic activity of said compound is determined.

24. The method of any one of items 1-6 further comprising a step wherein the cytostatic activity of said compound is determined.

25. The method of item 18 or 20, wherein said subject is a human.

## Claims

1. A method of identifying a compound that modulates tRNA splicing ligase, the method comprising:

   (a) contacting a compound or a member of a library of compounds with an animalia tRNA splicing ligase or a fungal tRNA splicing ligase and a substrate for the tRNA splicing ligase, wherein the substrate comprises a population of 5' tRNA half-molecules and a population of 3' tRNA half-molecules; and
   (b) detecting the amount of substrate ligated, wherein an alteration in the amount of substrate ligated in the presence of the compound relative to the amount of substrate ligated in the absence of the compound or the presence of a negative control indicates that the compound modulates human tRNA splicing ligase activity.

2. The method of claim 1, wherein the animalia tRNA splicing ligase is in a cell-free extract.

3. The method of claim 1, wherein the fungal tRNA splicing ligase is a purified tRNA splicing ligase or is in a cell-free extract.

4. The method of claim 1 or 2, wherein the 5' tRNA half-molecules and the 3' tRNA half-molecules are purified by a method comprising:

   (a) incubating a purified human tRNA splicing endonuclease with a nucleic acid substrate comprising a tRNA intron in a mature domain of a precursor tRNA, wherein the purified human tRNA splicing endonuclease comprises a first fusion protein comprising the amino acid sequence of Hs Sen34p (Accession No. XP_085899) and a first peptide tag and/or a second fusion protein comprising the amino acid sequence of Hs Sen2p (SEQ ID NO:1) and a second peptide tag; and
   (b) purifying the resulting 5' tRNA half-molecules and 3' tRNA half-molecules.

5. The method of claim 1 or 3, wherein the 5' tRNA half-molecules and the 3' tRNA half-molecules are purified by a method comprising:

   (a) incubating a purified yeast tRNA splicing endonuclease with a nucleic acid substrate comprising a tRNA intron in a mature domain of a precursor tRNA, wherein the purified yeast tRNA splicing endonuclease comprises a first fusion protein comprising the amino acid sequence of Sc Sen34p (Accession No. YAR008w) and a first peptide tag and/or a second fusion protein comprising the amino acid sequence of Sc Sen2p (SEQ ID NO:3) and a second peptide tag; and
   (b) purifying the resulting 5' tRNA half-molecules and 3' tRNA half-molecules.

6. The method of claim 1, 2, 3, 4 or 5 wherein i) the termini of the 5' tRNA half-molecules are labeled with a fluorophore and the termini of the 3' tRNA half-molecules are labeled with a quencher, or the termini of the 5' tRNA half-molecules are labeled with a quencher and the termini of the 3' tRNA half-molecules are labeled with a fluorophore, and ii) a fluoresecent signal is detected.

7. The method of claim 6, wherein:

   (a) a reduction in the detection of the fluorescent signal in the presence of the compound relative to the detection of the fluorescent signal in the absence of the compound or the presence of a negative control indicates that the compound increases tRNA splicing ligase activity; and
   (b) an increase in the detection of the fluorescent signal in the presence of the compound relative to the detection of the fluorescent signal in the absence of the compound or the presence of a negative control indicates that the compound reduces tRNA splicing ligase activity.

8. The method of claim 1, 2, 3, 4 or 5, wherein i) the termini of the 5' tRNA half-molecules are labeled with a fluorescent donor moiety and the termini of the 3' tRNA half-molecules are labeled with a fluorescent acceptor moiety, or the termini of the 5' tRNA half-molecules are labeled with a fluorescent acceptor moiety and the termini of the 3' tRNA half-molecules are labeled with a fluorescent donor moiety; and ii) fluorescence emission is detected.

9. The method of claim 8, wherein:

(a) a reduction in the detection of the fluorescence emission in the presence of the compound relative to the detection of the fluorescence emission in the absence of the compound or the presence of a negative control indicates that the compound reduces tRNA splicing ligase activity; and
(b) an increase in the detection of the fluorescence emission in the presence of the compound relative to the detection of the fluorescence emission in the absence of the compound or the presence of a negative control indicates that the compound increases tRNA splicing ligase activity.

10. The method of claim 4, wherein the first and/or second peptide tag is fused to the amino terminal or carboxyl terminal of Hs Sen34p or Hs Sen2p.

11. The method of claim 5, wherein the first and/or second peptide tag is fused to the amino terminal or carboxyl terminal of Sc Sen34p or Sc Sen2p.

12. The method of claim 4, 5, or 11, wherein the first and/or second peptide tag is an immunoglobulin constant region, a polyhistidine sequence, glutathione S-transferase, the E. coli maltose binding protein, a cellulose binding domain or the FLAG epitope.

HTS Fluorescent Screening

**Fig. 1A**

**Fig. 1B**

**Fig. 1C**

```
              1                                                          50
Hs Sen2p      MAEAVFHAPK  RKRRVYETYE  SPLPIPFGQD  HGPLKEFKIF  RAEMINNNVI
Hs Sen2 var.  MAEAVFHAPK  RKRRVYETYE  SPLPIPFGQD  HGPLKEFKIF  RAEMINNNVI
Sc Sen2p      ~~~~~~~~~~  ~~~~~~~~~~  ~~~~~~~~~~  ~~~~~~~~~~  ~~~~~~~~~~

              51                                                         100
Hs Sen2p      VRNAEDIEQL  YGKGYFGKGI  LSRSRPSFTI  SDPKLVAKWK  DMKTNMPI..
Hs Sen2 var.  VRNAEDIEQL  YGKGYFGKGI  LSRSRPSFTI  SDPKLVAKWK  DMKTNMPI..
Sc Sen2p      ~~~~~~~~~~  ~~~~~~~~~~  ~~~~~~~~~~  ~MSKGRVNQK  RYKYPLPIHP

              101                                                        150
Hs Sen2p      ...ITSKRYQ  HSVEWAAELM  RRQGQDESTV  RRILKDYTKP  LE.HPPVKRN
Hs Sen2 var.  ...ITSKRYQ  HSVEWAAELM  RRQGQDESTV  RRILKDYTKP  LE.HPPVKRN
Sc Sen2p      VDDLPELILH  NPLSWLYWAY  RYYKSTNALN  DKVHVDFIGD  TTLHITVQ..

              151                                                        200
Hs Sen2p      EEAQVHDKLN  SGMVSNMEGT  AGGERPSVVN  GDSGKSGGVG  DPREPLGCLQ
Hs Sen2 var.  EEAQVHDKLN  SGMVSNMEGT  AGGERPSVVN  GDSGKSGGVG  DPREPLGCLQ
Sc Sen2p      DDKQMLYLWN  NGFFGT..GQ  FSRSEPTWKA  RTEARLGLND  TPLHNRGGTK

              201                                                        250
Hs Sen2p      EGSGCHPTTE  SFEKSVR.ED  ASPLPHVCCC  KQDALILQRG  LHHEDGSQHI
Hs Sen2 var.  EGSGCHPTTE  SFEKSVR.ED  ASPLPHVCCC  KQDALILQRG  LHHEDGSQHI
Sc Sen2p      SNTETEMTLE  KVTQQRRLQR  LEFKKERAKL  ERELLELRKK  GGHID.EENI

              251                                                        300
Hs Sen2p      GLLHPGDRGP  DHEYVLVEEA  ECAMSEREAA  PNEELVQRNR  LICRRNPYRI
Hs Sen2 var.  GLLHPGDRGP  DHEYVLVEEA  ECAMSEREAA  PNEELVQRNR  LICRRNPYRI
Sc Sen2p      LLEKQRESLR  KFKLKQTEDV  GIVAQQQDIS  ESNLRDEDNN  LLDENGDLLP

              301                                                        350
Hs Sen2p      FEYLQLSLEE  AFFLVYALGC  LSIYYEKEPL  TIVKLWKAFT  VVQPTFRTTY
Hs Sen2 var.  FEYLQLSLEE  ..........  .......EPL  TIVKLWKAFT  VVQPTFRTTY
Sc Sen2p      LESLELMPVE  AMFLTFALPV  LDISPACLAG  KLFQFDAKYK  DIH.SFVRSY

              351                                                        400
Hs Sen2p      MAYHYFRSKG  WVPKVGLKYG  TDLLIYRKGP  PFVHASYSVI  IELVDDHFEG
Hs Sen2 var.  MAYHYFRSKG  WVPKVGLKYG  TDLLIYRKGP  PFVHASYSVI  IELVDDHFEG·
Sc Sen2p      VIYHHYRSHG  WCVRSGIKFG  CDYLIYKRGP  PFQHAEFCV.  ..MGLDH...

              401                                                        450
Hs Sen2p      SLRRPLSWKS  LAALSRVSVN  VSKELMLCYL  IKPSTMTD..  ...KEMESPE
Hs Sen2 var.  SLRRPLSWKS  LAALSRVSVN  VSKELMLCYL  IKPSTMTD..  ...KEMESPE
Sc Sen2p      DVSKDYTWYS  ..SIARVVGG  AKKTFVLCYV  ERLISEQEAI  ALWKSNNFTK

              451                      477
Hs Sen2p      CMKRIKVQEV  ILSRWVSSRE  RSDQDDL
Hs Sen2 var.  CMKRIKVQEV  ILSRWVSSRE  RSDQDDL
Sc Sen2p      LFNSFQVGEV  LYKRWVPGRN  RD~~~~~
```

*Fig. 2*

Europäisches
Patentamt

European
Patent Office

Office européen
des brevets

# EUROPEAN SEARCH REPORT

Application Number

EP 10 18 5107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 00/67580 A (SMITHKLINE BEECHAM CORP [US]; SMITHKLINE BEECHAM PLC [GB]; GONTAREK RI) 16 November 2000 (2000-11-16) * pages 23-24 * | 1-12 | INV. A01N61/00 C12Q1/00 G01N33/566 G01N33/573 |
| Y | PHIZICKY E M ET AL: "YEAST TRNA LIGASE MUTANTS ARE NONVIABLE AND ACCUMULATE TRNA SPLICING INTERMEDIATES", JOURNAL OF BIOLOGICAL CHEMISTRY, AMERICAN SOCIETY OF BIOLOCHEMICAL BIOLOGISTS, BIRMINGHAM,, US, vol. 267, no. 7, 5 March 1992 (1992-03-05) , pages 4577-4582, XP001189521, ISSN: 0021-9258 * the whole document * | 1-12 | G01N33/574 C12Q1/68 |
| Y | ZILLMANN M ET AL: "Conserved mechanism of tRNA splicing in eukaryotes.", MOLECULAR AND CELLULAR BIOLOGY NOV 1991, vol. 11, no. 11, November 1991 (1991-11), pages 5410-5416, XP002439990, ISSN: 0270-7306 * the whole document * | 1-12 | |
| Y | LASKI F A ET AL: "CHARACTERIZATION TRANSFER RNA PRECURSOR SPLICING IN MAMMALIAN EXTRACTS", JOURNAL OF BIOLOGICAL CHEMISTRY, vol. 258, no. 19, 1983, pages 11974-11980, XP002458567, ISSN: 0021-9258 * the whole document * | 1-12 | TECHNICAL FIELDS SEARCHED (IPC) C12Q |

-/--

The present search report has been drawn up for all claims

| Place of search | Date of completion of the search | Examiner |
|---|---|---|
| Munich | 7 July 2011 | Dolce, Luca |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EUROPEAN SEARCH REPORT**

Application Number

EP 10 18 5107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | REYES V M ET AL: "A synthetic substrate for tRNA splicing.", ANALYTICAL BIOCHEMISTRY OCT 1987, vol. 166, no. 1, October 1987 (1987-10), pages 90-106, XP002439991, ISSN: 0003-2697 * the whole document * | 1-12 | |
| A | GREER C L ET AL: "MECHANISM OF ACTION OF A YEAST RNA LIGASE IN TRANSFER RNA SPLICING", CELL, vol. 32, no. 2, 1983, pages 537-546, XP002440002, ISSN: 0092-8674 * the whole document * | 1-12 | |
| A | GREER C L ET AL: "Substrate recognition and identification of splice sites by the tRNA-splicing endonuclease and ligase from Saccharomyces cerevisiae.", MOLECULAR AND CELLULAR BIOLOGY JAN 1987, vol. 7, no. 1, January 1987 (1987-01), pages 76-84, XP002439992, ISSN: 0270-7306 * the whole document * | 1-12 | TECHNICAL FIELDS SEARCHED (IPC) |
| A | BELFORD H G ET AL: "Multiple nucleotide cofactor use by yeast ligase in tRNA splicing. Evidence for independent ATP- and GTP-binding sites.", THE JOURNAL OF BIOLOGICAL CHEMISTRY 5 FEB 1993, vol. 268, no. 4, 5 February 1993 (1993-02-05), pages 2444-2450, XP002439993, ISSN: 0021-9258 * the whole document * | 1-12 | |

-/--

The present search report has been drawn up for all claims

| Place of search | Date of completion of the search | Examiner |
|---|---|---|
| Munich | 7 July 2011 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 10 18 5107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ABELSON ET AL: "tRNA splicing", JOURNAL OF BIOLOGICAL CHEMISTRY, AMERICAN SOCIETY OF BIOLOCHEMICAL BIOLOGISTS, BIRMINGHAM,, US, vol. 273, no. 21, 22 May 1998 (1998-05-22), pages 12685-12688, XP002984355, ISSN: 0021-9258 * the whole document * | 1-12 | |
| A | TROTTA C R ET AL: "The yeast tRNA splicing endonuclease: a tetrameric enzyme with two active site subunits homologous to the archael tRNA endonucleases", CELL, CELL PRESS, CAMBRIDGE, NA, US, vol. 89, 13 June 1997 (1997-06-13), pages 849-858, XP002984356, ISSN: 0092-8674 * the whole document * | 1-12 | |

**TECHNICAL FIELDS SEARCHED (IPC)**

The present search report has been drawn up for all claims

| Place of search | Date of completion of the search | Examiner |
|---|---|---|
| Munich | 7 July 2011 | Dolce, Luca |

EPO FORM 1503 03.82 (P04C01)

## ANNEX TO THE EUROPEAN SEARCH REPORT
## ON EUROPEAN PATENT APPLICATION NO.

EP 10 18 5107

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2011

| Patent document cited in search report | Publication date | Patent family member(s) | Publication date |
|---|---|---|---|
| WO 0067580 A | 16-11-2000 | NONE | |

EPO FORM P0459

For more details about this annex : see Official Journal of the European Patent Office, No. 12/82

## REFERENCES CITED IN THE DESCRIPTION

### Patent documents cited in the description

- US 4458066 A, Caruthers
- US 4415732 A
- WO 9525798 A
- EP 0524448 A
- US 6475719 B
- US 6342379 B
- US 6217847 B
- US 6265177 B, Squirrell
- US 5625048 A
- US 5491084 A
- US 5804387 A
- US 6251384 B, Tan
- US 5268463 A
- US 6472205 B
- US 5955604 A
- US 5741657 A
- US 4740459 A
- US 5726041 A
- US 6280940 B, Potts
- US 6472156 B
- US 6451543 B
- US 6348322 B
- US 6323039 B
- US 6297018 B
- US 6291201 B
- US 6280981 B
- US 5843658 A
- US 5439797 A
- US 5496934 A
- US 5202247 A
- US 5137819 A
- US 4215051 A
- US 6190619 B, Kilcoin
- US 6194612 B, Boger
- US 645154 A
- WO 02083837 A
- WO 02083953 A
- US 5885430 A
- US 5916428 A
- US 6027627 A
- US 6063251 A
- US 5699157 A
- US 5842787 A
- US 5869004 A
- US 5876675 A
- US 5942443 A
- US 5948227 A
- US 6042709 A
- US 6042710 A
- US 6046056 A
- US 6048498 A
- US 6086740 A
- US 6132685 A
- US 6150119 A
- US 6150180 A
- US 6153073 A
- US 6167910 A
- US 6171850 A
- US 6186660 A
- WO 021083953 A
- US 5786893 A, Fink
- US 5581085 A, Reffner
- US 5841139 A, Sostek
- US 5521153 A
- US 5883120 A
- US 5521169 A
- WO 02098370 A
- WO 02069904 A
- WO 9308656 A
- US 6162432 A
- US 5698155 A

### Non-patent literature cited in the description

- Principles of Cancer Patient Management. **STOCKDALE.** Scientific American: Medicine. 1998, vol. 3
- Principles Of Cancer Patient Management. **STOCKDALE.** Scientific American Medicine. 1998, vol. 3
- **E.S. BENEKE.** Human Mycoses. Upjohn Co, 1979
- **J.M.B. SMITH.** Opportunistic Mycoses of Man and Other Animals. CAB International, 1989
- Scrip's Antifungal Report. PJB Publications Ltd, 1992
- **H. NIELSON ; J. STENDERUP ; B. BRUUN.** Fungemia with Saccharomycetacea. *Scand. J. Infect. Dis.,* 1990, vol. 22, 581-584

- **WOLF, A.M.** Fungal Diseases of the Nasal Cavity of the Dog and Cat. *Vet. Clin. of North Amer.:Small Anim. Prac.,* 1992, vol. 22, 1119-1132
- **P.A. GERDING ; I. KAKOMA.** Microbiology of the Canine and Feline Eye. *Vet. Clin. of North Amer.:Small Anim. Prac.,* 1990, vol. 20, 615-625
- **W.H. RADENTZ.** Fungal Skin Infections Associated with Animal Contact. *AFP,* 1991, vol. 43, 1253-1256
- **M.R. LAPPIN.** *Vet. Clin. of North Amer.:Small Anim. Prac.,* vol. 23, 57-78
- **F.L. MCEWEN ; G.R. STEPHENSON.** The Use and Significance of Pesticides in the Environment. Wiley, 1979
- Control of Seed and Soil-Borne Plant Diseases. **R. RODRIGUEZ-KABANA ; P.A. BACKMAN ; E.A. CURL.** Antifungal Compounds. Marcel Dekker Inc, 1977
- **G. ORDISH ; J.F. MITCHELL.** World Fungicide Usage. In Fungicides, an Advanced Treatise. Academic Press, 1967, vol. 1, 39-62
- Estimated Crop Losses Without the Use of Fungicides and Nematicides and Without Nonchemical Controls. **E.W. PALM.** CRC Handbook of Pest Management in Agriculture. vol. 1, 139f
- Fungicides in Wood Preservation. **M.P. LEVI.** Antifungal Compounds. Marcel Dekker Inc, 1977
- Fungicides in Industry. **C.C. YEAGER.** Antifungal Compounds. Marcel Dekker Inc, 1977
- **J.R. GRAYBILL.** New Antifungal Agents. *Eur. J. Clin. Microbiol. Dis.,* 1989, vol. 8, 402-412
- **Y. KOLTIN.** Targets for Antifungal Drug Discovery. *Annual Reports in Medicinal Chemistry,* 1989, vol. 25, 141-148
- **L. SILVER ; K. BOSTIAN.** Screening of Natural Products for Antimicrobial Agents. *Eur. J. Clin. Microbiol. Dis.,* 1990, vol. 9, 455-461
- New Approaches for Antifungal Drugs. Birkhauser, 1992
- **J. DEKKER.** *Preventing and Managing Fungicide Resistance, Pesticide Resistance: Strategies and Tactics in Man*
- tRNA Processing Nucleases. **DEUTSCHER, M.P.** tRNA:Structure, Biosynthesis and Function. American Society for Microbiology, 1995, 51-65
- **BJORK, G.** Biosynthesis and Function of Modified Nucleosides. *tRNA: Structure, Biosynthesis and Function,* 1995, 165-205
- tRNA Splicing: An RNA World Add-On or an Ancient Reaction?. **TROTTA, C.R. ; ABELSON, J.N.** RNA World II. Cold Spring Harbor Laboratory Press, 1999
- **ABELSON et al.** *Journal of Biological Chemistry,* 1998, vol. 273, 12685-12688
- **REYES et al.** *Cell,* 1988, vol. 55, 719-730
- **FRUSCOLONI et al.** *EMBO Rep,* 2001, vol. 2, 217-221

- **IKEMURA, T. ; OKEKI, H.** *Cold Spring Harbor Symp. Quant. Biol.,* 1983, vol. 47, 1087-1097
- **BJORK, G.** Biosynthesis and Function of modified Nucleoside in tRNA: Structure, Biosynthesis and Function. American Society for Microbiology, 1995
- **CARUTHERS et al.** *Genetic Engineering,* 1982, vol. 4, 1-17
- Users Manual Model 392 and 394 Polynucleotide Synthesizers. 1990, 6-16-22
- **OJWANG et al.** *Biochemistry,* 1997, vol. 36, 6033-6045
- **HWANG et al.** *Proc. Natl. Acad. Sci. USA,* 1999, vol. 96 (23), 12997-13002
- **SAMBROOK.** Molecular Cloning, A Laboratory Manual. 1989
- DNA Cloning. 1985, vol. I, II
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **PERBAL.** A Practical Guide to Molecular Cloning. 1984
- Gene Transfer Vectors for Mammalian Cells. Cold Spring Harbor Laboratory, 1987
- Methods in Enzymology. vol. 154-155
- METHODS IN ENZYMOLOGY. 1987
- Immunochemical Methods in Cell and Molecular Biology. Academic Press, 1987
- Expression of Proteins in Mammalian Cells Using Vaccinia Viral Vectors in Current Protocols in Molecular Biology. 1991, vol. 2
- **SAMBROOK et al.** Molecular Cloning, A Laboratory Manual. Cold Spring Harbor Laboratory, 1990
- Current Protocols in Molecular Biology. John Wiley & Sons, 1998
- **GREER ; SZALAY.** *Luminescence,* 2002, vol. 17 (1), 43-74
- **DEVINE et al.** *Biochim. Biophys. Acta,* 1993, vol. 1173 (2), 121-132
- **WOOD et al.** *Science,* 1989, vol. 244, 700-702
- **LORENZ et al.** *Proc Natl Acad Sci U S A,* 1991, vol. 88 (10), 4438-4442
- **SULA-NEWBY et al.** *Biochem J.,* 1996, vol. 313, 761-767
- **MANUKHOV et al.** *Genetika,* 2000, vol. 36 (3), 322-30
- **MIYAMOTO et al.** *J Biol Chem.,* 1988, vol. 263 (26), 13393-9
- **COHN et al.** *Proc Natl Acad Sci USA.,* 1983, vol. 80 (1), 120-3
- **PRASHER et al.** *Gene,* 1992, vol. 111, 229-233
- **YANG et al.** *Nature Biotechnol.,* 1996, vol. 14, 1252-1256

- **CODY et al.** *Biochemistry,* 1993, vol. 32, 1212-1218
- **MORIN et al.** *J. Cell Physiol.,* 1972, vol. 77, 313-318
- **HEIM et al.** *Nature,* 1995, vol. 373, 663-664
- **DELAGRAVE et al.** *Biotechnology,* 1995, vol. 13, 151-154
- **CORMACK et al.** *Gene,* 1996, vol. 173, 33-38
- **CRAMER et al.** *Nature Biotechnol.,* 1996, vol. 14, 315-319
- **CHALFIE et al.** *Science,* 1994, vol. 263, 802-805
- **HEIM et al.** *Proc. Natl. Acad. Sci. USA,* 1994, vol. 91, 12501-12504
- **MORISE et al.** *Biochemistry,* 1974, vol. 13, 2656-2662
- **WARD et al.** *Photochem. Photobiol.,* 1980, vol. 31, 611-615
- **RIZZUTO et al.** *Curr. Biology,* 1995, vol. 5, 635-642
- **KAETHER ; GERDES.** *FEBS Lett,* 1995, vol. 369, 267-271
- **HU ; CHENG.** *FEBS Lett,* 1995, vol. 369, 331-33
- **DAVIS et al.** *Dev. Biology,* 1995, vol. 170, 726-729
- **NIELSEN et al.** *Proc Natl Acad Sci USA,* 1983, vol. 80 (17), 5198-5202
- **EUSTICE et al.** *Biotechniques,* 1991, vol. 11, 739-742
- **HENDERSON et al.** *Clin. Chem.,* 1986, vol. 32, 1637-1641
- **JEFFERSON et al.** *Proc. Natl. Acad. Sci. USA,* 1986, vol. 86, 844.7-84.51
- **CHRISTENSEN et al.** *Biochem. J.,* 1990, vol. 266, 853-861
- **O'CALLAGHAN et al.** *Antimicrob. Agents. Chemother,* 1968, vol. 8, 57-63
- **STRATTON.** *J. Antimicrob. Chemother.,* 1988, vol. 22 (A), 23-35
- **RICHMOND ; SYKES.** *Adv.Microb.Physiol.,* 1978, vol. 9, 31-88
- **AMBLER.** *Phil. Trans. R. Soc. Lond. [Ser.B.,* 1980, vol. 289, 321-331
- **KADONAGA et al.** *J.Biol.Chem.,* 1984, vol. 259, 2149-2154
- **SUTCLIFFE.** *Proc. Natl. Acad. Sci. USA,* 1978, vol. 75, 3737-3741
- **BERNOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. U.S.A.,* 1981, vol. 78, 1441-1445
- **BRINSTER et al.** *Nature,* 1982, vol. 296, 39-42
- **VILLA-KAMAROFF et al.** *Proc. Natl. Acad. Sci. U.S.A.,* 1978, vol. 75, 3727-3731

- **DEBOER et al.** *Proc. Natl. Acad. Sci. U.S.A.,* 1983, vol. 80, 21-25
- Useful proteins from recombinant bacteria. *Scientific American,* 1980, vol. 242, 74-94
- **HERRERA-ESTRELLA et al.** *Nature,* vol. 303, 209-213
- **GARDNER et al.** *Nucl. Acids Res.,* 1981, vol. 9, 2871
- **HERRERA-ESTRELLA et al.** *Nature,* 1984, vol. 310, 115-120
- **SWIFT et al.** *Cell,* 1984, vol. 38, 639-646
- **ORMITZ et al.** *Cold Spring Harbor Symp. Quant. Biol.,* 1986, vol. 50, 399-409
- **MACDONALD.** *Hepatology,* 1987, vol. 7, 425-515
- **HANAHAN.** *Nature,* 1985, vol. 315, 115-122
- **GROSSCHEDL et al.** *Cell,* 1984, vol. 38, 647-658
- **ADAMES et al.** *Nature,* 1985, vol. 318, 533-538
- **ALEXANDER et al.** *Mol. Cell. Biol,* 1987, vol. 7, 1436-1444
- **LEDER et al.** *Cell,* 1986, vol. 45, 485-495
- **PINKERT et al.** *Genes and Devel.,* 1987, vol. 1, 268-276
- **KRUMLAUF et al.** *Mol. Cell. Biol.,* 1985, vol. 5, 1639-1648
- **HAMMER et al.** *Science,* 1987, vol. 235, 53-58
- **KELSEY et al.** *Genes and Devel.,* 1987, vol. 1, 161-171
- **MOGRAM et al.** *Nature,* 1985, vol. 315, 338-340
- **KOLLIAS et al.** *Cell,* 1986, vol. 46, 89-94
- **READHEAD et al.** *Cell,* 1987, vol. 48, 703-712
- **SANI.** *Nature,* 1985, vol. 314, 283-286
- **MASON et al.** *Science,* 1986, vol. 234, 1372-1378
- **GRAHAM ; VAN DER EB.** *Virol.,* 1978, vol. 52, 546
- **WIGLER et al.** *Cell,* 1977, vol. 11, 223
- **SZYBALSKA ; SZYBALSKI.** *Proc. Natl. Acad. Sci. USA,* 1962, vol. 48, 2026
- **LOWY et al.** *Cell,* 1980, vol. 22, 817
- **WIGLER et al.** *Natl. Acad. Sci. USA,* 1980, vol. 77, 3567
- **O'HARE et al.** *Proc. Natl. Acad. Sci. USA,* 1981, vol. 78, 1527
- **MULLIGAN ; BERG.** *Proc. Natl. Acad. Sci. USA,* 1981, vol. 78, 2072
- **COLBERRE-GARAPIN et al.** *J. Mol. Biol.,* 1981, vol. 150, 1
- **SANTERRE et al.** *Gene,* 1984, vol. 30, 147
- **KRIEG ; MELTON.** *Nature,* 1984, vol. 308, 203

- **DIGNAM et al.** *Methods Enzymol.,* 1990, vol. 182, 194-203
- **WU et al.** *Methods in Enzymol,* 1987, vol. 152, 343-349
- **MAKRIDES.** *Microbiol Rev,* 1996, vol. 60, 512-538
- **MAKRIDES.** *Microbiol Rev,* vol. 60, 512-538
- **HUYNH et al.** DNA Cloning Techniques'', Vol. I: A Practical Approach. IRL Press, 1984, vol. I, 49-78
- **STUDIER et al.** *Methods Enzymol.,* 1990, vol. 185, 60-89
- **WILLIAMS et al.** *Cancer Res.,* 1989, vol. 49, 2735-42
- **TAYLOR et al.** *Mol. Cell Biol.,* 1990, vol. 10, 165-75
- **SZYBALSKI ; SZYBALSKI.** *Proc. Natl. Acad. Sci. USA,* 1962, vol. 48, 2026
- Current Protocols in Molecular Biology. Greene Publish. Assoc. & Wiley Interscience, vol. 2
- **SMITH.** *Methods Mol. Cell Bio,* 1993, vol. 4, 220-229
- **GUAN et al.** *Gene,* 1987, vol. 67, 21-30
- **TOMME et al.** *Protein Eng.,* 1994, vol. 7, 117-123
- Short Protocols in Molecular Biology. John Wiley & Sons, Inc, 1999
- **KRIEGLER M.** Gene Transfer and Expression: A Laboratory Manual. Freeman & Co, 1990
- **GRAHAM et al.** *J. Gen. Virol.,* 1977, vol. 36, 59
- **URLAUB ; CHASIN.** *Proc. Natl. Acad. Sci.,* 1980, vol. 77, 4216
- **MATHER.** *Biol. Reprod,* 1980, vol. 23, 243-251
- **HAMER et al.** *Cell,* 1979, vol. 17, 725
- **VAN DOREN et al.** *Mol Cell Biol,* 1984, vol. 4, 1653
- **MCLAUGHLIN et al.** *J Virol,* 1988, vol. 62, 1963
- **ZINN et al.** *Proc Natl Acad Sci,* 1982, vol. 79, 4897
- **LOGAN ; SHENK.** *Proc. Natl. Acad. Sci. (USA,* 1984, vol. 81, 3655-3659
- Current Protocols in Molecular Biology. Greene Publish. Assoc. & Wiley Interscience, 1988, vol. 2
- Expression and Secretion Vectors for Yeast. **GRANT.** Methods in Enzymology. Acad. Press, 1987, vol. 153, 516-544
- **GLOVER.** DNA Cloning. IRL Press, 1986, vol. II
- Heterologous Gene Expression in Yeast. **BITTER.** Methods in Enzymology. Acad. Press, 1987, vol. 152, 673-684
- The Molecular Biology of the Yeast Saccharomyces. Cold Spring Harbor Press, 1982, vol. I, II
- **SMITH et al.** *J Virol,* 1983, vol. 46, 584
- Appendix 5 of Current Protocols in Molecular Biology. Greene Publish. Assoc. & Wiley Interscience, 1988
- **HANAHAN.** *DNA Cloning, A Practical Approach,* 1985, vol. 1, 109-136
- **WIGLER et al.** *Cell,* 1977, vol. 11, 223-232
- **SCHAEFER-RIDDER et al.** *Science,* 1982, vol. 215, 166-168
- **WOLFF et al.** *Proc Natl Acad Sci,* 1987, vol. 84, 3344
- **CAPPECHI.** *Cell,* 1980, vol. 22, 479-488
- **LANGONE.** *J. Immunol. meth.,* 1982, vol. 51, 3
- **WILCHEK et al.** *Biochem. Intl.,* 1982, vol. 4, 629
- **SJOBRING et al.** *J. Biol. Chem.,* 1991, vol. 26, 399
- Antibodies A Laboratory Manual. Cold Spring Harbor laboratory, 1988, 617-618
- **SMITH.** *Methods Mol. Cell Bio.,* 1993, vol. 4, 220-229
- Antibodies A Laboratory Manual. Cold Spring Harbor laboratory, 1988
- Current Protocols in Immunology. John Wiley, 1991
- **TROTTA et al.** *Cell,* 1997, vol. 89, 849-858
- **XU et al.** *Methods in Enzymology,* 1990, vol. 181, 463-471
- **PHIZICKY et al.** *Journal of Biol. Chem.,* 1986, vol. 261 (6), 2978-2986
- **EGNER et al.** *J.Org. Chem.,* 1995, vol. 60, 2652
- **ANDERSON et al.** *J. Org. Chem.,* 1995, vol. 60, 2650
- **FITCH et al.** *J. Org. Chem.,* 1994, vol. 59, 7955
- **LOOK et al.** *J. Org. Chem.,* 1994, vol. 49, 7588
- **METZGER et al.** *Angew. Chem., Int. Ed. Engl.,* 1993, vol. 32, 894
- **YOUNGQUIST et al.** *Rapid Commun. Mass Spect.,* 1994, vol. 8, 77
- **CHU et al.** *J. Am. Chem. Soc.,* 1995, vol. 117, 5419
- **BRUMMEL et al.** *Science,* 1994, vol. 264, 399
- **STEVANOVIC et al.** *Bioorg. Med. Chem. Lett.,* 1993, vol. 3, 431
- **LAM et al.** *Chem. Rev.,* 1997, vol. 97, 41-448
- **OHLMEYER et al.** *Proc. Natl. Acad. Sci. USA,* 1993, vol. 90, 10922-10926
- **NEFZI et al.** *Chem. Rev.,* 1997, vol. 97, 449-472
- t al, eds, 1994, Current Protocols in Molecular Biology. Wiley & Sons, Inc, 1994, vol. 1
- Current Protocols in Molecular Biology. John Wiley Sons, Inc, 1994, vol. 1
- Current Protocols in Molecular Biology. John Wiley & Sons, Inc, 1994, vol. 1, 10.8.1

- Current Protocols in Molecular Biology. John Wiley & Sons, Inc, 1994, vol. 1, 11.2.1
- **STRYER, L.** *Ann. Rev. Biochem.,* 1978, vol. 47, 819-846
- **C. R. CANTOR ; P. R. SCHIMMEL.** BIOPHYSICAL CHEMISTRY part II, Techniques for the Study of Biological Structure and Function. W. H. Freeman and Co, 1980, 448-455
- **SELVIN, P. R.** *Methods in Enzymology,* 1995, vol. 246, 300-335
- **HAUGLAND et al.** *P.N.A.S. U.S.A.,* 1969, vol. 63, 24-30
- Fluorescence Spectroscopy. Marcel Dekker, 1971
- **WHITE et al.** Fluorescence Analysis: A practical Approach. Marcel Dekker, 1970
- Handbook of Fluorescent Probes and Research Chemicals. Molecular Probes, Inc, 1996
- **ADAMS et al.** *Nature,* 1991, vol. 349, 694-697
- **QIN ; PYLE.** *Methods,* 1999, vol. 18 (1), 60-70
- **BUVOLI et al.** *Molecular and Cellular Biology,* 2000, vol. 20, 3116-3124
- **PARK ; BHANDARY.** *Molecular and Cellular Biology,* 1998, vol. 18, 4418-4425
- **SARKAR ; HOPPER.** *Mol. Biol. Cell,* 1998, vol. 9, 3041-3055
- **ABELSON et al.** *Journal of Biol. Chem.,* 1998, vol. 273 (21), 2685-88
- **BELFORT et al.** *Cell,* 1997, vol. 89, 1003-6
- **BELFORD et al.** *Journal of Biological Chem.,* 1993, vol. 268 (4), 2444-2450
- **GOMES et al.** *Biochemical and Biophysical Res. Comm.,* 1998, vol. 237, 588-94
- **GREER et al.** *Cell,* 1982, vol. 32, 537-46
- **XU et al.** *Methods in Enzymology,* 1990, vol. 181, 463-71
- **PHYIZICKY et al.** *Journal of Biol. Chem.,* 1986, vol. 261 (6), 2978-86
- **PICK et al.** *J. Biol. Chem.,* 1986, vol. 261, 6684
- **GREER et al.** *Cell,* 1983, 537
- **GOMES et al.** *Biochemical and Biophysical Res. Comm.,* 1997, vol. 237 (3), 588-594
- **REYES et al.** *Anal. Biochem,* 1987, vol. 166, 90-106
- **GRUIC-SOVULJ et al.** *J. Biol. Chem.,* 1997, vol. 272, 32084-32091
- **XAVIER et al.** *Trends Biotechnol.,* 2000, vol. 18 (8), 349-356
- **SANNES-LOWERY et al.** *Anal. Chem.,* 1997, vol. 69, 5130-5135
- **HOFSTADLER et al.** *Anal. Chem.,* 1999, vol. 71, 3436-3440
- **GRIFFEY et al.** *Proc. Natl. Acad. Sci. USA,* 1999, vol. 96, 10129-10133
- **SHUKER et al.** *Science,* 1996, vol. 274, 1531-1534
- **MOORE.** *Curr. Opin. Biotechnol.,* 1999, vol. 10, 54-58
- **FEJZO et al.** *Chem. Biol.,* 1999, vol. 6, 755-769
- **BLUNDELL et al.** *Nat Rev Drug Discov,* 2002, vol. 1 (1), 45-54
- **FENNIRI et al.** *J. Am. Chem. Soc.,* 2000, vol. 123, 8151-8152
- **SWAFFORD et al.** *Mol. Cell. Biol.,* 1997, vol. 17, 1366-1374
- **MABRY et al.** *Cancer Cells,* 1991, vol. 3, 53-58
- **PARK ; GAZDAR.** *J. Cell Biochem.,* 1996, vol. 24, 131-141
- **HAMBLY et al.** *Breast Cancer Res. Treat.,* 1997, vol. 43, 247-258
- **GIERTHY et al.** *Chemosphere,* 1997, vol. 34, 1495-1505
- **PRASAD ; CHURCH.** *Biochem. Biophys. Res. Commun.,* 1997, vol. 232, 14-19
- **WEBBER et al.** *Prostate,* 1996, vol. 29, 386-394
- *PROSTATE,* vol. 30, 58-64
- *PROSTATE,* vol. 30, 136-142
- **BOULIKAS.** *Anticancer Res.,* 1997, vol. 17, 1471-1505
- **RIBEIRO et al.** *Int. J. Radiat. Biol.,* 1997, vol. 72, 11-20
- **BOOTH et al.** *Lab Invest.,* 1997, vol. 76, 843-857
- **VET et al.** *Biochim. Biophys Acta,* 1997, vol. 1360, 39-44
- **DREXLER.** *Leuk. Res.,* 1994, vol. 18, 919-927
- **TOHYAMA.** *Int. J. Hematol.,* 1997, vol. 65, 309-317
- **HOSHINO et al.** *Int. J. Cancer,* 1986, vol. 38, 369
- **CAMPANA et al.** *J. Immunol. Meth.,* 1988, vol. 107, 79
- **CHEN, J.** *Oncogene,* 1996, vol. 13, 1395-403
- **JEOUNG, J.** *J. Biol. Chem.,* 1995, vol. 270, 18367-73
- **LURIA et al.** General Virology. John Wiley & Sons, 1978, 436-446
- **HORDIJK et al.** *Science,* 1997, vol. 278, 1464-66
- **OHNISHI, T.** *Biochem. Biophys. Res. Commun.,* 1993, vol. 193, 518-25
- **VAIL ; MACEWEN.** *Cancer Invest,* 2000, vol. 18 (8), 781-92
- **ZHANG ; ROTH.** *In Vivo,* 1994, vol. 8 (5), 755-69
- **MORRIS et al.** *J La State Med Soc,* 1998, vol. 150 (4), 179-85
- **HOSOKAWA et al.** *Transgenic Res,* 2001, vol. 10 (5), 471-8

- **KADO et al.** *Cancer Res,* 2001, vol. 61 (6), 2395-8
- **WANG et al.** *Int J Pancreatol,* 2001, vol. 29 (1), 37-46
- **GHANEH et al.** *Gene Ther,* 2001, vol. 8 (3), 199-208
- **BRYANT et al.** *Lab Invest,* 2000, vol. 80 (4), 553-73
- **HOUGH et al.** *Proc Natl Acad Sci USA,* 1998, vol. 95 (23), 13853-8
- **HERBER et al.** *J Virol,* 1996, vol. 70 (3), 1873-81
- **FODDE ; SMITS.** *Trends Mol Med,* 2001, vol. 7 (8), 369-73
- **KURAGUCHI et al.** *Oncogene,* 2000, vol. 19 (50), 5755-63
- **ARENDRUP et al.** *Infection,* 2002, vol. 30 (5), 286-291
- **KAMIE.** *Mycopathologia,* 2001, vol. 152 (1), 5-13
- **GUHAD et al.** *FEMS Microbiol Lett.,* 2000, vol. 192 (1), 27-31
- **YAMAGATA et al.** *J Clin Microbiol.,* 2000, vol. 38 (9), 32606
- **ANDRUTIS et al.** *J Clin Microbiol.,* 2000, vol. 38 (6), 2317-2323
- **COCK et al.** *Rev Inst Med Trop Sao Paulo,* 2000, vol. 42 (2), 59-66
- **SHIBUYA et al.** *Microb Pathog.,* 1999, vol. 27 (3), 123-131
- **BEERS et al.** *J Lab Clin Med.,* 1999, vol. 133 (5), 423-33
- **NAJVAR et al.** *Antimicrob Agents Chemother.,* 1999, vol. 43 (2), 413-414
- **WILLIAMS et al.** *J Infect Dis.,* 1988, vol. 178 (4), 1217-1221
- **YOSHIDA.** *Kansenshogaku Zasshi,* 1998, vol. 72 (6), 621-630
- **ALEXANDRAKIS et al.** *Br J Ophthalmol.,* 1998, vol. 82 (3), 306-11
- **CHAKRABARTI et al.** *J Med Vet Mycol.,* 1997, vol. 35 (4), 295-97
- **MARTIN et al.** *Antimicrob Agents Chemother.,* 1997, vol. 41 (1), 13-16
- **CHU et al.** *Avian Dis.,* 1996, vol. 40 (3), 715-19
- **FIDEL et al.** *J Infect Dis.,* 1996, vol. 173 (2), 425-31
- **COLE et al.** *FEMS Microbiol Lett. 15,* 1995, vol. 126 (2), 177-80
- **POLLOCK et al.** *Nat Genet.,* 1995, vol. 9 (2), 202-09
- **UCHIDA et al.** *Jpn J Antibiot.,* 1994, vol. 47 (10), 1407-12
- **MAEBASHI et al.** *J Med Vet Mycol,* 1994, vol. 32 (5), 349-59
- **JENSEN ; SCHONHEYDER.** *J Exp Anim Sci.,* 1993, vol. 35 (4), 155-60
- **GOKSALAN ; ANAISSIE.** *Infect Immun.,* 1992, vol. 60 (8), 3339-44
- **KURUP et al.** *J Immunol.,* 1992, vol. 148 (12), 3783-88
- **SINGH et al.** *Mycopatholgia,* 1990, vol. 112 (3), 127-37
- **SALKOWSKI ; BALISH.** *Infect Immun.,* 1990, vol. 58 (10), 3300-06
- **AHMAD et al.** *Am J Kidney Dis.,* 1985, vol. 7 (2), 153-56
- **ALTURE-WERBER E ; EDBERG SC.** *Mycopathologia,* 1985, vol. 89 (2), 69-73
- **KANE et al.** *Antimicrob Agents Chemother,* 1981, vol. 20 (5), 595-99
- **BARBEE et al.** *Am J Pathol.,* 1977, vol. 86 (1), 281-84
- **MAESTRONE et al.** *Am J Vet Res.,* 1973, vol. 34 (6), 833-36
- National Committee for Clinical Laboratories Standards. *Proposed Standard M27T,* 1995
- **PFALLER et al.** *Infectious Dis. Clin. N. Am.,* 1993, vol. 7, 435-444
- **CLANCY et al.** *Journal of Clinical Microbiology,* 1997, vol. 35 (11), 2878-82
- **RYDER et al.** *Antimicrobial Agents and Chemotherapy,* 1998, vol. 42 (5), 1057-61
- **PFALLER et al.** *Journal of Clinical Microbiology,* 1994, vol. 32 (8), 1993-6
- **TIBALLI et al.** *Journal of Clinical Microbiology,* 1995, vol. 33 (4), 915-7
- **JAHN et al.** *Journal of Clinical Microbiology,* 1995, vol. 33 (3), 661-667
- **GOOTZ, T. D.** *Clin. Microbiol. Rev.,* 1990, vol. 3, 13-31
- **MOORE et al.** *Compound Research,* 1994, vol. 7, 265-269
- **SRINIVAS et al.** *J. Biol. Chem.,* 1992, vol. 267, 7121-7127
- **ROTIVINEN et al.** *Acta Pharmaceutical Fennica,* 1988, vol. 97, 159-166
- **RIPKA.** *New Scientist,* 1998, vol. 54-57
- **MCKINALY ; ROSSMANN.** *Annu. Rev. Pharmacol. Toxiciol.,* 1989, vol. 29, 111-122
- **PERRY ; DAVIES.** OSAR: Quantitative Structure-Activity Relationships in Drug Design. Alan R. Liss, Inc, 1989, 189-193
- **LEWIS ; DEAN.** *Proc. R. Soc. Lond.,* 1989, vol. 236, 125-140141-162
- **ASKEW et al.** *J. Am. Chem. Soc.,* 1989, vol. 111, 1082-1090
- **FISHMAN et al.** Medicine. J.B. Lippincott Co, 1985
- **MURPHY et al.** Informed Decisions: The Complete Book of Cancer Diagnosis, Treatment, and Recovery. Penguin Books U.S.A., Inc, 1997
- Physician's Desk Reference. 2002
- **LANGER.** *Science,* 1990, vol. 249, 1527-1533

- **TREAT et al.** Liposomes in the Therapy of Infectious Disease and Cancer. Liss, 1989, 353-365
- **LOPEZ-BERESTEIN.** LIPOSOMES IN THE THERAPY OF INFECTIOUS DISEASE AND CANCER. 317-327
- **GOODSON.** *Medical Applications of Controlled Release,* 1984, vol. 2, 115-138
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **RANGER ; PEPPAS.** *J. Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105
- Remington's Pharmaceutical Sciences. Mack Publishing Co, 1995, 1447-1676